# EUROPEAN PATENT SPECIFICATION

(11) **EP 1 471 786 B1**
(45) Date of publication and mention of the grant of the patent: **27.12.2006**
(21) Application number: 02785838.0
(22) Date of filing: 05.12.2002
(51) Int. Cl.: A01N 43/56, A01N 43/58, C07D 401/14

(54) **MICROBIOCIDAL N-PHENYL-N- 4-(4-PYRIDYL)-2-PYRIMIDIN-2-YL|-AMINE DERIVATIVES**
MIKROBIOZIDE N-PHENYL-N-¬4-(4-PYRIDYL)-2-PYRIMIDIN-2-YL|-AMIN-DERIVATE
DERIVES DE N-PHENYL-N- 4-(4-PYRIDYL)-2-PYRIMIDIN-2-YL|-AMINE, MICROBICIDES

(30) Priority: 07.12.2001 GB 0129391
(43) Date of publication of application: 03.11.2004
(73) Proprietor: Syngenta Participations AG, 4058 Basel (CH)
(72) Inventor: ACKERMANN, Peter, Syngenta Crop Protection AG, CH-4058 Basel (CH); STIERLI, Daniel, Syngenta Crop Protection AG, CH-4058 Basel (CH); JUNG, Pierre M. J., Syngenta Crop Protection AG, CH-4058 Basel (CH); MAIENFISCH, Peter, Syngenta Crop Protection AG, CH-4058 Basel (CH); CEDERBAUM, Fredrik E., Syngenta Crop Protection AG, CH-4058 Basel (CH); WENGER, Jean-Frederic, Syngenta Crop Protection AG, CH-4058 Basel (CH)
(74) Representative: Waterman, John Richard
(86) International application number: PCT/IB2002/005148
(87) International publication number: WO 2003/047347

(56) References cited:
- EP-A- 0 431 421
- WO-A-01/93682
- WO-A-95/09847
- WO-A-02/053560

## Description

The present invention relates to novel N-phenyl-[4-(4-pyridyl)-pyrimidin-2-yl]-amine derivatives, to a method of protecting plants against attack or infestation by phytopathogenic organisms, such as nematodes or insects or especially microorganisms, preferably fungi, bacteria and viruses, or combinations of two or more of these organisms, by applying a N-phenyl-[4-(4-pyridyl)-pyrimidin-2-yl]-amine derivative as specified hereinafter to a part and/or to the site of a plant, to the use of said derivative for protecting plants against said organisms, and to compositions comprising said derivative as the active component. The invention further relates to the preparation of these novel N-phenyl-[4-(4-pyridyl)-pyrimidin-2-yl]-amine derivatives.

Certain N-phenyl-4-(4-pyridyl)-2-pyrimidineamine derivatives have been described in the art as having pharmacological properties e.g. in the PCT patent applications WO 95/09851 and WO 95/09853, as tumor-inhibiting anti-cancer substances and in WO 97/19065 and WO98/18782 for the treatment of immune diseases.

Surprisingly, it has now been found that the new N-phenyl-[4-(4-pyridyl)-pyrimidin-2-yl]-amines are effective in plant protection and related areas, showing advantageous properties in the treatment of plant diseases caused by organisms.

The novel N-phenyl-[4-(4-pyridyl)-pyrimidin-2-yl]-amine derivatives according to the invention are those of the formula I 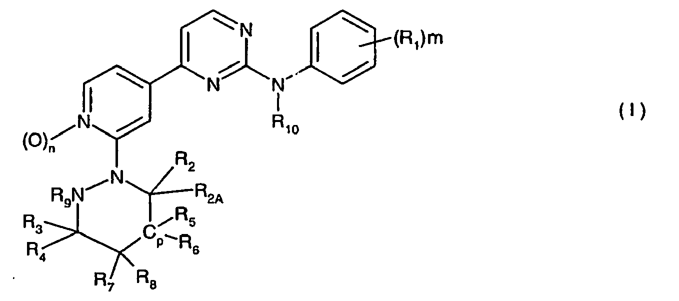 wherein
m is 0, 1,2 or 3;
n and p are independently of each other 0 or 1;
R₁ is halogen, optionally substituted alkyl, optionally substituted alkoxy, optionally substituted alkenyloxy, optionally substituted alkynyloxy, optionally substituted thioalkyl optionally substituted aryl, COOR₁₁, CONR₁₂R₁₃, S(O)_{q}R₁₄ , SO₂NR₁₅R₁₆ or NR₁₅ₐR₁₆ₐ; when there is more than on R₁ group, they may be the same or different;
q is 1 or 2;
R₂, R₂ₐ, R₃, R₄, R₅, R₆, R₇, R₈ are each independently hydrogen, optionally substituted alkyl, COR₁₇, COOR₁₈ or optionally substituted aryl, and in addition R₂ and R₃ may also independently be optionally substituted alkoxy, optionally substituted alkenyloxy, optionally substituted alkynyloxy, or optionally substituted alkylthio, COOR₁₉, CONR₂₀R₂₁, OH or SH;
R₆ and R₇ may also be independently halogen, optionally substituted alkoxy, optionally substituted alkenyloxy, optionally substituted alkynyloxy, optionally substituted alkenylamino, optionally substituted alkynylamino, optionally substituted alkylthio, optionally substituted cycloalkyl, optionally substituted heteroaryl, optionally substituted hetrocyclyl, optionally substituted cycloalkyloxy, OH, SH, N₃, NR₂₂R₂₃ or N(R₂₄)COR₂₅; or the ring members CR₃R₄ or CR₂R_{2A}. are independently of each other a carbonyl group (C=O) or a thonyl group (C=S);
or one or two of the adjacent pairs of groups R₉ and R₄, R₄ and R₈, R₅ and R₈, or, if p is zero, R_{2A} and R₈ may form a bond, provided that if there are 2 double bonds in the ring the double bonds are not adjacent each other;
or the pair of groups R₇ and R₈ or the pair of groups R₆ and R₇ together with the atom to which they are attached form a C₃-C₇ saturated ring;
R₉ is hydrogen, optionally substituted alkyl, optionally substituted alkenyl or optionally substituted alkynyl;
R₁₀ is hydrogen, C₁-C₄-alkyl, C₃-C₄-alkenyl, C₃-C₄-alkynyl, -CH₂OR₂₆, CH₂SR₂₇,-C(O)R₂₈, -C(O)OR₂₉, SO₂R₃₀, SOR₃₁ or SR₃₂;
R₂₆, R₂₇, R₂₈, R₂₉, R₃₀, R₃₁ R₃₂ are independently C₁-C₈-alkyl C₁-C₈-alkoxyalkyl, C₁-C₈ haloalkyl or phenylC₁-C₂-alkyl wherein the phenyl may be substituted by up to three groups selected from halo or C₁-C₄-alkyl,
R₁₁, R₁₂, R₁₃, R₁₄, R₁₅, R₁₆ R₁₅ₐ, R₁₆ₐ, R₁₇, R₁₈, R₁₉, R₂₀, R₂₁, R₂₂, R₂₃, R₂₄, and R₂₅ are independently H or optionally substituted alkyl; or a salt thereof.

One group of preferred compounds are of those of formula (I') which are compounds of formula I wherein
m is 0, 1, 2 or 3;
n and p are independently of each other 0 or 1;
R₁ is halogen, optionally substituted alkyl, optionally substituted alkoxy, optionally substituted thioalkyl optionally substituted aryl, COOR₁₁, CONR₁₂R₁₃, S(O)_{q}R₁₄,
SO₂NR₁₅R₁₆ or NR₁₅ₐR₁₆ₐ; when there is more than on R₁ group, they may be the same or different;
q is 1 or 2;
R₂, R₂ₐ, R₃, R₄, R₅, R₆, R₇, R₈ are each independently hydrogen, optionally substituted alkyl, COR₁₇, COOR₁₈ or optionally substituted aryl, and in addition R₂ and R₃ may also independently be optionally substituted alkoxy or optionally substituted alkylthio, COOR₁₉, CONR₂₀R₂₁, OH or SH;
R₆ and R₇ may also be independently halogen, optionally substituted alkoxy, optionally substituted alkylthio, OH, SH, N₃, NR₂₂R₂₃ or N(R₂₄)COR₂₅;
or the ring members CR₃R₄ or CR₂R_{2A} are independently of each other a carbonyl group (C=O) or a thiocarbonyl group (C=S);
or one or two of the adjacent pairs of groups R₉ and R₄, R₄ and R₈, R₅ and R₈, or, if p is zero, R_{2A} and R₈ may form a bond, provided that if there are 2 double bonds in the ring the double bonds are not adjacent each other;
or the pair of groups R₇ and R₈ together with the atom to which they are attached form a C₃-C₇ saturated ring;
R₉ is hydrogen or optionally substituted alkyl;
R₁₀ is hydrogen, C₁-C₄-alkyl, C₃-C₄-alkenyl, C₃-C₄-alkynyl, -CH₂OR₂₆, CH₂SR₂₇, - C(O)R₂₈, -C(O)OR₂₉, SO₂R₃₀, SOR₃₁ or SR₃₂;
R₂₆, R₂₇, R₂₈, R₂₉, R₃₀, R₃₁, R₃₂ are independently C₁-C₈-alkyl C₁-C₈-alkoxyalkyl, C₁-C₈ haloalkyl or phenylC₁-C₂-alkyl wherein the phenyl may be substituted by up to three groups selected from halo or C₁-C₄-alkyl,
R₁₁, R₁₂, R₁₃, R₁₄, R₁₅, R₁₆ R₁₅ₐ, R₁₆ₐ, R₁₇, R₁₈, R₁₉, R₂₀, R₂₁, R₂₂, R₂₃, R₂₄, and R₂₅ are independently H or optionally substituted alkyl; or a salt thereof.

In the context of the present specification alkyl as a group *per se* and as a structural element of hydroxyalkyl, thioalkyl, alkoxy, alkenyl, alkenyloxy, alkynyl alkynyloxy or haloalkoxy - is preferably C₁-C₆-alkyl, more preferably lower alkyl, and is linear i.e. methyl, ethyl, propyl, butyl, pentyl or hexyl, or branched, e.g. isopropyl, isobutyl, sec.-butyl, tert.-butyl, isopentyl, neopentyl or isohexyl. Lower alkyl is preferably methyl or ethyl.

Specific examples of alkenyl and alkynyl include allyl, 2-butenyl, 3-butenyl, propargyl, 2-butinyl and 3 butynyl.

When present, the optional substituents on an alkyl, alkenyl or alkynyl moiety include one or more of halogen, nitro, cyano, oxo (and acetals and ketals formed therefrom), C₃₋₇ cycloalkyl (itself optionally substituted with C₁₋₆ alkyl or halogen), C₅₋₇ cycloalkenyl (itself optionally substituted with C₁₋₆ alkyl or halogen), hydroxy, C₃₋₁₀ alkoxy, C₃₋₁₀ alkoxy(C₃₋₁₀)alkoxy, C₁₋₆ alkoxy-carbonyl(C₃₋₁₀)alkoxy, C₃₋₁₀ haloalkoxy, phenyl(C₁₋₄)alkoxy (where the phenyl group is optionally substituted by one or more of C₁₋₆ alkyl, C₁₋₆ alkoxy, C₁₋₆ haloalkyl, CN, nitro or halogen), C₃₋₇ cycloalkyloxy (where the cycloalkyl group is optionally substituted with C₁₋₆ alkyl or halogen), C₃₋₁₀ alkenyloxy, C₃₋₁₀ alkynyloxy, SH, C₃₋₁₀ alkylthio, C₃₋₁₀ haloalkylthio, phenyl(C₁₋₄) alkylthio (where the phenyl group is optionally substituted by one or more of C₁₋₆ alkyl, C₁₋₆ alkoxy, C₁₋₆ haloalkyl, CN, nitro or halogen), C₃₋₇ cycloalkylthio (where the cycloalkyl group is optionally substituted with C₁₋₆ alkyl or halogen), tri(C₁₋₄) alkylsilyl(C₁₋₆)alkylthio, phenylthio (where the phenyl group is optionally substituted by one or more of C₁₋₆ alkyl, C₁₋₆ alkoxy, C₁₋₆ haloalkyl, CN, nitro or halogen), C₁₋₆ alkylsulfonyl, C₁₋₆ haloalkylsulfonyl, C₁₋₆ alkylsulfinyl, C₁₋₆ haloalkylsulfinyl, phenylsulfonyl (where the phenyl group is optionally substituted by one or more of C₁₋₆ alkyl, C₁₋₆ alkoxy, C₁₋₆ haloalkyl, CN, nitro or halogen), tri(C₁₋₄)alkylsilyl, phenyldi(C₁₋₄) alkylsilyl, (C₁₋₄)alkyldiarylsilyl, triphenylsilyl, C₃₋₁₀ alkylcarbonyl, HO₂C, C₃₋₁₀ alkoxycarbonyl, aminocarbonyl, C₁₋₆ alkylaminocarbonyl, di(C₁₋₆ alkyl)-aminocarbonyl, N-(C₁₋₃ alkyl)-N-(C₁₋₃ alkoxy)aminocarbonyl, C₁₋₆ alkylcarbonyloxy, phenylcarbonyloxy (where the phenyl group is optionally substituted by one or more of C₁₋₆ alkyl, C₁₋₆ alkoxy, C₁₋₆ haloalkyl, CN, nitro or halogen), di(C₁₋₆)alkylaminocarbonyloxy, phenyl (itself optionally substituted by one or more of C₁₋₆ alkyl, C₁₋₆ alkoxy, C₁₋₆ haloalkyl, CN, nitro or halogen), naphthyl (itself optionally substituted by C₁₋₆ alkyl or halogen), heteroaryl (itself optionally substituted by C₁₋₆ alkyl or halogen), heterocyclyl (itself optionally substituted with C₁₋₆ alkyl or halogen), phenyloxy (where the phenyl group is optionally substituted by substituted by one or more of C₁₋₆ alkyl, C₁₋₆ alkoxy, C₁₋₆ haloalkyl, CN, nitro or halogen), naphthyloxy (where the naphthyl group is optionally substituted by C₁₋₆ alkyl or halogen), heteroaryloxy, (where the heteroaryl group is optionally substituted by C₁₋₆ alkyl or halogen), heterocyclyloxy (where the heterocyclyl group is optionally substituted with C₁₋₆ alkyl or halogen), amino, C₁₋₆ alkylamino, di(C₁₋₆) alkylamino, C₁₋₆ alkylcarbonylamino and N-(C₁₋₆)alkylcarbonyl-N-(C₁₋₆)alkylamino.

Preferred substituents on an alkyl, alkenyl or alkynyl moiety include one or more of halogen, nitro, cyano, C₃₋₇ cycloalkyl (itself optionally substituted with C₁₋₆ alkyl or halogen), C₅₋₇ cycloalkenyl (itself optionally substituted with C₁₋₆ alkyl or halogen), hydroxy, C₃₋₁₀ alkoxy, C₃₋₁₀ alkoxy(C₃₋₁₀)alkoxy, C₁₋₆ alkoxy-carbonyl(C₃₋₁₀)alkoxy, C₃₋₁₀ haloalkoxy, phenyl(C₁₋₄)alkoxy (where the phenyl group is optionally substituted by C₁₋₆ alkyl or halogen), C₃₋₇ cycloalkyloxy (where the cycloalkyl group is optionally substituted with C₁₋₆ alkyl or halogen), C₃₋₁₀ alkenyloxy, C₃₋₁₀ alkynyloxy, SH, C₃₋₁₀ alkylthio, C₃₋₁₀ haloalkylthio, phenyl(C₁₋₄)alkylthio (where the phenyl group is optionally substituted by C₁₋₆ alkyl or halogen), C₃-₇ cycloalkylthio (where the cycloalkyl group is optionally substituted with C₁₋₆ alkyl or halogen), tri(C₁₋₄)alkylsilyl(C₁₋₆)alkylthio, phenylthio (where the phenyl group is optionally substituted by C₁₋₆ alkyl or halogen), C₁₋₆ alkylsulfonyl, C₁₋₆ haloalkylsulfonyl, C₁₋₆ alkylsulfinyl, C₁₋₆ haloalkylsulfinyl, phenylsulfonyl (where the phenyl group is optionally substituted by C₁₋₆ alkyl or halogen), tri(C₁₋₄)alkylsilyl, phenyldi(C₁₋₄)alkylsilyl, (C₁₋₄)alkyldiarylsilyl, triphenylsilyl, C₃₋₁₀ alkylcarbonyl, HO₂C, C₃₋₁₀ alkoxycarbonyl, aminocarbonyl, C₁₋₆ alkylaminocarbonyl, di(C₁₋₆ alkyl)-aminocarbonyl, N-(C₁₋₃ alkyl)-N-(C₁₋₃ alkoxy)aminocarbonyl, C₁₋₆ alkylcarbonyloxy, phenylcarbonyloxy (where the phenyl group is optionally substituted by C₁₋₆ alkyl or halogen), di(C₁₋₆)alkylaminocarbonyloxy, phenyl (itself optionally substituted by C₁₋₆ alkyl or halogen), heteroaryl (itself optionally substituted by C₁₋₆ alkyl or halogen), heterocyclyl (itself optionally substituted with C₁₋₆ alkyl or halogen), phenyloxy (where the phenyl group is optionally substituted by C₁₋₆ alkyl or halogen), heteroaryloxy, (where the heteroaryl group is optionally substituted by C₁₋₆ alkyl or halogen), heterocyclyloxy (where the heterocyclyl group is optionally substituted with C₁₋₆ alkyl or halogen), amino, C₁₋₆ alkylamino, di(C₁₋₆) alkylamino, C₁₋₆ alkylcarbonylamino and N-(C₁₋₆)alkylcarbonyl-N-(C₁₋₆)alkylamino.

More preferred substituents on an alkyl, alkenyl and alkynyl moiety include one or more of halogen, nitro, cyano, C₃₋₇ cycloalkyl (itself optionally substituted with C₁₋₆ alkyl or halogen), hydroxy, C₃₋₁₀ alkoxy, C₃₋₁₀ alkoxy(C₃₋₁₀)alkoxy, C₁₋₆ alkoxy-carbonyl(C₃₋₁₀) alkoxy, C₃₋₁₀ haloalkoxy, SH, C₃₋₁₀ alkylthio, C₃₋₁₀ haloalkylthio, C₁₋₆ alkylsulfonyl, C₁₋₆ haloalkylsulfonyl, C₁₋₆ alkylsulfinyl, C₁₋₆ haloalkylsulfinyl, phenylsulfonyl (where the phenyl group is optionally substituted by C₁₋₆ alkyl or halogen), HO₂C, C₃₋₁₀ alkoxycarbonyl, aminocarbonyl, C₁₋₆ alkylaminocarbonyl, heteroaryl (itself optionally substituted by C₁₋₆ alkyl or halogen), heterocyclyl (itself optionally substituted with C₁₋₆ alkyl or halogen), phenyloxy (where the phenyl group is optionally substituted by C₁₋₆ alkyl or halogen), amino, C₁₋₆ alkylamino and di(C₁₋₆) alkylamino.

Aryl includes naphthyl, anthracyl, fluorenyl and indenyl but is preferably phenyl.

The term heteroaryl refers to an aromatic ring containing up to 10 atoms including one or more heteroatoms (preferably one or two heteroatoms) selected from O, S and N. Examples of such rings include pyridine, pyrimidine, furan, quinoline, quinazoline, pyrazole, thiophene, thiazole, oxazole and isoxazole.

The terms heterocycle and heterocyclyl refer to a non-aromatic ring containing up to 10 atoms including one or more (preferably one or two) heteroatoms selected from O, S and N. Examples of such rings include 1,3-dioxolane, tetrahydrofuran and morpholine.

When present, the optional substituents on heterocyclyl include C₁₋₆ alkyl as well as those optional substituents given above for an alkyl moiety.

Cycloalkyl includes cyclopropyl, cyclopentyl and cyclohexyl.

Cycloalkenyl includes cyclopentenyl and cyclohexenyl.

When present, the optional substituents on heteroaryl and aryl rings are selected, independently, from halogen, nitro, cyano, NCS-, C₁₋₆ alkyl, C₁₋₆ haloalkyl, C₁₋₆ alkoxy-(C₁₋₆)alkyl, C₂₋₆ alkenyl, C₂₋₆ haloalkenyl, C₂₋₆ alkynyl, C₃₋₇ cycloalkyl (itself optionally substituted with C₁₋₆ alkyl or halogen), C₅₋₇ cycloalkenyl (itself optionally substituted with C₁₋₆ alkyl or halogen), hydroxy, C₁₋₁₀ alkoxy, C₁₋₁₀ alkoxy(C₁₋₁₀)alkoxy, tri(C₁₋₄)alkyl-silyl(C₁₋₆)alkoxy, C₁₋₆ alkoxycarbonyl(C₁₋₁₀)alkoxy, C₁₋₁₀ haloalkoxy, aryl(C₁₋₄)alkoxy (where the aryl group is optionally substituted), C₃₋₇ cycloalkyloxy (where the cycloalkyl group is optionally substituted with C₁₋₆ alkyl or halogen), C₁₋₁₀ alkenyloxy, C₁₋₁₀ alkynyloxy, SH, C₁₋₁₀ alkylthio, C₁₋₁₀ haloalkylthio, aryl(C₁₋₄)alkylthio (where the aryl group may be further optionally substituted), C₃₋₇ cycloalkylthio (where the cycloalkyl group is optionally substituted with C₁₋₆ alkyl or halogen), tri(C₁₋₄) alkylsilyl(C₁₋₆)alkylthio, arylthio (where the aryl group is optionally substituted), C₁₋₆ alkylsulfonyl, C₁₋₆ haloalkylsulfonyl, C₁₋₆ alkylsulfinyl, C₁₋₆ haloalkylsulfinyl, arylsulfonyl (where the aryl group is optionally substituted), tri(C₁₋₄)alkylsilyl, aryldi(C₁₋₄) alkylsilyl, (C₁₋₄)alkyldiarylsilyl, triarylsilyl, C₁₋₁₀ alkylcarbonyl, HO₂C, C₁₋₁₀ alkoxycarbonyl, aminocarbonyl, C₁₋₆ alkylaminocarbonyl, di(C₁₋₆ alkyl)aminocarbonyl, N-(C₁₋₃ alkyl)-N-(C₁₋₃ alkoxy)aminocarbonyl, C₁₋₆ alkylcarbonyloxy, arylcarbonyloxy (where the aryl group is optionally substituted), di(C₁₋₆)alkylamino-carbonyloxy, aryl (itself optionally substituted), heteroaryl (which itself may be further optionally substituted), heterocyclyl (itself optionally substituted with C₁₋₆ alkyl or halogen), aryloxy (where the aryl group is optionally substituted), heteroaryloxy (where the heteroaryl group is optionally substituted), heterocyclyloxy (where the heterocyclyl group is optionally substituted with C₁₋₆ alkyl or halogen), amino, C₁₋₆ alkylamino, di(C₁₋₆) alkylamino, C₁₋₆ alkylcarbonylamino and N-(C₁₋₆)alkylcarbonyl-N-(C₁₋₆)alkylamino.

For substituted phenyl amd heteroaryl moieties it is preferred that one or more substituents are independently selected from halogen, C₁₋₆ alkyl, C₁₋₆ haloalkyl, C₁₋₆ alkoxy(C₁₋₆)alkyl, C₁₋₆ alkoxy, C₁₋₆ haloalkoxy, C₁₋₆ alkylthio, C₁₋₆ haloalkylthio, C₁₋₆ alkylsulfinyl, C₁₋₆ haloalkylsulfinyl, C₁₋₆ alkylsulfonyl, C₁₋₆ haloalkylsulfonyl, C₂₋₆ alkenyl, C₂₋₆ haloalkenyl, C₂₋₆ alkynyl, C₃₋₇ cycloalkyl, nitro, cyano, CO₂H, C₁₋₆ alkylcarbonyl, C₁₋₆ alkoxycarbonyl, R₃₃R₃₄N or R₃₅R₃₆NC(O); wherein R₃₃, R₃₄, R₃₅ and R₃₆ are, independently, hydrogen or C₁₋₆ alkyl.

In the context of the specification the term halogen is fluorine, bromine, iodine or preferably chlorine; similarly haloalkyl is preferably C₁-C₆-alkyl, more preferably lower alkyl, that is linear or branched and is substituted by one or more, for example in the case of halo-ethyl up to five, halogen atoms, especially fluorine (an example is trifluoromethyl.

Haloalkoxy is preferably C₁-C₆-alkoxy, more preferably lower alkoxy, that is linear or branched and that is substituted by one or more, for example in the case of halo-ethyl up to five, halogen atoms, especially fluorine; trifluoromethoxy and 1,1,2,2-tetrafluoroethoxy are especially preferred.

The moiety attached to the 2-position of the pyridine ring in the compounds of the invention, namely the moiety 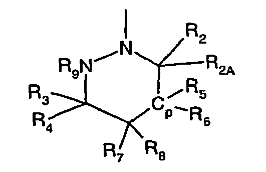 includes 5- and 6-membered ring systems, which are common in the art of heterocycles. Thus examples of the moieties include 2,4-dihydro-pyrazol-3-ones, 2,4-dihydropyrazole-3-thione, 1H-pyrazoles, 2H-pyridazin-3-ones, 4,5-dihydro-2H-pyridazin-3-ones, 1,2-dihydro-pyrazol-3-ones, 1,2-dihydro-pyrazole-3-thione, pyrazolidin-3-one, pyrazolidine-3-thione, 2H-pyridazin-3-thione and 4,5-dihydro-2H-pyridazin-3-thione.

More preferred ring systems for the moiety positioned at the 2-position of the pyridyl ring are those selected from the group comprising, 1H-pyrazoles, 2,4-dihydropyrazol-3-ones, 1,2-dihydro-pyrazol-3-ones, 4,5-dihydro-2H-pyridazin-3-ones.

The compounds of formula I can form acid addition salts, for example with inorganic acids, such as hydrochloric acid, sulfuric acid or a phosphoric acid, or with suitable organic carboxylic or sulfonic acids, for example aliphatic mono- or dicarboxylic acids, such as trifluoroacetic acid, acetic acid, propionic acid, glycolic acid, succinic acid, maleic acid, fumaric acid, hydroxymaleic acid, malic acid, tartaric acid, citric acid, oxalic acid or amino acids, such as arginine or lysine, aromatic carboxylic acids, such as benzoic acid, 2-phenoxy-benzoic acid, 2-acetoxy-benzoic acid, salicylic acid, 4-aminosalicylic acid, aromatic-aliphatic carboxylic acids, such as mandelic acid or cinnamic acid, heteroaromatic carboxylic acids, such as nicotinic acid or isonicotinic acid, aliphatic sulfonic acids, such as methane-, ethane- or 2-hydroxy-ethane-sulfonic acid, or aromatic sulfonic acids, for example benzene-, p-toluene- or naphthalene-2-sulfonic acid.

The pytidine-N-oxides of formula I can form acid addition salts with strong acids, such as hydrochloric acid, nitric acid, phosphoric acid or sulfonic acids, such as benzenesulfonic acid.

Formula I according to the invention shall include all the possible isomeric forms, as well as mixtures, e.g. racemic mixtures, and any mixtures of rotamers.

In view of the close relationship between the compounds of formula I in free form and in the form of their salts, including also salts that can be used as intermediates, for example in the purification of the compounds of formula I or in order to identify those compounds, herein-before and hereinafter any reference to the (free) compounds is to be understood as including also the corresponding salts, where appropriate and expedient.

Among the compounds of formula I according to the present invention the following groups of compounds are preferred. These groups are in any combination those wherein
n is 0;
p is 0 or 1;
m is 1, 2 or 3 or m is 1 and R₁ is preferably at the 3- or 4- position of the phenyl ring, preferably at the 3- position.
R₁ is selected from the group comprising halogen, C₁₋₃ haloalkoxy, CH(OH)R, COR, SO₂NRR', CH(NR'R")R, COORa or CONRbRc where Ra, Rb, Rc, R, R', R" are independently H or lower alkyl or
R₁ is selected from the group comprising chlorine, fluorine, trifluoromethyl, trifluoromethoxy, or 1,1,2,2-tetrafluoroethoxy, or
R₁ is 3-chloro;
R₂ is selected from the group comprising hydrogen, methyl, ethyl, methoxy, methoxymethyl, ethoxymethyl, or
R₂ is selected from the group comprising hydrogen, methyl or methoxy or
R₂ is methyl or
the ring members CR₂R_{2A} are a carbonyl group (C=O) or a thiocarbonyl group (C=S); R_{2A} is selected from the group comprising hydrogen, methyl; ethyl, methoxymethyl, ethoxymethyl, or
R_{2A} is hydrogen, methyl, or
R_{2A} forms a bond together with R₈;
R₃ and R₄ are independently selected from the group comprising hydrogen, methyl, ethyl, hydroxy, trifluoromethyl, methoxy, methoxymethyl, ethoxymethyl, or
R₃ and R₄ are independently selected from the group comprising hydrogen methyl or methoxy or
R₃ and R₄ are independently hydrogen or methyl or
the ring members CR₃R₄ are a carbonyl group (C=O) or a thiocarbonyl group (C=S); or R₄ together with either R₉ or R₈ forms a bond;
R₅, R₆, R₇, R₈ are each independently hydrogen, methyl, trifluoromethyl,
R₆ and R₇ may also be independently chloro, methoxy, ethoxy, diethylamine
R₇ may also be formyl or
the groups R₇ and R₈ together with the carbon atom to which they are attached form a cyclopropyl ring or
R₅ together with R₈ form a bond or
R₅, R₆, R₇, R₈ are each independently hydrogen, methyl;
R₉ is hydrogen or methyl;
R₁₀ is hydrogen, methyl, ethyl, allyl, propargyl, methoxymethyl, thiomethoxymethyl or ethoxymethyl, or
R₁₀ is hydrogen or methoxymethyl.

In a further group of preferred compounds R₂, R_{2A}, R₃, R₄, R₅, R₆, R₇, R₈ and R₉ independently of each other are hydrogen or methyl;

In a further group of preferred compounds R₇ is hydrogen, methyl, ethyl, allyl, propargyl, methoxymethyl, thiomethoxymethyl or ethoxymethyl, more preferably hydrogen or methoxymethyl.

Preferred individual compounds of the formula I are:
(3-Chloro-phenyl)-{4-[2-(3,4,5-trimethyl-pyrazol-1-yl)-pyridin-4-yl]-pyrimidin-2-yl}-amine;
(3-Chloro-phenyl)-{4-[2-(5-methoxy-3-methoxymethyl-pyrazol-1-yl)-pyridin-4-yl]-pyrimidin-2-yl}-amine;
(3-Chloro-phenyl)-{4-[2-(5-methoxy-3-methoxymethyl-4-methyl-pyrazol-1-yl)-pyridin-4-yl]-pyrimidin-2-yl}-amine;
(3-Chloro-phenyl)-{4-[2-(5-methoxy-4-methyl-pyrazol-1-yl)-pyridin-4-yl]-pyrimidin-2-yl}-amine;
(3-Chloro-phenyl)-{4-[2-(5-ethoxy-3,4-dimethyl-pyrazol-1-yl)-pyridin-4-yl]-pyrimidin-2-yl}-amine;
2-{4-[2-(3-Chloro-phenylamino)-pyrimidin-4-yl]-pyridin-2-yl}-5-methoxymethyl-1,4-dimethyl-1,2-dihydro-pyrazol-3-one;
2-(4- {2-[(3-Chloro-phenyl)-methoxymethyl-amino]-pyrimidin-4-yl}-pyridin-2-yl)-1,5-dimethyl-1,2-dihydro-pyrazol-3-one;
2-{4-[2-(3-Chloro-phenylamino)-pyrimidin-4-yl]-pyridin-2-yl}-1-ethyl-4,5-dimethyl-1,2-dihydro-pyrazol-3-one;
2-{4-[2-(3-Chloro-phenylamino)-pyrimidin-4-yl]-pyridin-2-yl}-1,4-dimethyl-1,2-dihydro-pyrazol-3-one;
2-{4-[2-(3-Chloro-phenylamino)-pyrimidin-4-yl]-pyridin-2-yl}-1,5-dimethyl-1,2-dihydro-pyrazol-3-one;
2-{4-[2-(3-Chloro-phenylamino)-pyrimidin-4-yl]-pyridin-2-yl}-5-methoxymethyl-4,4-dimethyl-2,4-dihydro-pyrazol-3-one;
2-{4-[2-(3-Chloro-phenylamino)-pyrimidin-4-yl]-pyridin-2-yl}-4,4-dimethyl-2,4-dihydro-pyrazol-3-one;
2-{4-[2-(3-Chloro-phenylamino)-pyrimidin-4-yl]-pyridin-2-yl}-4,4,5-trimethyl-2,4-dihydro-pyrazole-3-thione;
5-{4-[2-(3-Chloro-phenylamino)-pyrimidin-4-yl]-pyridin-2-yl}-7-methyl-5,6-diazaspiro[2.4]hept-6-en-4-one;
2-{4-[2-(3-Chloro-phenylamino)-pyrimidin-4-yl]-pyridin-2-yl}-4-ethyl-4,5-dimethyl-2,4-dihydro-pyrazol-3-one;
(3-Chloro-phenyl)-{4-[2-(5-methoxy-3-methyl-pyrazol-1-yl)-pyridin-4-yl]-pyrimidin-2-yl}-amine;
2-{4-[2-(3-Chloro-phenylanfino)-pyrimidin-4-yl]-pyridin-2-yl}-1,4,5-trimethyl-1,2-dihydro-pyrazol-3-one;
2-{4-[2-(3-Chloro-phenylamino)-pyrimidin-4-yl]-pyridin-2-yl}-4,4,5-trimethyl-2,4-dihydro-pyrazol-3-one;
2-{4-[2-(3-Chloro-phenylamino)-pyrimidin-4-yl]-pyridin-2-yl}-1,5-dimethyl-1,2-dihydro-pyrazol-3-one;
4,5-Dichloro-2-{4-[2-(3-chloro-phenyIamino)-pyrimidin-4-yl]-pyridin-2-yl }-2H-pyridazin-3-one;
2-{4-[2-(3-Chloro-phenylamino)-pyrimidin-4-yl]-pyridin-2-yl}-6-methyl-2H-pyridazin-3-one;
2-{4-[2-(3-Chloro-phenylamino)-pyrimidin-4-yl]-pyridin-2-yl}-6-methyl-4,5-dihydro-2H-pyridazin-3-one;
2-{4-[2-(3-Chloro-phenylamino)-pyrimidin-4-yl]-pyridin-2-yl}-6-Phenyl-4,5-dihydro-2H-pyridazin-3-one;
4-Chloro-2-{4-[2-(3-chloro-phenylamino)-pyrimidin-4-yl]-pyridin-2-yl}-5-ethoxy-2H-pyridazin-3-one;
4-Chloro-2-{4-[2-(3-chloro-phenylamino)-pyrimidin-4-yl]-pyridin-2-yl}-5-ethylsulfanyl-2H-pyridazin-3-one;
5-Azido-4-chloro-2-{4-[2-(3-chloro-phenylamino)-pyrimidin-4-yl]-pyridin-2-yl}-2H-pyridazin-3-one;
1-{4-[2-(3-Chloro-phenylamino)-pyrimidin-4-yl]-pyridin-2-yl}-2-methyl-pyrazolidin-3-one;
(3-Chloro-phenyl)-{4-[2-(5-methoxy-3,4-dimethyl-pyrazol-1-yl)-pyridin-4-yl]-pyrimidin-2-yl}-amine;
2-{4-[2-(3-Chloro-phenylamino)-pyrimidin-4-yl]-pyridin-2-yl}-5-methoxymethyl-1-methyl-1,2-dihydro-pyrazol-3-one;
2-{4-[2-(3-Chloro-phenylamino)-pyrimidin-4-yl]-pyridin-2-yl}-1,5-dimethyl-3-oxo-2,3-dihydro-1H-pyrazole-4-carbaldehyde;
5-Chloro-2-{4-[2-(3-chloro-phenylamino)-pyrimidin-4-yl]-pyridin-2-yl}-4-(oxetan-3-yloxy)-2H-pyridazin-3-one; and
4-Chloro-2-{4-[2-(3-chloro-phenylamino)-pyrimidin-4-yl]-pyridin-2-yl}-5-(tetrahydro-furan-2-ylmethoxy)-2H-pyridazin-3-one.

The compounds according to the invention may be prepared according to methods per se known in the art (this does mean, however, that, where novel compounds are produced, the respective process of manufacture is also novel). The procedures for the preparation of compounds of formula I may be outlined as follows:
A) reacting a compound of the formula (II) 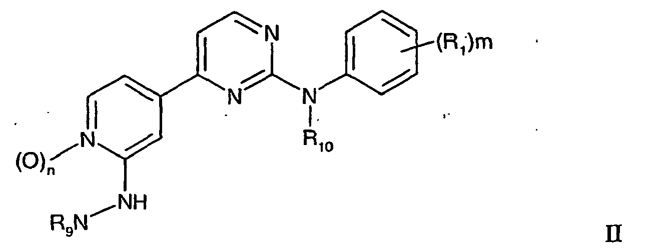 (or a salt thereof) with β-ketoester of the formula III to V under acid catalysed conditions 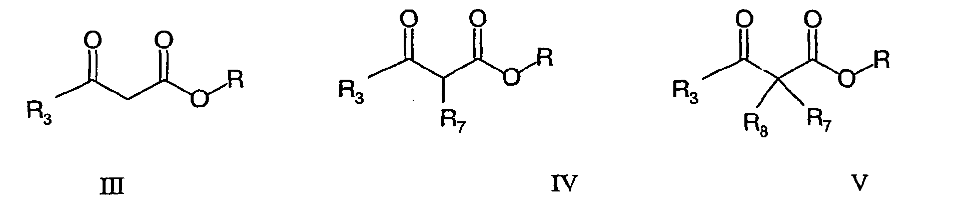 wherein R is H or optionally substituted alkyl and the other moieties in II to V have the meanings given for a compound of formula I thus obtaining a compound of the sub-formula Ia 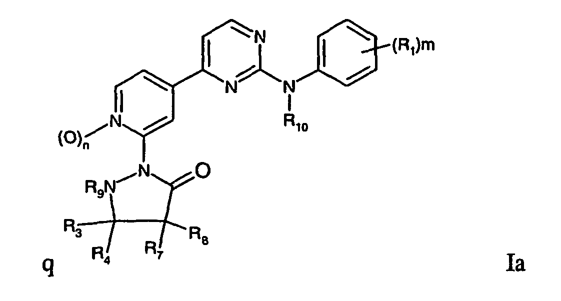
   Compounds of formula II may be prepared by the methods described in WO 01/93682 and illustrated in Synthesis Example 1.
B) reacting a compound of subformula Ia with a thionating reagent such as for example Lawesson reagent to obtain a compound of subformula Ib 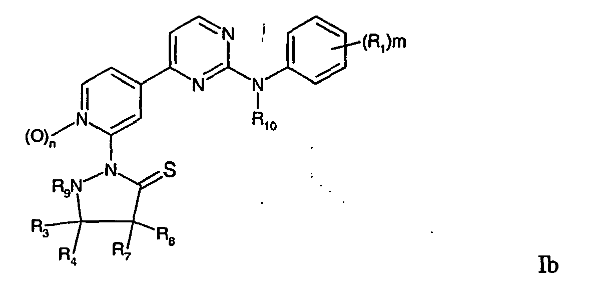
C) compounds of sub-formula Ia and Ib can be mono- or bis-alkylated to form compounds of structure I wherein p is 0 and all the other moieties have the meanings given for a compound of formula I
D) reacting a compound of the formula II (or a salt thereof) 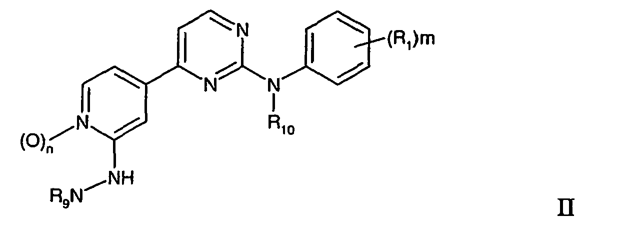 with a substituted acrylate of formula VI 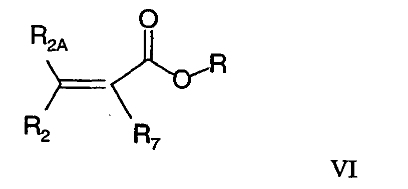 or with an alkyl propiolate of formula VII 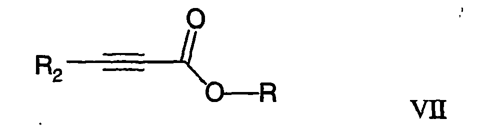 thus obtaining a compound of subformula Ic wherein the moieties have the meanings given for a compound of formula I 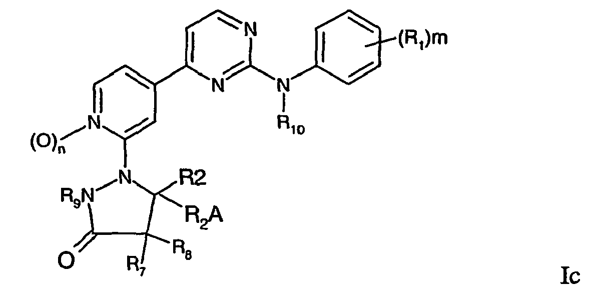
E) Conversion of the C=O group into the corresponding C=S group in subformula Ic can be achieved by reacting Ic with a thionating reagent such as e.g. Lawesson reagent thus producing compounds of subformula Id 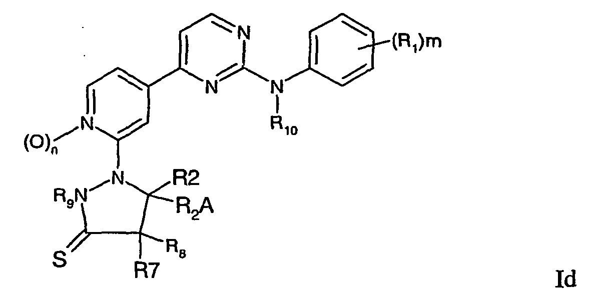
F) compounds of subformula Ic and Id can be alkylated to form compounds of structure I wherein p is 0, R3 is optionally substituted alkoxy or optionally substituted alkylthio and all the other moieties have the meanings given for a compound of formula I
G) reacting a compound of the formula II (or a salt thereof) 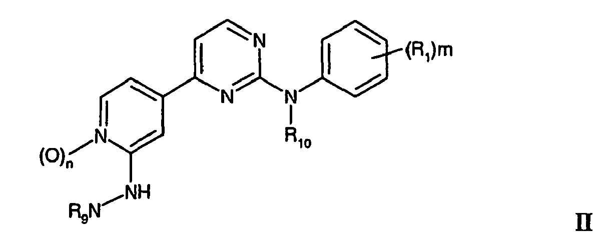 with a substituted 1,3 dicarbonyl compound of formula VIII 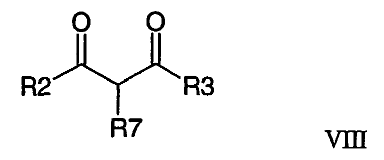
H) reacting a compound of the formula II (or a salt thereof) 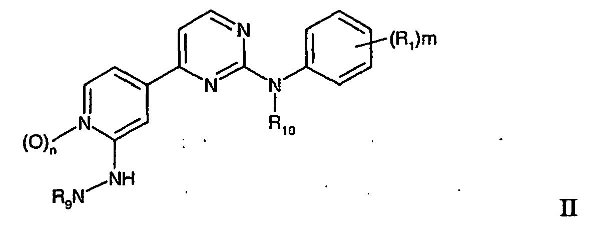 with a 1,4 dicarbonyl compounds of formula IX or X wherein R is H or optionally substituted alkyl 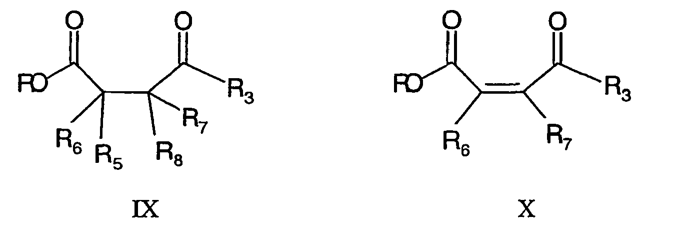
I) reacting a compound of the formula 1.6 (or a salt thereof) 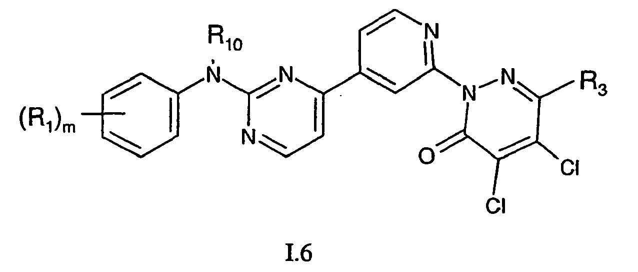 With a nucleophile to form compounds of formula I
   Compounds of forumla 1.6 are prepared by the methods of W Davey and D J Tivey, J Chem Soc 1958, p1230 and illustrated in Example 7.
J) reacting a compound of the formula XI (or a salt thereof) with a cyclic hydrazine system of formula XII in the presence of a base and a metal catalyst, such as palladium(II) or palladium(0) complexes commonly used for Buchwald -Hartwig aminations

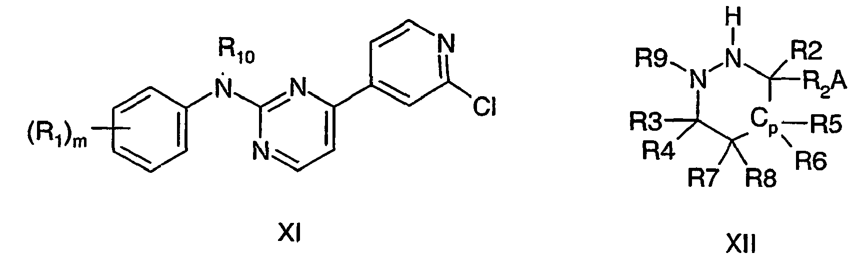

The R group moieties in compounds VIII, IX, X, XI and XII are as for those defined for compounds of formula I.

Compounds of formula III to XII inclusive are known compounds or may be prepared by compounds known processes.

The reaction types A to J and additional methods which can be applied per se or as analogous procedures for the synthesis of compounds of formula I are described for example in:
For 5-membred heterocylces:
   J. Bernstein; et al.; J. Am. Chem. Soc. 1947,69,1157;
   H. Priewe, A. Poljak; Chem. Ber. **1955,** 88, 1932;
   Patent Application CH 77-10606 19770831 (1982) ;
   EP 0 680 954 A2 ;
For 6 membred heterocycles
   Francis, John E.; Doebel, Karl. J.; Schutte, Paula M. Bachmann, Ernst F. Can. J. Chem. 1982, 60, 1214-1232. Sauter, Fritz; Stanetty, Peter; Blaschke, Alfred; Vyplel, Hermann J. Chem Miniprint, 4, 1981, 1087-1096. Mikhailovskii, A. Chem. Hetreocycl. Compd. (Engl. Trans.), 1998, 34, 2, 163-166. J. Med. Chem. 1999, 42, 6, 1088-1099.
   Krutosikova, Alzbeta; Dandarova, Miloslava; Konecny, Vaclav;
   Collect.Czech.Chem.Commun.; EN; 55; 11; 1990; 2707-2714.
   Benjamin, Louis E. Earley James V. Gilman Norman W. J. Heterocyclic. Chem. 1986, 23, 119-124.. Patent, Chem. Fabr. Schering, DE 406214. Gregory; Wiggins; J.Chem.Soc.; 1949; 2546, 2549. Lancelot, Jean-Charles; Robba, Max; Chem.Pharm.Bull. 36; 7; 1988; 2381-2385.
   Example on Phenylhydrazine : Bourel, Line; Tartar, Andre; Melnyk, Patricia; TELEAY; Tetrahedron Lett.; 37; 24; 1996; 4145-4148. Sawhney, S. N., Bhutani Sanjay, Vir, Indian J.Chem.Sect.B; 26, 5; 1987, 348-350. P. Coudert, J. Couquelet, P. Tronche J. *of Heterocyclic. Chem.* **1988**, 25, 799.
   The chloro atoms of formula I.6 can be substituted by aryl groups under palladium catalysed conditions according to procedures described in: Bert U. W. Maes, Omar 'kyek, Janez Komrlj, Guy L. F. Lemière, Eddy Esmans, Jef Rozenski, Roger A. Dommisse and Achiel Haemers Tetrahedron, **2001**, 57(7), 1323-1330.
   β-Ketoesters of formula III - V are known or can be prepared according to procedures described in:
   Hyoung R.K. Synlett 1998,789-791; Freskos J.N. Tetrahedron letters, Vol. 35, No. 6, pp. 835-838 (1994);
   J. Chem. Soc., Perkin Trans. 1, (4), 839-61 (1988); Bull. Soc. Chim. Belg., 94(7), 449-56 (1985);
   Collins D.J. Aust. J. Chem., **43**, 617-22 (1990);

Procedures for the alkylation of compounds of the subformula la to Id are described in the experimental section using Williamson conditions.
Conversion of C=O groups (in Ia and Ic) into C=S groups (subformulas Ib and Id) is described in the experimental section using Lawesson reagent under standard conditions or according to procedures given in
Ley, Steven V.; Leach, Andrew G.; Storer, R. Ian. J. Chem. Soc., Perkin Trans. 1 (2001), (4), 358-361.

Procedures for the palladium catalysed C-N linkage reaction (Burchwald-Hartwig amination) of compounds of formula XI with cyclic hydrazine ring systems of formula XII are given in the experimental part and are described in PCT/IB01/02821.

### Examples:

The subsequent examples are intended to illustrated the invention, without however limiting the scope thereof.

### Synthesis Example 1: (3-Chloro-phenyl)-[4-(2-hydrazino-pyridin-4-yl)-pyrimidin-2-yl]-amine

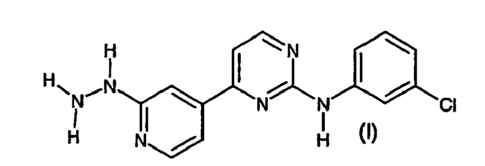

A mixture of (3-chloro-phenyl)-[4-(2-chloro-pyridin-4-yl)-pyrimidin-2-yl]-amine (4.8g, 0.015mol) in hydrazine (20ml, 0.41mol) is refluxed for 90 minutes. The reaction is poured into ethanol (300ml) with efficient stirring. The resulting precipitate is filtered with suction to yield the title compound, m.p. 201-203°C.

### Synthesis Example 2: 2-{4-[2-(3-Chloro-phenylamino)-pyrimidin-4-yl]-pyridin-2-yl}-5-methyl-2,4-dihydro-pylazol-3-one

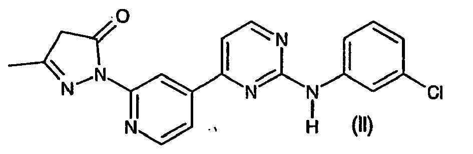

A mixture of (3-Chloro-phenyl)-[4-(2-hydrazino-pyridin-4-yl)-pyrimidin-2-yl]-amine (3.14g, 0.010mol) and Methyl acetoacetate (1.28g, 0.011mol) in EtOH (30ml) and Acetic acid (30ml) is stirred at reflux for one hour. At room temperature the resulting precipitate is filtered with suction to yield the title compound,(3.50g, 92%) m.p. 149-150°C.

### Synthesis Example 3:

A mixture of 2-{4-[2-(3-Chloro-phenylamino)-pyrimidin-4-yl]-pyr;din-2-yl}-5-methyl-2,4-dihydro-pyrazol-3-one (3.42g, 0.009mol), iodomethane (2.52g, 0.018mol) and potassium carbonate anhydrous (3.78g, 0.027mol) in DMF (30ml) is stirred at room temperature for three hours. After stirring the resulting is partitioned between ethyl acetate and water. The organic phase is separated, dried over magnesium sulfate, filtered and evaporated under reduced pressure. The residue is purified twice by silicagel chromatography to give all possible Isomers of the title compounds IIIa to IIIf. IIIa (0.10g, 2.8%) m.p. 185-188°C,
IIIb (0.29g, 8.1%) m.p. 163-166°C,
IIIc (0.52g, 14.1%) m.p. 192-194°C,
IIId (0.53g, 14.4%) m.p. 89-94°C,
IIIe (0.29g, 8.0%) m.p. 149-150°C,
IIIf (0.11g, 3.0%) m.p. 149-150°C,

### Synthesis Example IIIa: 2-{4-[2-(3-Chloro-phenylamino)-pyrimidin-4-yl]-pyridin-2-yl} 1-4.5-dimethyl-2,4-dihydro-pyrazol-3-one

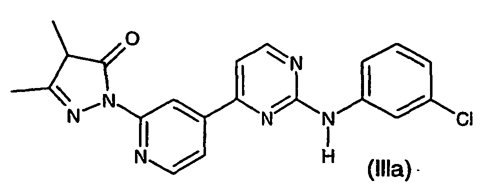

### Synthesis Example IIIb: 2-{4-[2-(3-Chloro-phenylamino)-pyrimidin-4-yl]-pyridin-2-yl}-4,4,5-trimethyl-2,4-dihydro-pyrazol-3-one

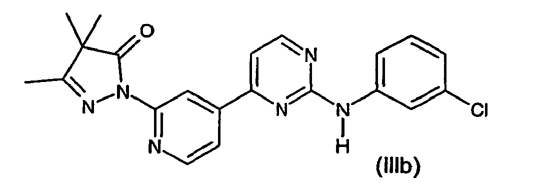

### Synthesis Example IIIc: 2-{4-[2-(3-Chloro-phenylamino)-pyrimidin-4-yl]-pyridin-2-yl}-1,5-dimethyl-1,2-dihydro-pyrazol-3-one

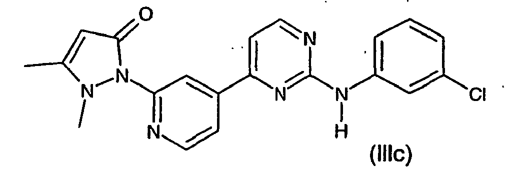

### Synthesis Example IIId: 2-{4-[2-(3-Chloro-phenylamino)-pyrimidin-4-yl]-pyridin-2-yl}-1,4,5-trimethyl-1,2-dihydro-pyrazol-3-one

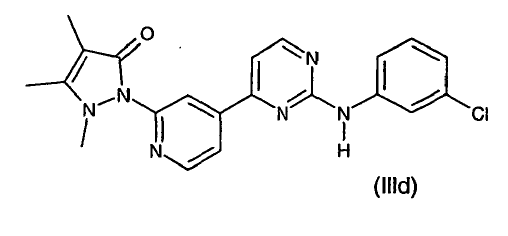

### Synthesis Example IIIe: (3-Chloro-phenyl)-{4-[2-(5-methoxy-3-methyl-pyrazol-1-yl)-pyridin-4-yl]-pyrimidin-2-yl}-amine

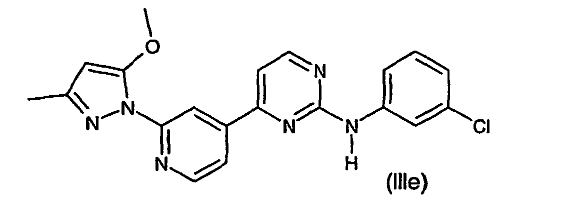

### Synthesis Example IIIf: (3-Chloro-phenyl)-{4-[2-(5-methoxy-3,4-dimethylpyrazol-1-yl)-pyridin-4-yl]-pyrimidin-2-yl}-amine

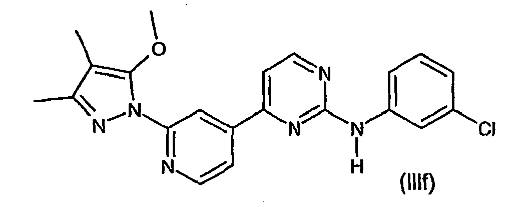

### Synthesis Example 4:

### Synthesis Example IVb: 2-{4-[2-(3-Chloro-phenylamino)-pyrimidin-4-yl]-pyridin-2-yl}-4,4,5-trimethyl-2,4-dihydro-pyrazole-3-thione

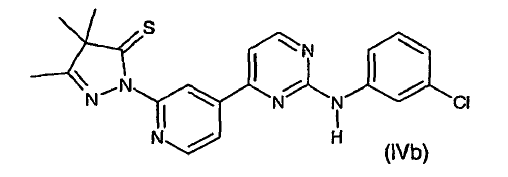

A mixture of 2-{4-[2-(3-Chloro-phenylamino)-pyrimidin-4-yl]-pyridin-2-yl}-4,4,5-trimethyl-2,4-dihydro-pyrazol-3-one (0.21g, 0.0005mol) and Lawesson reagent (0.22g 0.0005mol) in toluene (3ml) is stirred at 100°C for one hour. After cooling the resulting solution is directly purified by silicagel column chromatography to the title compounds (IVb) (0.19g, 88.1%) m.p. 167-168°C,

### Synthesis Example 5:

### Synthesis Example V: 1-{4-[2-(3-Chloro-phenylamino)-pyrimidin-4-yl]-pyridin-2-yl}-4,5-dihydro-1H-pyrazol-3-ol

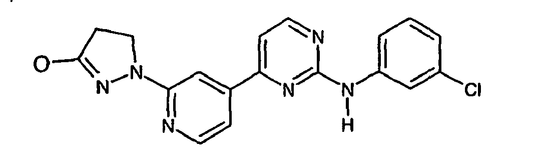

To a mixture of (3-Chloro-phenyl)-[4-(2-hydrazino-pyridin-4-yl)-pynmidin-2-yI]-amine (7.82g, 0.025mol) and Methyl acrylate (2.58g, 0.030mol) in tert BuOH (80ml) is added Potassium tert-butoxyde (5.6g, 0.05mol) in portions at 25°C. After stirring for two hours the resulting brown solution is poured in water (500ml), acidified with acetic acid and partitioned between ethyl acetate and water. The organic phase is separated, dried over magnesium sulfate, filtered and evaporated under reduced pressure. The residue is purified by crystallizing from acetone. The resulting precipitate is filtered with suction to yield the title compound. (1.55g, 16.9%) m.p. 222-226°C.

### Synthesis Example 6:

A mixture of 1-{4-[2-(3-Chloro-phenylamino)-pyrimidin-4-yl]-pyridin-2-yl}-4,5-dihydro-1H-pyrazol-3-ol (0.734g, 0.0020mol), iodomethane (175p1, 0.0028mo1) and potassium carbonate anhydrous (0.497g, 0.0036mol) in acetonitrile (4ml) and DMF (2ml) is stirred at 45°C for seven hours. After stirring the resulting is partitioned between ethyl acetate and water. The organic phase is separated, dried over magnesium sulfate, filtered and evaporated under reduced pressure. The residue is purified by silicagel chromatography to give both possible Isomers of the title compounds.
Vla (0.192g, 25.2%) mp. 143-144°C
Vlb (0.036g, 4.7%) mp. 202-205°C

### Synthesis Example VIa: 1-{4-[2-(3-Chloro-phenylamino)-pyrimidin-4-yl]-pyridin-2-yl}-2-methyl-pyrazolidin-3-on

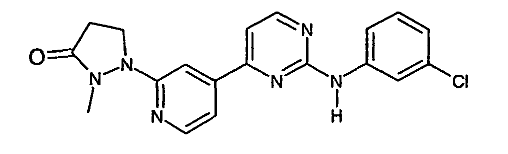

### Synthesis Example VIb: (3-Chloro-phenyl)-{4-[2-(3-methoxy-4,5-dihydro-pyrazol-1-yl)-pyridin-4-yl]-pyrimidin-2-yl}-amine

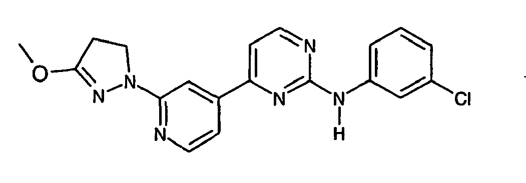

### Synthesis Example 7 :4,5-Dichloro-2-{4-[2-(3-chloro-phenylamino)-pyrimidin-4-yl]-pyridin-2-yl } -2H-pyridazin-3-one

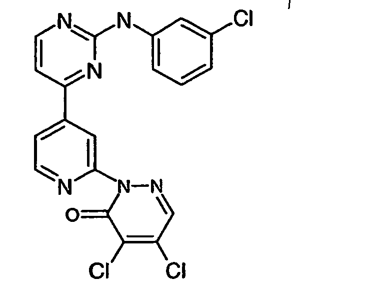

To a suspension of (3-Chloro-phenyl)-[4-(2-hydrazino-pyridin-4-yl)-pyrimidin-2-yl]-amine (5g) in acetic acid (80 mL) was added 2.8g of Mucochloric acid. The mixture was heated at 125°C for 4h. The solvent was concentrated and the crude was poured into water (500mL). The suspension was neutralised by addition of solid potassium carbonate until pH 7. The aqueous phase was extracted with ethyl acetate (3x 200 mL). The organic phases were combined, dried over MgSO4, and concentrated. Flash silica chromatography, eluting with ethyl acetate-tetrahydrofuran (1-0 to 1-1), afforded the title compound as a solid (3.11g, 44%). Mp 238-240°C, 1H NMR (DMSO-d6) 10.3 (1H, s, NH), 8.84 (1H, d, 5Hz), 8.76 (1H, d, 5Hz), 8.42 (1H, s), 8.38(1H, s), 8.28 (1H, dd, 2Hz, 5Hz), 8.04(1H, t, 2Hz), 7.74(1H, dd), 7.62(1H, d, 5Hz), 7.32(1H, t, 8Hz), 7.02(1H, dd, 2Hz, 8Hz).13CNMR (DMSO-d6) 160.3, 160.2, 159.9, 155.5, 153.5, 150.2, 146.6, 141.7, 136.7, 136.6, 134.0, 132.9, 130.1, 121.9, 121.1, 118.7, 118.2, 117.3, 109.3.

### Synthesis Example 8 : 2-{4-[2-(3-Chloro-phenylamino)-pyrimidin-4-yl]-pyridin-2-yl}-6-methyl-2H-pyridazin-3-one

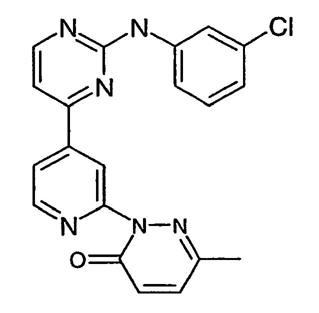

To a suspension of (3-Chloro-phenyl)-[4-(2-hydrazino-pyridin-4-yl)-pyrimidin-2-yl]-amine (2g) in acetic acid (40 mL) was acetate (3x 200 mL). ). The organic phase were combined, dried over MgSO4, and concentrated. Flash silica chromatography, eluting with ethyl acetate-tetrahydrofuran (1-0 to 1-1), afforded the title compound as a solid (1.39g, 55%). Mp 187-189°C, 1H NMR (DMSO-d6) 9.8 (1H, s, NH), 8.86 (1H, d, 5Hz), 8.80 (1H, d, 5Hz), 8.35 (1H, s), 8.28(1H, dd, 2Hz, 5Hz), 8.14 (1H, t, 2Hz), 7.73 (1H, m), 7.7.48(1H, d, 10Hz), 7.36(1H, t, 8Hz), 7.14(1H, d, 10Hz), 7.08(1H, dd, 1Hz, 7Hz), 2.39 (3H,s), 13CNMR (DMSO-d6) 160.8, 160.5, 160.3, 159.0, 154.8,150.5, 146.7, 145.4, 142.2, 135.4, 133.3, 130.8, 130.5, 121.5, 119.2,118.6, 117.7, 109.6, 20.58.

### Synthesis Example 9 :2-{4-[2-(3-Chloro-phenylamino)-pyrimidin-4-yl]-pyridin-2-yl}-6-methyl-4,5-dihydro-2H-pyridazin-3-one

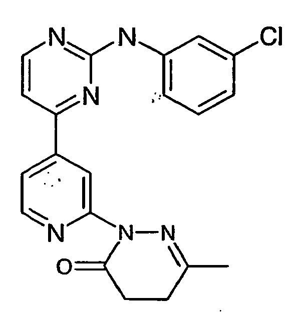

To a suspension of (3-Chloro-phenyl)-[4-(2-hydrazino-pyridin-4-yl)-pyhrnidin-2-yl]-amine (2g) in n-Butanol (40 mL) was added of 0.744g of levulinic acid. The mixture was heated at reflux. After 3h, the mixture was cooled at 0°C and the 4-({4-[2-(3-Chlorophenylamino)-pyrimidin-4-yl]-pyridin-2-yl}-hydrazono)-4-methyl-butyric acid (1.92g, 73%) was recovered by filtration. Mp 218-220°C, 1H NMR (DMSO-d6) 12.1 (1H, OHacid), 9.93 (1H, s, NH), 9.48 (1H, s, NH), 8.64 (1H, d, 5Hz), 8.24 (1H, d, 5Hz), 8.0 (1H, s), 7.76 (2H, m), 7.46 (1H, d, 5Hz), 7.38 (1H, dd, 2Hz, 5Hz), 7.30 (1H, t, 8Hz), 6.98 (1H, dd, 1Hz, 8Hz), 2.51 (4H, s), 1.92 (3H, s), 13C NMR (DMSO-d6 176.2, 164.6, 161.6, 161.2, 150.5, 150.1, 147.5, 144.0, 134.9, 132.1, 122.97, 120.13, 119.2, 113.7, 111.1, 105.9, 35.33 (CH2), 32.56(CH2), 18.09 (CH3), MS (ES-) 409 (M-1, 100), 819 (2M-1, 30). The 4-({4-[2-(3-Chloro-phenylamino)-pyrimidin-4-yl]-pyridin-2-yl}-hydrazono)-4-methyl-butyric acid (1.5g) was dissolved in acetic acid (40 mL). The solution was stirred at 110°C for 3h then the solution was poured in a mixture of water and ice (250 mL) and neutralised with a solution saturated of sodium hydrogenocarbonate until pH 7. The mixture was extracted with ethyl acetate (3x100 mL). The organic phase were combined, dried over MgSO4, and concentrated. Flash silica chromatography, eluting with ethyl acetate-tetrahydrofuran (3-1), afforded the title compound as a solid (0.7263g, 51%). Mp 189-192°C, 1H NMR (DMSO-d6) 10.07(1H, NH), 8.73 (1H, d, 5Hz), 8.69 (1H, d, 5Hz), 8.16 (1H, s), 8.06 (2H, m), 7.73 (1H, dd, 3Hz, 10Hz), 7.60(1H, d, 5Hz), 7.31 (1H, t, 8Hz), 7.03(1H, dd, 3hz, 8Hz), 2.63 (4H, m), 2.07 (3H, s). 13C NMR (DMSO-d6) 165.3, 160.3, 159.6, 159.3, 154.9, 153.9,149.0, 141.1, 132.5, 129.7, 120.7, 119.2, 117.8, 117.6, 116.7, 108.66, 26.4, 25.3, 22.0. MS (ES+) 393 (MH+, 100), 785 (2MH+, 60).

### Synthesis Example 10 : 2-{4-[2-(3-Chloro-phenylamino)-pyrimidin-4-yl]-pyridin-2-yl}-6-Phenyl-4,5-dihydro-2H-pyridazin-3-one

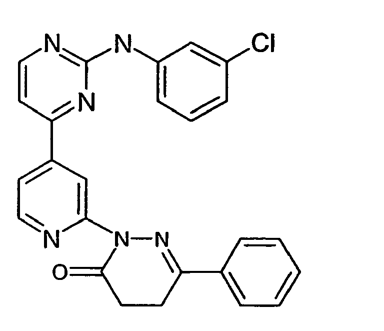

To a suspension of (3-Chloro-phenyl)-[4-(2-hydrazino-pyridin-4-yl)-pyrimidin-2-yl]-amine (2g) in n-Butanol (40 mL) was added of 1.14g of 3-benzoylpropionic acid. The mixture was heated at reflux. After 3h, the mixture was cooled at 0°C and the 4-({4-[2-(3-Chloro-phenylamino)-pyrimidin-4-yl]-pyridin-2-yl}-hydrazono)-4-phenyl-butyric acid (2.19g, 72%) was recovered by filtration. Mp 144-146°C, 1H NMR (DMSO-d6) .12.0 (1H, OH), 10.4 (1H, s, NH), 10.1 (1H, s, NH), 8.77 (1H, d, 5Hz), 8.42 (1H, d, 5Hz) 8.12 (1H, s), 8.06(1H, s), 7.80(3H, m), 7.6 (1H, d, 5Hz) 7.53 (1H, d, 5Hz), 7.45 (3H, m), 7.34 (1H, t, 8Hz), 7.08 (1H, m) 3.4 (2H, m), 2.95 (2H, m), MS (ES+) 473 (MH+, 100),. MS (ES-) 471 (M-1, 100). To a solution of 4-({4-[2-(3-Chloro-phenylamino)-pyrimidin-4-yl]-pyridin-2-yl}-hydrazono)-4-phenyl-butyric acid (0.48g) in tetrahydrofuranne (40 mL) was additionned N, N'-dicyclohexylcarbodiimide (0.23g) and 1-Hydroxybenzotriazole (0.1401g). The solution was stirred at reflux for 2h then the solvent was evaporated. The crude was chromatographied, eluting with ethyl acetate to gave the title compound as a solid (0.3366g, 78%). Mp 165-167°C, 1H NMR (CDC13) 9.6 (1H,s, NH), 8.76 (1H, d, 5Hz), 8.59 (1H, d, 5Hz), 8.21 (1H, s), 7.86 (4H, m), 7.44 (4H, m), 7.26(2H, m), 7.01 (1H, m), 3.18 (2H, t, 8Hz), 2.87 (2H, t, 8Hz). 13C NMR (CDC13) 166.3, 162.4, 160.4, 159.8, 154.8, 152.9, 150.1, 146.7, 140.9, 135.7, 134.9, 130.5, 130.3, 129.0, 126.7, 122.9, 120.1, 119.6, 118.8, 117.55, 109.6, 28.4, 23.6. MS (ES+) 455 (MH+, 100), 909 (2MH+, 10).

### Synthesis Example 11 : 4-Chloro-2-{4-[2-(3-chloro-phenylamino)-pyrimidin-4-yl]-pyridin-2-yl}-5-ethoxy-2H-pyridazin-3-one

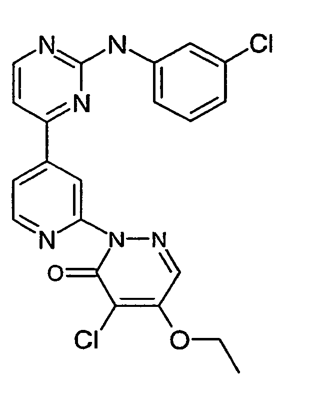

To a suspension of 4,5-Dichloro-2-{4-[2-(3-chloro-phenylamino)-pyrimidin-4-yl]-pyridin-2-yl}-2H-pyridazin-3-one (0.3g) in ethanol (10 mL) was added of 0.220 mg of potassium carbonate. The mixture was heated to reflux for 4h. The suspension was poured into water (50 mL) and extracted with ethyl acetate (2x100mL). The organic phase were combined, dried over MgSO₄, and concentrated. Flash silica chromatography, eluting with ethyl acetate-hexane (9 : 1), afforded the title compound as a solid (0.130g, 35%). Mp 196-198°C, , 1H NMR (CDCl₃) 8.80 (1H, d, 3Hz), 8.60 (1H, d, 6Hz), 8.42 (1H, s), 8.02 (2H, d, m), 7.93 (1H, s), 7.46 (1H, dd, 6Hz, 3Hz), 7.31 (1H,m), 7.29(1H, d, 3Hz), 7.28(1H, s), 4.43 (2H, q, 6Hz), 1.56 (3H, t, 6Hz).

### Synthesis Example 12 : 4-Chloro-2-{4-[2-(3-chloro-phenylamino)-pyrimidin-4-yl]-pyridin-2-yl}-5-ethylsulfanyl-2H-pyridazin-3-one.

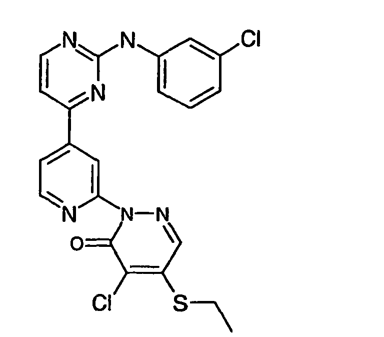

To a suspension of 4,5-Dichloro-2-{4-[2-(3-chloro-phenylamino)-pyrimidin-4-yl]-pyridin-2-yl}-2H-pyridazin-3-one (0.3g) in acetonitrile (10 mL) was added of 0.15 mL of ethylmercaptan and 0.280 mg of potassium carbonate. The mixture was heated to reflux for 2h. The suspension was filtered and the solid was washed with ethyl acetate to gave the title compound (0.220g, 46%).Mp 80-100°C, 1H NMR (DMSO-d6) 8.8 (1H, d, 3Hz), 859 (1H, d, 6Hz), 8.37 (1H, s, NH), 7.98 (1H, d, 6Hz, 3Hz), 7.92 (1H, t, 3Hz), 7.86 (1H, s), 7.46 (1H, d, 9Hz, 3Hz), 7.40 (1H,s), 7.27(2H, m), 7.04(1H, d, 9Hz), 3.12 (2H, q, 6Hz), 1.47 (3H, t, 6Hz).

### Synthesis Example 13 : 5-Azido-4-chloro-2-{4-[2-(3-chloro-phenylamino)-pyrimidin-4-yl]-pyridin-2-yl}-2H-pyridazin-3-one.

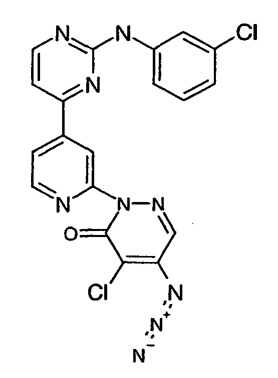

To a suspension of 4,5-Dichloro-2-{4-[2-(3-chloro-phenylamino)-pyrimidin-4-yl]-pyridin-2-yl}-2H-pyridazin-3-one (0.3g) in acetonitrile (10 mL) was added of 0.09g of sodium azide. The mixture was heated to reflux for 4h. The suspension was filtered to give the title compound as a solid (0.280g, 95%). Mp 184-186°C, 1H NMR (DMSO-d6) 10.1 (1H, s, NH), 8.75 (1H, d, 6Hz), 8.67 (1H, d, 3Hz), 8.28 (1H, s), 8.27 (1H, s), 8.18(1H, dd, 1Hz, 3Hz), 7.97 (1H, m), 7.65 (1H,dd), 7.57(1H, d, 6Hz), 7.25(1H, t, 9Hz), 6.94 (1H, dd).

### Synthesis Example 14 : 5-Chloro-4-cyclopropylamin-2-{4-[2-(3-chloro-phenylamino)-pyrimidin-4-yl]-pyridin-2-yl}-2H-pyridazin-3-one and 5-Cyclopropylamin-4-chloro-2-{4-[2-(3-chloro-phenylamino)-pyrimidin-4-yl]-pyridin-2-yl}-2H-pyridazin-3-one.

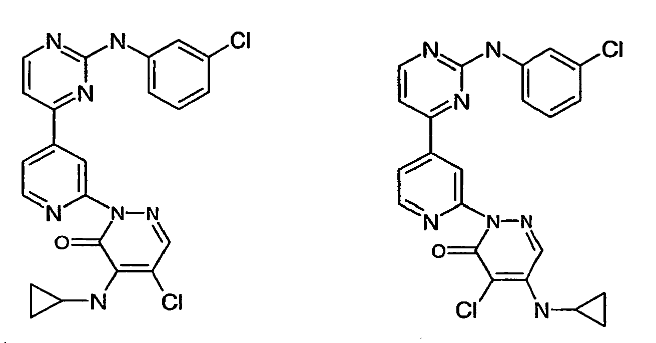

A suspension of 4,5-Dichloro-2-{4-[2-(3-chloro-phenylamino)-pyrimidin-4-yl]-pyridin-2-yl}-2H-pyridazin-3-one (0.3g) in cyclopropylamine (10 mL) was heated at reflux for 2h. The solvent was evaporated under vacuum. Flash silica chromatography, eluting with ethyl acetate-hexane (1 : 1), afforded the 5-Chloro-4-cyclopropylamin-2-{4-[2-(3-chloro-phenylamino)-pyrimidin-4-yl]-pyridin-2-yl}-2H-pyridazin-3-one as a solid (Mp 117-121°C, 0.082g, 26%) and the 5- cyclopropylamin -4- chloro -2-{4-[2-(3-chlorophenylamino)-pyrimidin-4-yl]-pyridin-2-yl}-2H-pyridazin-3-one (Mp 90-100°C, 0.180g, 58%) as a solid.

### Synthesis Example 15 : 5-Chloro-4-butanol-2-{4-[2-(3-chloro-phenylamino)-pyrimidin-4-yl]-pyridin-2-yl}-2H-pyridazin-3-one.

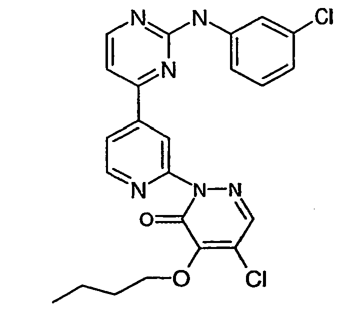

To a solution of butanol (0.18 mL) in tetrahydrofuran (40 mL) was added a solution of Lithium diisopropylamide (1.5 M, 1.3 mL) at room temperature. The solution was stirred for 15 minutes followed by the addition of 4,5-Dichloro-2-{4-[2-(3-chlorophenylamino)-pyrimidin-4-yl]-pyridin-2-yl}-2H-pyridazin-3-one (0.7g). The mixture was heated at 85°C for 1h. The suspension was poured into brine (200 mL) and extracted with ethyl acetate (3x100mL). The organic phase were combined, dried over MgSO4, and concentrated under vacuum. Flash silica chromatography, eluting with ethyl acetate-cyclohexane (1:1), afforded the title compound as a solid (0.4128g, 55%). Mp 118-127°C.

### Synthesis Example 16 : 1-{4-[2-(3-Chloro-phenylamino)-pyrimidin-4-yl]-pyridin-2-yl}-2-methyl-tetrahydro-pyridazine-3,6-dione.

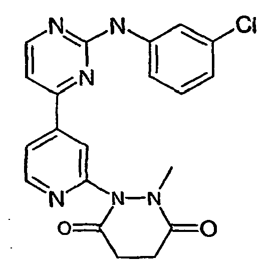

To a solution of succinic anhydride (2.16g) in chloroform (60 mL) 1.16 mL of methyl hydrazine were added at room temperature. The solution was stirred 2h at room temperature then heated at reflux for 1h. The solvent was evaporated. 1g of the obtained solid was dissolved in tetrahydrofuran (10 mL) followed by the addition of 1.55g of N,N'-dicyclohexylcarbodiimide and 1.01g of 1-hydroxybenzotriazole. The mixture was heated at reflux for 1 h. The mixture was cooled to 0°C and a solution of oxalic acid (0.617g) in methanol was added. The suspension was filtered and the solvent was evaporated. Flash silica chromatography, eluting with ethyl acetate-methanol (5%), afforded the 1-Methyl-tetrahydro-pyridazine-3,6-dione as a solid (0.4128g, 24%). 0.014g of Pd(dba)3 and xantphos (0.018g) were dissolved in toluene (2 mL). The mixture was stirred at room temperature for 20 minutes. Then the 1-Methyl-tetrahydro-pyridazine-3,6-dione, the (3-Chloro-phenyl)-[4-(2-chloro-pyridin-4-yl)-pyrimidin-2-yl]-amine (0.2g) and sodium terbutanolate (0.085g) were added. The mixture was heated at reflux for 2h. The suspension was poured into water (50 mL) and extracted with ethyl acetate (3x100mL). The organic phase was separated, dried over MgSO4, filtered and concentrated. Flash silica chromatography, eluting with ethyl acetate, afforded the title compound as a solid (0.169g, 65%). Mp 201-204°C.
The compounds in the following Tables further illustrate the invention

### Table 1

Compounds of the general structure I.1 wherein R₁ to R₁₀, m, n, and p correspond with a line of table A and B 1

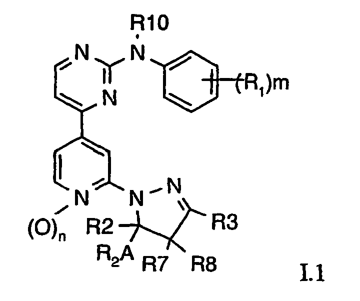

### Table 2

Compounds of the general structure 1.2 wherein R₁ to R₁₀, m, n, and p correspond with a line of table A and B2

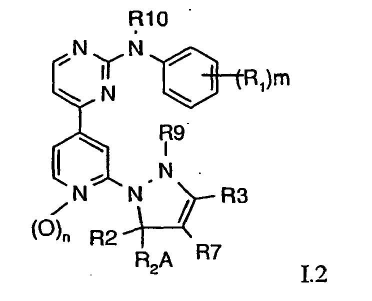

### Table 3

Compounds of the general structure I.3 wherein R₁ to R₁₀, m, n, and p correspond with a line of table A and B3

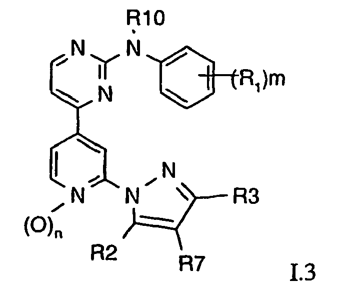

### Table 4

Compounds of the general structure I.4 wherein R₁ to R₁₀, m, n, and p correspond with a line of table A and B4

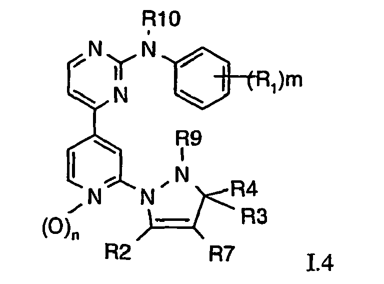

### Table 5

Compounds of the general structure I.5 wherein R₁ to R₁₀, m, n, and p correspond with a line of table A and B5

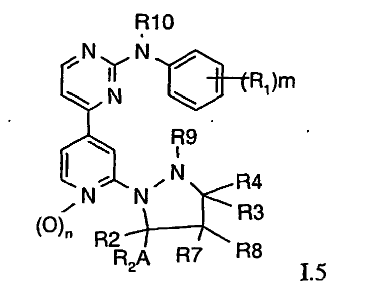

### Table 6

Compounds of the general structure 1.6 wherein R₁ to R₁₀, m, n, and p correspond with a line of table A and B6

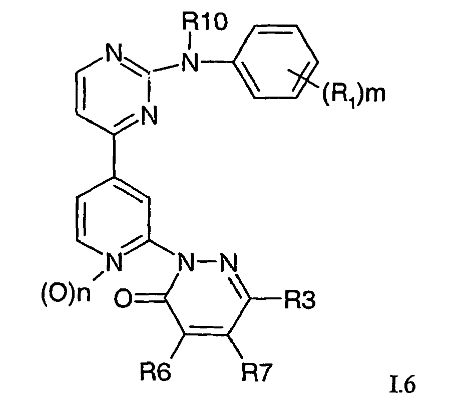

### Table 7

Compounds of the general structure I.7 wherein R₁ to R₁₀, m, n, and p correspond with a line of table A and B7

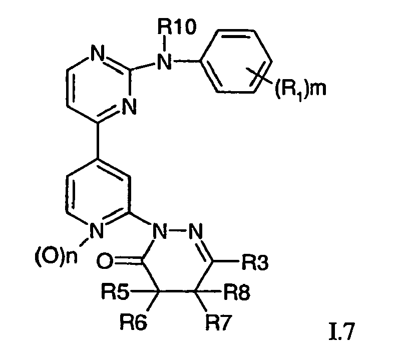

### Table 8

Compounds of the general structure I.8 wherein R1 to R10, m, n, and p correspond with a line of table A and B8

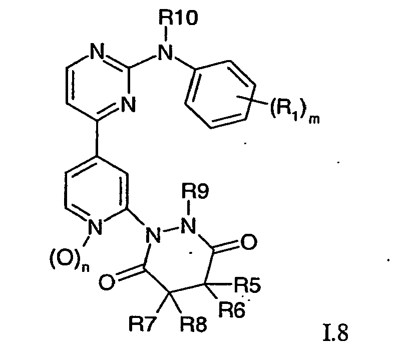

Compounds of general structure I are any combination of the definitions given in Table A and the appropriate Table B, wherein n, R₁₀, m and R₁ correspond with a line of Table A and wherein R₂ - R₉ and p correspond with a line of the appropriate Table B.

**Table A:**

| No. | n | R₁₀ | 2-R₁ | 3-R₁ | 4-R₁ | 5-R₁ | 6-R₁ |
|---|---|---|---|---|---|---|---|
| 001 | 0 | CH₃ | H | OH | H | H | F |
| 002 | 0 | CH₃ | H | OH | H | F | H |
| 003 | 0 | CH₃ | CH₃ | H | H | H | H |
| 004 | 0 | CH₃ | H | Cl | H | H | F |
| 005 | 0 | CH₃ | H | Cl | H | H | CH₃ |
| 006 | 0 | CH₃ | H | CH₃ | Cl | H | H |
| 007 | 0 | CH₃ | F | H | Cl | H | H |
| 008 | 0 | CH₃ | H | Cl | H | H | H |
| 009 | 0 | CH₃ | H | C(O)H | H | H | H |
| 010 | 0 | CH₃ | H | CH₂OH | H | H | H |
| 011 | 0 | CH₃ | H | CH(OH)CH₃ | H | H | H |
| 012 | 0 | CH₃ | H | F | H | H | H |
| 013 | 0 | CH₃ | H | CH₃ | H | H | H |
| 014 | 0 | CH₃ | H | H | H | CF₃ | H |
| 015 | 0 | CH₃ | H | H | H | OCF₃ | H |
| 016 | 0 | CH₃ | H | N(CH₃)₂ | H | H | F |
| 017 | 0 | CH₃ | H | SO₂N(CH₃)₂ | H | H | H |
| 018 | 0 | CH₃ | H | H | H | CONH₂ | H |
| 019 | 0 | CH₃ | H | H | H | OCH₂C 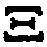 CH | H |
| 020 | 0 | CH₃ | H | SC₄H₉ | H | H | H |
| 021 | 0 | H | H | OH | H | F | H |
| 022 | 0 | H | H | OH | H | H | F |
| 023 | 0 | H | CH₃ | H | H | H | H |
| 024 | 0 | H | H | Cl | H | H | F |
| 025 | 0 | H | H | Cl | H | H | CH₃ |
| 026 | 0 | H | H | CH₃ | Cl | H | H |
| 027 | 0 | H | F | H | Cl | H | H |
| 028 | 0 | H | H | Cl | H | H | H |
| 029 | 0 | H | H | C(O)H | H | H | H |
| 030 | 0 | H | H | CH₂OH | H | H | H |
| 031 | 0 | H | H | CH(OH)CH₃ | H | H | H |
| 032 | 0 | H | H | F | H | H | H |
| 033 | 0 | H | H | CH₃ | H | H | H |
| 034 | 0 | H | H | H | H | CF₃ | H |
| 035 | 0 | H | H | H | H | OCF₃ | H |
| 036 | 0 | H | H | N(CH₃)₂ | H | H | F |
| 037 | 0 | H | H | SO₂N(CH₃)₂ | H | H | H |
| 038 | 0 | H | H | H | H | CONH₂ | H |
| 039 | 0 | H | H | H | H | OCH₂C 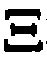 CH | H |
| 040 | 0 | H | H | SC₄H₉ | H | H | H |
| 041 | 0 | CH₂OCH₃ | H | OH | H | H | F |
| 042 | 0 | CH₂OCH₃ | H | OH | H | F | H |
| 043 | 0 | CH₂OCH₃ | CH₃ | H | H | H | H |
| 044 | 0 | CH₂OCH₃ | H | Cl | H | H | F |
| 045 | 0 | CH₂OCH₃ | H | Cl | H | H | CH₃ |
| 046 | 0 | CH₂OCH₃ | H | CH₃ | Cl | H | H |
| 047 | 0 | CH₂OCH₃ | F | H | Cl | H | H |
| 048 | 0 | CH₂OCH₃ | H | Cl | H | H | H |
| 049 | 0 | CH₂OCH₃ | H | C(O)H | H | H | H |
| 050 | 0 | CH₂OCH₃ | H | CH₂OH | H | H | H |
| 051 | 0 | CH₂OCH₃ | H | CH(OH)CH₃ | H | H | H |
| 052 | 0 | CH₂OCH₃ | H | F | H | H | H |
| 053 | 0 | CH₂OCH₃ | H | CH₃ | H | H | H |
| 054 | 0 | CH₂OCH₃ | H | H | H | CF₃ | H |
| 055 | 0 | CH₂OCH₃ | H | H | H | OCF₃ | H |
| 056 | 0 | CH₂OCH₃ | H | N(CH₃)₂ | H | H | F |
| 057 | 0 | CH₂OCH₃ | H | SO₂N(CH₃)₂ | H | H | H |
| 058 | 0 | CH₂OCH₃ | H | H | H | CONH₂ | H |
| 059 | 0 | CH₂OCH₃ | H | H | H | OCH₂C 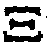 CH | H |
| 060 | 0 | CH₂OCH₃ | H | SC₄H₉ | H | H | H |
| 061 | 0 | CH₂OCH₃ | H | OH | H | H | F |
| 062 | 0 | CH₂OCH₃ | H | Cl | H | H | H |
| 063 | 0 | CH₂OCH₃ | H | C(O)H | H | H | H |
| 064 | 0 | CH₂OCH₃ | H | CH₂OH | H | H | H |
| 065 | 0 | CH₂OCH₃ | H | CH(OH)CH₃ | H | H | H |
| 066 | 0 | CH₂OCH₃ | H | F | H | H | H |
| 067 | 0 | CH₂OCH₃ | H | CH₃ | H | H | H |
| 068 | 0 | CH₂OCH₃ | H | H | H | CF₃ | H |
| 069 | 0 | CH₂OCH₃ | H | H | H | OCF₃ | H |
| 070 | 0 | CH₂OCH₃ | H | N(CH₃)₂ | H | H | F |
| 071 | 0 | CH₂OCH₃ | H | SO₂N(CH₃)₂ | H | H | H |
| 072 | 0 | CH₂OCH₃ | H | H | H | CONH₂ | H |
| 073 | 0 | CH₂SCH₃ | H | OH | H | H | F |
| 074 | 0 | CH₂SCH₃ | H | Cl | H | H | H |
| 075 | 0 | CH₂SCH₃ | H | C(O)H | H | H | H |
| 076 | 0 | CH₂SCH₃ | H | CH₂OH | H | H | H |
| 077 | 0 | CH₂SCH₃ | H | CH(OH)CH₃ | H | H | H |
| 078 | 0 | CH₂SCH₃ | H | F | H | H | H |
| 079 | 0 | CH₂SCH₃ | H | CH₃ | H | H | H |
| 080 | 0 | CH₂SCH₃ | H | H | H | CF₃ | H |
| 081 | 0 | CH₂SCH₃ | H | H | H | OCF₃ | H |
| 082 | 0 | CH₂SCH₃ | H | N(CH₃)₂ | H | H | F |
| 083 | 0 | CH₂SCH₃ | H | SO₂N(CH₃)₂ | H | H | H |
| 084 | 0 | CH₂SCH₃ | H | H | H | CONH₂ | H |
| 085 | 0 | CH₂CH=CH₂ | H | OH | H | H | F |
| 086 | 0 | CH₂CH=CH₂ | H | Cl | H | H | H |
| 087 | 0 | CH₂CH=CH₂ | H | C(O)H | H | H | H |
| 088 | 0 | CH₂CH=CH₂ | H | CH₂OH | H | H | H |
| 089 | 0 | CH₂CH=CH₂ | H | CH(OH)CH₃ | H | H | H |
| 090 | 0 | CH₂CH=CH₂ | H | F | H | H | H |
| 091 | 0 | CH₂CH=CH₂ | H | CH₃ | H | H | H |
| 092 | 0 | CH₂CH=CH₂ | H | H | H | CF₃ | H |
| 093 | 0 | CH₂CH=CH₂ | H | H | H | OCF₃ | H |
| 094 | 0 | CH₂CH=CH₂ | H | N(CH₃)₂ | H | H | F |
| 095 | 0 | CH₂CH=CH₂ | H | SO₂N(CH₃)₂ | H | H | H |
| 096 | 0 | CH₂CH=-CH₂ | H | H | H | CONH₂ | H |
| 097 | 0 | CH₂C 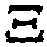 CH | H | OH | H | H | F |
| 098 | 0 | CH_{2C} 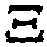 CH | H | Cl | H | H | H |
| 099 | 0 | CH₂C 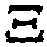 CH | H | C(O)H | H | H | H |
| 100 | 0 | CH₂C 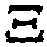 CH | H | CH₂OH | H | H | H |
| 101 | 0 | CH₂C 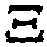 CH | H | CH(OH)CH₃ | H | H | H |
| 102 | 0 | CH₂C 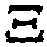 CH | H | F | H | H | H |
| 103 | 0 | CH₂C 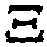 CH | H | CH₃ | H | H | H |
| 104 | 0 | CH₂C 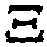 CH | H | H | H | CF₃ | H |
| 105 | 0 | CH₂C 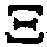 CH | H | H | H | OCF₃ | H |
| 106 | 0 | CH₂C 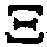 CH | H | N(CH₃)₂ | H | H | F |
| 107 | 0 | CH₂C 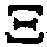 CH | H | SO₂N(CH₃)₂ | H | H | H |
| 108 | 0 | CH₂C 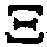 CH | H | H | H | CONH₂ | H |
| 109 | 0 | CH₂C 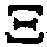 CH | H | OH | H | H | F |
| 110 | 0 | CH₂Ph | H | Cl | H | H | H |
| 111 | 0 | CH₂Ph | H | C(O)H | H | H | H |
| 112 | 0 | CH₂Ph - | H | CH₂OH | H | H | H |
| 113 | 0 | CH₂Ph | H | CH(OH)CH₃ | H | H | H |
| 114 | 0 | CH₂Ph | H | F | H | H | H |
| 115 | 0 | CH₂Ph | H | CH₃ | H | H | H |
| 116 | 0 | CH₂Ph | H | H | H | CF₃ | H |
| 117 | 0 | CH₂Ph | H | H | H | OCF₃ | H |
| 118 | 0 | CH₂Ph | H | N(CH₃)₂ | H | H | F |
| 119 | 0 | CH₂Ph | H | SO₂N(CH₃)₂ | H | H | H |
| 120 | 0 | H | H | Cl | CH₃ | H | H |
| 121 | 0 | H | H | Cl | CH₃ | H | H |
| 122 | 0 | H | H | Cl | OCH₃ | H | H |
| 123 | 0 | H | H | F | H | F | H |
| 124 | 0 | H | H | Cl | H | Cl | H |
| 125 | 0 | H | H | Br | H | H | H |

**Table B-1**

| | | | | | | |
|---|---|---|---|---|---|---|
| 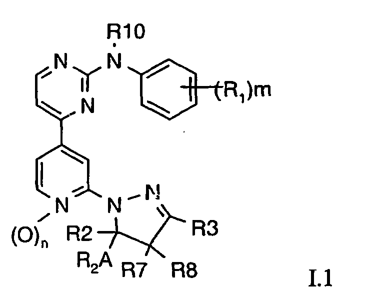 | | | | | | |

| No. | R₂ | R_{2A} | R₃ | R₇ | R₈ | R₉ |
|---|---|---|---|---|---|---|
| 01 | C=O | | CH₃ | CH₃ | CH₃ | |
| 02 | C=S | | CH₃ | CH₃ | H | |
| 03 | C=O | | CH₃ | CH₃ | CH₂CH₃ | |
| 04 | C=O | | CH₃ | CH₂-CH₂ | | |
| 05 | C=O | | CH₃ | CH₃ | Ph | |
| 06 | C=S | | CH₃ | CH₃ | CH₃ | |
| 07 | C=O | | H | CH₃ | CH₃ | |
| 08 | C=O | | CH₂OCH₃ | CH₃ | CH₃ | |
| 09 | C=O | | CH₃ | CH₃ | CH₂Ph | |
| 010 | C=O | | CH₃ | CH₂CH₂OC(O)CH₃ | H | |
| 011 | C=O | | CH₃ | CO₂Et | H | |
| 012 | C=O | | CH₃ | CHO | H | |
| 013 | C=O | | CH₃ | CF₃ | H | |
| 014 | C=O | | CF₃ | CF₃ | H | |
| 015 | C=O | | CF₃ | H | H | |
| 016 | C=O | | CH₂CH₃ | H | H | |
| 017 | C=O | | CH₂CH₃ | CH₃ | H | |
| 018 | C=O | | n-C₄H₉ | CH₃ | H | |
| 019 | C=O | | Ph | CH₃ | H | |
| 020 | C=S | | n-C₄H₉ | CH₃ | H | |
| 021 | C=S | | CH₃ | CH₃ | CH₂CH₃ | |
| 022 | C=S | | CH₃ | CH₂-CH₂ | | |
| 023 | C=S | | CH₃ | CH₃ | Ph | |
| 024 | C=S | | H | CH₃ | CH₃ | |
| 025 | C=S | | CH2OCH3 | CH₃ | CH₃ | |
| 026 | C=S | | CH₃ | CH₃ | CH₂Ph | |
| 027 | C=S | | CH₃ | CH₂CH₂OC(O)CH₃ | H | |
| 028 | C=S | | CH₃ | CO₂Et | H | |
| 029 | C=S | | CH₃ | CHO | H | |
| 030 | C=S | | CH₃ | CF₃ | H | |
| 031 | C=S | | CF₃ | CF₃ | H | |
| 032 | C=S | | CF₃ | H | H | |
| 033 | C=S | | CH₂CH₃ | H | H | |
| 034 | C=S | | CH₂CH₃ | CH₃ | H | |
| 035 | C=S | | Ph | CH₃ | H | |
| 036 | C=O | | CH₃ | CO₂Et | CH₃ | |
| 037 | C=O | | CH₃ | CHO | CH₃ | |
| 038 | C=O | | CH₃ | CF₃ | CH₃ | |
| 039 | C=O | | CF₃ | CF₃ | CH₃ | |
| 040 | C=O | | CF₃ | CH₃ | H | |
| 041 | C=O | | CH₂CH₃ | CF₃ | H | |
| 042 | C=O | | CH₂CH₃ | CH₃ | H | |
| 043 | C=S | | CH₃ | CO₂Et | CH₃ | |
| 044 | C=S | | CH₃ | CHO | CH₃ | |
| 045 | C=S | | CH₃ | CF₃ | CH₃ | |
| 046 | C=S | | CF₃ | CF₃ | CH₃ | |
| 047 | C=S | | CF₃ | CH₃ | H | |
| 048 | C=S | | CH₂CH₃ | CF₃ | H | |
| 049 | C=S | | CH₂CH₃ | CH₃ | H | |
| 050 | H | H | OCH₃ | H | H | |
| 051 | H | H | OCH₂Ph | H | H | |
| 052 | H | H | OCH₂CCH | H | H | |
| 053 | H | H | 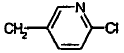 | H | H | |

**Table B-2**

| | | | | | |
|---|---|---|---|---|---|
| 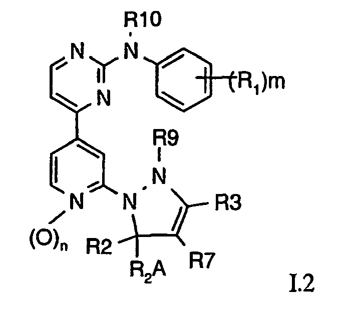 | | | | | |

| No. | R₂ | R_{2A} | R₃ | R₇ | R₉ |
|---|---|---|---|---|---|
| 01 | C=O | | CH₃ | C(O)₂Me | CH₃ |
| 02 | C=O | | CH₃ | CH₃ | CH₃ |
| 03 | C=O | | CH₃ | H | CH₃ |
| 04 | C=O | | CH₃ | CHO | CH₃ |
| 05 | C=O | | H | CH₃ | CH₃ |
| 06 | C=O | | CH₃ | CH₃ | CH₂-CH₃ |
| 07 | C=O | | CH₃ | CH₃ | CH₂PH |
| 08 | C=O | | CH₃ | H | CH₃ |
| 09 | C=O | | CH₂OCH₃ | CH₃ | CH₃ |
| 010 | C=O | | CH₃ | CH₃ | Ac |
| 011 | C=O | | CH₂OCH₃ | H | CH₃ |
| 012 | C=S | | CH₃ | H | CH₃ |
| 013 | C=S | | CH₃ | C(O)₂Me | CH₃ |
| 014 | C=S | | CH₃ | CH₃ | CH₃ |
| 015 | C=S | | CH₃ | CHO | CH₃ |
| 016 | C=S | | H | CH₃ | CH₃ |
| 017 | C=S | | CH₃ | CH₃ | CH₂-CH₃ |
| 018 | C=S | | CH₃ | CH₃ | CH₂PH |
| 019 | C=S | | CH₃ | H | CH₃ |
| 020 | C=S | | CH₂OCH₃ | CH₃ | CH₃ |
| 021 | C=S | | CH₃ | CH₃ | Ac |
| 022 | C=S | | CH₂OCH₃ | H | CH₃ |
| 023 | C=O | | CH₂Ph | CH₃ | CH₃ |
| 024 | C=O | | n-C₄H₉ | CH₃ | CH₃ |
| 025 | C=O | | CH₂CH₃ | CH₃ | CH₂CH₃ |
| 026 | C=O | | CH₂CH₃ | CH₃ | CH₃ |
| 027 | C=O | | CF₃ | n-C₄H₉ | CH₃ |
| 028 | C=O | | CH₂Ph | H | CH₃ |
| 029 | C=O | | n-C₄H₉ | H | CH₃ |
| 030 | C=O | | CH₂CH₃ | CH₂Ph | CH₂CH₃ |
| 031 | C=O | | CH₂CH₃ | H | CH₃ |
| 032 | C=O | | CF₃ | CH₃ | CH₃ |
| 033 | C=S | | CH₂Ph | CH₃ | CH₃ |
| 034 | C=S | | n-C₄H₉ | CH₃ | CH₃ |
| 035 | C=S | | CH₂CH₃ | CH₃ | CH₂CH₃ |
| 036 | C=S | | CH₂CH₃ | CH₃ | CH₃ |
| 037 | C=S | | CF₃ | n-C₄H₉ | CH₃ |
| 038 | C=S | | CH₂Ph | H | CH₃ |
| 039 | C=S | | n-C₄H₉ | H | CH₃ |
| 040 | C=S | | CH₂CH₃ | CH₂Ph | CH₂CH₃ |
| 041 | C=S | | CH₂CH₃ | H | CH₃ |
| 042 | C=S | | CF₃ | CH₃ | CH₃ |

**Table B-3**

| | | | |
|---|---|---|---|
| 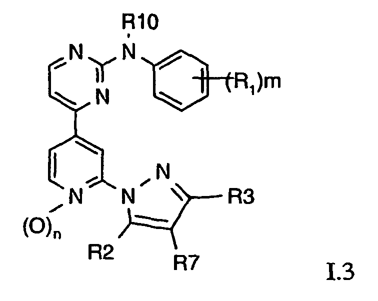 | | | |

| No. | R₂ | R₃ | R₇ |
|---|---|---|---|
| 01 | OCH3 | CH₃ | H |
| 02 | Oac | CH₃ | CH₃ |
| 03 | OC₂H₅ | CH₃ | CH₃ |
| 04 | OC₂H₅ | H | CH₃ |
| 05 | OC₂H₅ | CH₃ | H |
| 06 | OC₂H₅ | H | Ph |
| 07 | OC₂H₅ | CH₂OCH₃ | CH₃ |
| 08 | OC₂H₅ | CH₂OCH₃ | CH₂CH₃ |
| 09 | OH | CH₂OCH₃ | CH₃ |
| 010 | OH | CH₂OCH₃ | CH₂CH₃ |
| 011 | OH | CH₂OCH₃ | H |
| 012 | OCH3 | H | CH₃ |
| 013 | OCH3 | CH₂OCH₃ | CH₃ |
| 014 | OCH3 | CH₂OCH₃ | H |
| 015 | OH | CH₃ | CH₂CH₃ |
| 016 | OH | H | CH₃ |
| 017 | CH₃ | CH₃ | CH₃ |
| 018 | OAc | CH₃ | H |
| 019 | OH | CH₃ | H |
| 020 | OCH₂Ph | CH₃ | CH₃ |
| 021 | SCH3 | CH₃ | CH₃ |
| 022 | SCH3 | CH₃ | CH₂CH₃ |
| 023 | SCH3 | CH₃ | H |
| 024 | SCH3 | CH₃ | CH₂CH₃ |
| 025 | SCH3 | H | H |
| 026 | SCH3 | H | CH₃ |
| 027 | CH₃ | CH₃ | |
| 028 | CH₃ | CH₃ | CH₃ |
| 029 | CH₃ | CH₃ | C(O)₂Et |
| 030 | CH(CH₃)₂ | CH(CH₃)₂ | H |
| 031 | CH₃ | CH₃ | Cl |
| 032 | H | OCH3 | H |
| 033 | CH₂OCH₃ | H | C(O)₂Me |
| 034 | CH₂OCH₃ | H | CONHMe |
| 035 | c-C₃H₅ | CH₃ | H |
| 036 | I-C₃H₇ | CH₃ | C(O)₂Et |
| 037 | CH₃ | CH₃ | Ph |
| 038 | CH₃ | CF₃ | H |
| 039 | H | OH | H |
| 040 | 2,4-F₂-Ph | C(O)₂Me | H |
| 041 | 2,4-F₂-Ph | CONHMe | H |
| 042 | SCH3 | CH₂OCH₃ | CH₃ |
| 043 | SCH3 | CH₂OCH₃ | H |
| 044 | SH | CH₃ | CH₂CH₃ |
| 045 | SH | H | CH₃ |
| 046 | SCH3 | CH₃ | H |
| 047 | SCH₂Ph | CH₃ | CH₃ |
| 048 | SC₂H₅ | CH₃ | CH₃ |
| 049 | SC₂H₅ | H | CH₃ |
| 050 | SC₂H₅ | CH₃ . | H |
| 051 | SC₂H₅ | H | Ph |
| 052 | SC₂H₅ | CH₂OCH₃ | CH₃ |
| 053 | SC₂H₅ | CH₂OCH₃ | CH₂CH₃ |

**Table B-4**

| | | | | | |
|---|---|---|---|---|---|
| 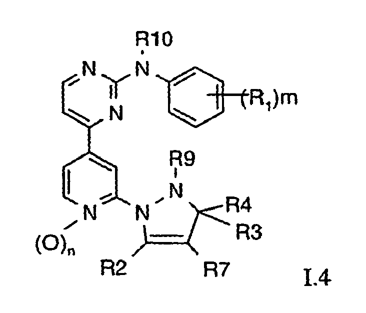 | | | | | |

| No. | R₂ | R₃ | R₄ | R₇ | R₉ |
|---|---|---|---|---|---|
| 01 | H | C=O | | H | CH₃ |
| 02 | H | C=O | | H | 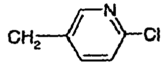 |
| 03 | H | C=O | | H | Benzyl |
| 04 | H | C=O | | H | CH₂C 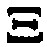 CH |
| 05 | CH₃ | C=O | | H | CH₃ |
| 06 | CH₃ | C=O | | H | CH₂CH₃ |
| 07 | CH₃ | C=O | | H | n-C₄H₉ |
| 08 | CH₃ | C=O | | H | CH₂Ph |
| 09 | CH₂CH₃ | C=O | | H | CH₃ |
| 010 | CH₂CH₃ | C=O | | H | CH₃ |
| 011 | Ph | C=O | | H | CH₃ |
| 012 | Ph | C=O | | H | CH₂CH₃ |
| 013 | Ph | C=O | | H | Ph |
| 014 | Ph | C=O | | H | n-C₄H₉ |
| 015 | H | C=S | | H | CH₃ |
| 016 | H | C=S | | H | Benzyl |
| 017 | H | C=S | | H | CH₂C 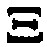 CH |
| 018 | CH₃ | C=S | | H | CH₃ |
| 019 | CH₃ | C=S | | H | CH₂CH₃ |
| 020 | CH₃ | C=S | | H | n-C₄H₉ |
| 021 | CH₃ | C=S | | H | CH₂Ph |
| 022 | CH₂CH₃ | C=S | | H | CH₃ |
| 023 | CH₂CH₃ | C=S | | H | CH₃ |
| 024 | Ph | C=S | | H | CH₃ |
| 025 | Ph | C=S | | H | CH₂CH₃ |
| 026 | Ph | C=S | | H | Ph |
| 027 | Ph | C=S | | H | n-C₄H₉ |

**Table B-5**

| | | | | | | | |
|---|---|---|---|---|---|---|---|
| 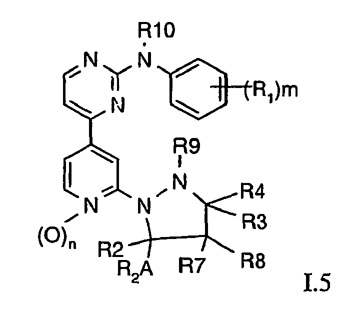 | | | | | | | |

| No. | R₂ | R_{2A} | R₃ | R₄ | R₇ | R₈ | R₉ |
|---|---|---|---|---|---|---|---|
| 01 | H | H | C=O | | H | H | CH₃ |
| 02 | H | H | C=O | | H | H | 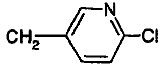 |
| 03 | H | H | C=O | | H | H | Benzyl |
| 04 | H | H | C=O | | H | H | CH₂C 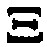 CH |
| 05 | CH₃ | H | C=O | | CH₃ | H | CH₂Ph |
| 06 | CH₃ | H | C=O | | CH₃ | H | CH₂C 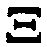 CH |
| 07 | CH₃ | H | C=O | | CH₃ | H | CH₂CH=CH₂ |
| 08 | CH₃ | H | C=O | | CH₃ | H | CH₃ |
| 09 | CH₃ | H | C=O | | CH₃ | H | CH₂CH₃ |
| 010 | CH₃ | H | C=O | | H | H | CH₂Ph |
| 011 | CH₃ | H | C=O | | H | H | CH₂C 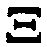 CH |
| 012 | CH₃ | H | C=O | | H | H | CH₂CH=CH₂ |
| 013 | CH₃ | H | C=O | | H | H | CH₃ |
| 014 | CH₃ | H | C=O | | H | H | CH₂CH₃ |
| 015 | CH₃ | CH₃ | C=O | | H | H | CH₂Ph |
| 016 | CH₃ | CH₃ | C=O | | H | H | CH₂C 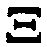 CH |
| 017 | CH₃ | CH₃ | C=O | | H | H | CH₂CH=CH₂ |
| 018 | CH₃ | CH₃ | C=O | | H | H | CH₃ |
| 019 | CH₃ | CH₃ | C=O | | H | H | CH₂CH₃ |
| 020 | CH₃ | H | C=S | | H | H | CH₂Ph |
| 021 | CH₃ | H | C=S | | H | H | CH₂C 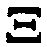 CH |
| 022 | CH₃ | H | C=S | | H | H | CH₂CH=CH₂ |
| 023 | CH₃ | H | C=S | | H | H | CH₃ |
| 024 | CH₃ | H | C=S | | H | H | CH₂CH₃ |
| 025 | CH₃ | CH₃ | C=S | | H | H | CH₂Ph |
| 026 | CH₃ | CH₃ | C=S | | H | H | CH₂C 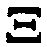 CH |
| 027 | CH₃ | CH₃ | C=S | | H | H | CH₂CH=CH₂ |
| 028 | CH₃ | CH₃ | C=S | | H | H | CH₃ |
| 029 | CH₃ | CH₃ | C=S | | H | H | CH₂CH₃ |
| 030 | C=O | | H | H | H | H | CH₃ |

**Table B-6**

| | | | |
|---|---|---|---|
| 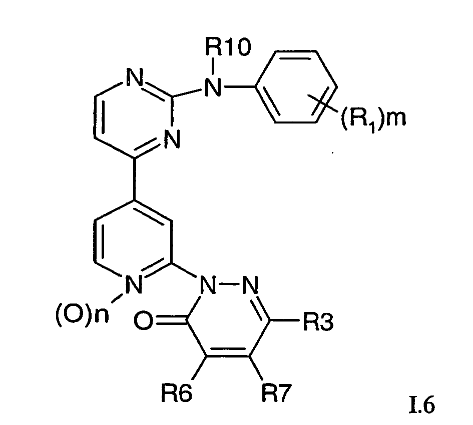 | | | |

| | R₃ | R₆ | R₇ |
|---|---|---|---|
| 1. | H | H | H |
| 2. | H | Cl | Cl |
| 3. | H | Cl | NHCH₃ |
| 4. | H | Cl | NHBu |
| 5. | H | Cl | N(CH₃)₂ |
| 6. | H | Cl | NBu₂ |
| 7. | H | Cl | NCH₃Bu |
| 8. | H | Cl | NEt₂ |
| 9. | H | Cl | NEtBu |
| 10. | H | Cl | SCH₃ |
| 11. | H | Cl | SBu |
| 12. | H | Cl | OCH₃ |
| 13. | H | Cl | OBu |
| 14. | H | Cl | CF₃ |
| 15. | H | Cl | OPh |
| 16. | H | Cl | CH₂OCH₃ |
| 17. | H | Cl | OCF₃ |
| 18. | H | Cl | OCF₂CF₃ |
| 19. | H | Cl | Ph |
| 20. | H | Cl | N₃ |
| 21. | H | H | I |
| 22. | H | H | CH₃ |
| 23. | H | H | Bu |
| 24. | H | H | OCH₃ |
| 25. | H | H | OBu |
| 26. | H | H | SCH₃ |
| 27. | H | H | SBu |
| 28. | H | H | NHCH₃ |
| 29. | H | H | NHBu |
| 30. | H | H | N(CH₃)₂ |
| 31. | H | H | NBu₂ |
| 32. | H | H | NCH₃Bu |
| 33. | H | H | NEt₂ |
| 34. | H | H | NEtBu |
| 35. | H | H | CF₃ |
| 36. | H | H | OPh |
| 37. | H | H | CH₂OCH₃ |
| 38. | H | H | OCF₃ |
| 39. | H | H | OCF₂CF₃ |
| 40. | H | H | Ph |
| 41. | H | H | N₃ |
| 42. | H | CH₃ | CH₃ |
| 43. | H | CH₃ | Bu |
| 44. | H | CH₃ | OCH₃ |
| 45. | H | CH₃ | OBu |
| 46. | H | CH₃ | SCH₃ |
| 47. | H | CH₃ | SBu |
| 48. | H | CH₃ | NHCH₃ |
| 49. | H | CH₃ | NHBu |
| 50. | H | CH₃ | N(CH₃)₂ |
| 51. | H | CH₃ | NBu₂ |
| 52. | H | CH₃ | NCH₃Bu |
| 53. | H | CH₃ | NEt₂ |
| 54. | H | CH₃ | NEtBu |
| 55. | H | CH₃ | CF₃ |
| 56. | H | CH₃ | OPh |
| 57. | H | CH₃ | CH₂OCH₃ |
| 58. | H | CH₃ | OCF₃ |
| 59. | H | CH₃ | OCF₂CF₃ |
| 60. | H | CH₃ | Ph |
| 61. | H | CH₃ | N₃ |
| 62. | H | nBu | CH₃ |
| 63. | H | nBu | Bu |
| 64. | H | nBu | OCH₃ |
| 65. | H | nBu | OBu |
| 66. | H | nBu | SCH₃ |
| 67. | H | nBu | SBu |
| 68. | H | nBu | NHCH₃ |
| 69. | H | nBu | NHBu |
| 70. | H | nBu | N(CH₃)₂ |
| 71. | H | nBu | NBu₂ |
| 72. | H | nBu | NCH₃Bu |
| 73. | H | nBu | NEt₂ |
| 74. | H | nBu | NEtBu |
| 75. | H | nBu | CF₃ |
| 76. | H | nBu | OPh |
| 77. | H | nBu | CH₂OCH₃ |
| 78. | H | nBu | OCF₃ |
| 79. | H | nBu | OCF₂CF₃ |
| 80. | H | nBu | Ph |
| 81. | H | nBu | N₃ |
| 82. | H | I | H |
| 83. | H | CH₃ | H |
| 84. | H | Bu | H |
| 85. | H | OCH₃ | H |
| 86. | H | OBu | H |
| 87. | H | SCH₃ | H |
| 88. | H | SBu | H |
| 89. | H | NHCH₃ | H |
| 90. | H | NHBu | H |
| 91. | H | N(CH₃)₂ | H |
| 92. | H | NBu₂ | H |
| 93. | H | NCH₃Bu | H |
| 94. | H | NEt₂ | H |
| 95. | H | NEtBu | H |
| 96. | H | CF₃ | H |
| 97. | H | OPh | H |
| 98. | H | CH₂OCH₃ | H |
| 99. | H | OCF₃ | H |
| 100. | H | OCF₂CF₃ | H |
| 101. | H | Ph | H |
| 102. | H | N₃ | H |
| 103. | H | CH₃ | CH₃ |
| 104. | H | Bu | CH₃ |
| 105. | H | OCH₃ | CH₃ |
| 106. | H | OBu | CH₃ |
| 107. | H | SCH₃ | CH₃ |
| 108. | H | SBu | CH₃ |
| 109. | H | NHCH₃ | CH₃ |
| 110. | H | NHBu | CH₃ |
| 111. | H | N(CH₃)₂ | CH₃ |
| 112. | H | NBu₂ | CH₃ |
| 113. | H | NCH₃Bu | CH₃ |
| 114. | H | NEt₂ | CH₃ |
| 115. | H | NEtBu | CH₃ |
| 116. | H | CF₃ | CH₃ |
| 117. | H | OPh | CH₃ |
| 118. | H | CH₂OCH₃ | CH₃ |
| 119. | H | OCF₃ | CH₃ |
| 120. | H | OCF₂CF₃ | CH₃ |
| 121. | H | Ph | CH₃ |
| 122. | H | N₃ | CH₃ |
| 123. | H | CH₃ | nBu |
| 124. | H | nBu | nBu |
| 125. | H | OCH₃ | nBu |
| 126. | H | OBu | nBu |
| 127. | H | SCH₃ | nBu |
| 128. | H | SBu | nBu |
| 129. | H | NHCH₃ | nBu |
| 130. | H | NHBu | nBu |
| 131. | H | N(CH₃)₂ | nBu |
| 132. | H | NBu₂ | nBu |
| 133. | H | NCH₃Bu | nBu |
| 134. | H | NEt₂ | nBu |
| 135. | H | NEtBu | nBu |
| 136. | H | CF₃ | nBu |
| 137. | H | OPh | nBu |
| 138. | H | CH₂OCH₃ | nBu |
| 139. | H | OCF₃ | nBu |
| 140. | H | OCF₂CF₃ | nBu |
| 141. | H | Ph | nBu |
| 142. | H | N₃ | nBu |
| 143. | H | NHCH₃ | Cl |
| 144. | H | NHBu | Cl |
| 145. | H | N(CH₃)₂ | Cl |
| 146. | H | NBu₂ | Cl |
| 147. | H | NCH₃Bu | Cl |
| 148. | H | NEt₂ | Cl |
| 149. | H | NEtBu | Cl |
| 150. | H | SCH₃ | Cl |
| 151. | H | SBu | Cl |
| 152. | H | OCH₃ | Cl |
| 153. | H | OBu | Cl |
| 154. | H | CF₃ | Cl |
| 155. | H | OPh | Cl |
| 156. | H | CH₂OCH₃ | Cl |
| 157. | H | OCF₃ | Cl |
| 158. | H | OCF₂CF₃ | Cl |
| 159. | H | Ph | Cl |
| 160. | H | N₃ | Cl |
| 161. | H | NHCH₃ | NHCH₃ |
| 162. | H | NHBu | NHBu |
| 163. | H | N(CH₃)₂ | N(CH₃)₂ |
| 164. | **H** | NBu₂ | NBu₂ |
| 165. | H | NCH₃Bu | NCH₃Bu |
| 166. | H | NEt₂ | NEt₂ |
| 167. | H | NEtBu | NEtBu |
| 168. | H | SCH₃ | SCH₃ |
| 169. | H | SBu | SBu |
| 170. | H | OCH₃ | OCH₃ |
| 171. | H | OBu | OBu |
| 172. | H | CF₃ | CF₃ |
| 173. | H | OPh | OPh |
| 174. | H | CH₂OCH | CH₂OCH₃ |
| 175. | H | OCF₃ | OCF₃ |
| 176. | H | OCF₂CF₃ | OCF₂CF₃ |
| 177. | H | Ph | Ph |
| 178. | H | N₃ | N₃ |
| 179. | CH₃ | H | H |
| 180. | CH₃ | Cl | Cl |
| 181. | CH₃ | Cl | NHCH₃ |
| 182. | CH₃ | Cl | NHBu |
| 183. | CH₃ | Cl | N(CH₃)₂ |
| 184. | CH₃ | Cl | NBu₂ |
| 185. | CH₃ | Cl | NCH₃Bu |
| 186. | CH₃ | Cl | NEt₂ |
| 187. | CH₃ | Cl | NEtBu |
| 188. | CH₃ | Cl | SCH₃ |
| 189. | CH₃ | Cl | SBu |
| 190. | CH₃ | Cl | OCH₃ |
| 191. | CH₃ | Cl | OBu |
| 192. | CH₃ | Cl | CF₃ |
| 193. | CH₃ | Cl | OPh |
| 194. | CH₃ | Cl | CH₂OCH₃ |
| 195. | CH₃ | Cl | OCF₃ |
| 196. | CH₃ | Cl | OCF₂CF₃ |
| 197. | CH₃ | Cl | Ph |
| 198. | CH₃ | Cl | N₃ |
| 199. | CH₃ | H | I |
| 200. | CH₃ | H | CH₃ |
| 201. | CH₃ | H | Bu |
| 202. | CH₃ | H | OCH₃ |
| 203. | CH₃ | H | OBu |
| 204. | CH₃ | H | SCH₃ |
| 205. | CH₃ | H | SBu |
| 206. | CH₃ | H | NHCH₃ |
| 207. | CH₃ | H | NHBu |
| 208. | CH₃ | H | N(CH₃)₂ |
| 209. | CH₃ | H | NBu₂ |
| 210. | CH₃ | H | NCH₃Bu |
| 211. | CH₃ | H | NEt₂ |
| 212. | CH₃ | H | NEtBu |
| 213. | CH₃ | H | CF₃ |
| 214. | CH₃ | H | OPh |
| 215. | CH₃ | H | CH₂OCH₃ |
| 216. | CH₃ | H | OCF₃ |
| 217. | CH₃ | H | OCF₂CF₃ |
| 218. | CH₃ | H | Ph |
| 219. | CH₃ | H | N3 |
| 220. | CH₃ | CH₃ | CH₃ |
| 221. | CH₃ | CH₃ | Bu |
| 222. | CH₃ | CH₃ | OCH₃ |
| 223. | CH₃ | CH₃ | OBu |
| 224. | CH₃ | CH₃ | SCH₃ |
| 225. | CH₃ | CH₃ | SBu |
| 226. | CH₃ | CH₃ | NHCH₃ |
| 227. | CH₃ | CH₃ | NHBu |
| 228. | CH₃ | CH₃ | N(CH₃)₂ |
| 229. | CH₃ | CH₃ | NBu₂ |
| 230. | CH₃ | CH₃ | NCH₃Bu |
| 231. | CH₃ | CH₃ | NEt₂ |
| 232. | CH₃ | CH₃ | NEtBu |
| 233. | CH₃ | CH₃ | CF₃ |
| 234. | CH₃ | CH₃ | OPh |
| 235. | CH₃ | CH₃ | CH₂OCH₃ |
| 236. | CH₃ | CH₃ | OCF₃ |
| 237. | CH₃ | CH₃ | OCF₂CF₃ |
| 238. | CH₃ | CH₃ | Ph |
| 239. | CH₃ | CH₃ | N₃ |
| 240. | CH₃ | nBu | CH₃ |
| 241. | CH₃ | nBu | Bu |
| 242. | CH₃ | nBu | OCH₃ |
| 243. | CH₃ | nBu | OBu |
| 244. | CH₃ | nBu | SCH₃ |
| 245. | CH₃ | nBu | SBu |
| 246. | CH₃ | nBu | NHCH₃ |
| 247. | CH₃ | nBu | NHBu |
| 248. | CH₃ | nBu | N(CH₃)₂ |
| 249. | CH₃ | nBu | NBu₂ |
| 250. | CH₃ | nBu | NCH₃Bu |
| 251. | CH₃ | nBu | NEt₂ |
| 252. | CH₃ | nBu | NEtBu |
| 253. | CH₃ | nBu | CF₃ |
| 254. | CH₃ | nBu | OPh |
| 255. | CH₃ | nBu | CH₂OCH₃ |
| 256. | CH₃ | nBu | OCF₃ |
| 257. | CH₃ | nBu | OCF₂CF₃ |
| 258. | CH₃ | nBu | Ph |
| 259. | CH₃ | nBu | N3 |
| 260. | CH₃ | I | H |
| 261. | CH₃ | CH₃ | H |
| 262. | CH₃ | Bu | H |
| 263. | CH₃ | OCH₃ | H |
| 264. | CH₃ | OBu | H |
| 265. | CH₃ | SCH₃ | H |
| 266. | CH₃ | SBu | H |
| 267. | CH₃ | NHCH₃ | H |
| 268. | CH₃ | NHBu | H |
| 269. | CH₃ | N(CH₃)₂ | H |
| 270. | CH₃ | NBu₂ | H |
| 271. | CH₃ | NCH₃Bu | H |
| 272. | CH₃ | NEt₂ | H |
| 273. | CH₃ | NEtBu | H |
| 274. | CH₃ | CF₃ | H |
| 275. | CH₃ | OPh | H |
| 276. | CH₃ | CH₂OCH₃ | H |
| 277. | CH₃ | OCF₃ | H |
| 278. | CH₃ | OCF₂CF₃ | H |
| 279. | CH₃ | Ph | H |
| 280. | CH₃ | N₃ | H |
| 281. | CH₃ | CH₃ | CH₃ |
| 282. | CH₃ | Bu | CH₃ |
| 283. | CH₃ | OCH₃ | CH₃ |
| 284. | CH₃ | OBu | CH₃ |
| 285. | CH₃ | SCH₃ | CH₃ |
| 286. | CH₃ | SBu | CH₃ |
| 287. | CH₃ | NHCH₃ | CH₃ |
| 288. | CH₃ | NHBu | CH₃ |
| 289. | CH₃ | N(CH₃)₂ | CH₃ |
| 290. | CH₃ | NBu₂ | CH₃ |
| 291. | CH₃ | NCH₃Bu | CH₃ |
| 292. | CH₃ | NEt₂ | CH₃ |
| 293. | CH₃ | NEtBu | CH₃ |
| 294. | CH₃ | CF₃ | CH₃ |
| 295. | CH₃ | OPh | CH₃ |
| 296. | CH₃ | CH₂OCH₃ | CH₃ |
| 297. | CH₃ | OCF₃ | CH₃ |
| 298. | CH₃ | OCF₂CF₃ | CH₃ |
| 299. | CH₃ | Ph | CH₃ |
| 300. | CH₃ | N₃ | CH₃ |
| 301. | CH₃ | CH₃ | nBu |
| 302. | CH₃ | Bu | nBu |
| 303. | CH₃ | OCH₃ | nBu |
| 304. | CH₃ | OBu | nBu |
| 305. | CH₃ | SCH₃ | nBu |
| 306. | CH₃ | SBu | nBu |
| 307. | CH₃ | NHCH₃ | nBu |
| 308. | CH₃ | NHBu | nBu |
| 309. | CH₃ | N(CH₃)₂ | nBu |
| 310. | CH₃ | NBu₂ | nBu |
| 311. | CH₃ | NCH₃Bu | nBu |
| 312. | CH₃ | NEt₂ | nBu |
| 313. | CH₃ | NEtBu | nBu |
| 314. | CH₃ | CF₃ | nBu |
| 315. | CH₃ | OPh | nBu |
| 316. | CH₃ | CH₂OCH₃ | nBu |
| 317. | CH₃ | OCF₃ | nBu |
| 318. | CH₃ | OCF₂CF₃ | nBu |
| 319. | CH₃ | Ph | nBu |
| 320. | CH₃ | N₃ | nBu |
| 321. | CH₃ | NHCH₃ | Cl |
| 322. | CH₃ | NHBu | Cl |
| 323. | CH₃ | N(CH₃)₂ | Cl |
| 324. | CH₃ | NBu₂ | Cl |
| 325. | CH₃ | NCH₃Bu | Cl |
| 326. | CH₃ | NEt₂ | Cl |
| 327. | CH₃ | NEtBu | Cl |
| 328. | CH₃ | SCH₃ | Cl |
| 329. | CH₃ | SBu | Cl |
| 330. | CH₃ | OCH₃ | Cl |
| 331. | CH₃ | OBu | Cl |
| 332. | CH₃ | CF₃ | Cl |
| 333. | CH₃ | OPh | Cl |
| 334. | CH₃ | CH₂OCH₃ | Cl |
| 335. | CH₃ | OCF₃ | Cl |
| 336. | CH₃ | OCF₂CF₃ | Cl |
| 337. | CH₃ | Ph | Cl |
| 338. | CH₃ | N₃ | Cl |
| 339. | CH₃ | NHCH₃ | NHCH₃ |
| 340. | CH₃ | NHBu | NHBu |
| 341. | CH₃ | N(CH₃)₂ | N(CH₃)₂ |
| 342. | CH₃ | NBu₂ | NBu₂ |
| 343. | CH₃ | NCH₃Bu | NCH₃Bu |
| 344. | CH₃ | NEt₂ | NEt₂ |
| 345. | CH₃ | NEtBu | NEtBu |
| 346. | CH₃ | SCH₃ | SCH₃ |
| 347. | CH₃ | SBu | SBu |
| 348. | CH₃ | OCH₃ | OCH₃ |
| 349. | CH₃ | OBu | OBu |
| 350. | CH₃ | CF₃ | CF₃ |
| 351. | CH₃ | OPh | OPh |
| 352. | CH₃ | CH₂OCH₃ | CH₂OCH₃ |
| 353. | CH₃ | OCF₃ | OCF₃ |
| 354. | CH₃ | OCF₂CF₃ | OCF₂CF₃ |
| 355. | CH₃ | Ph | Ph |
| 356. | CH₃ | N₃ | N₃ |
| 357. | nBu | H | H |
| 358. | nBu | Cl | Cl |
| 359. | nBu | Cl | NHCH₃ |
| 360. | nBu | Cl | NHBu |
| 361. | nBu | Cl | N(CH₃)₂ |
| 362. | nBu | Cl | NBu₂ |
| 363. | nBu | Cl | NCH₃Bu |
| 364. | nBu | Cl | NEt₂ |
| 365. | nBu | Cl | NEtBu |
| 366. | nBu | Cl | SCH₃ |
| 367. | nBu | Cl | SBu |
| 368. | nBu | Cl | OCH₃ |
| 369. | nBu | Cl | OBu |
| 370. | nBu | Cl | CF₃ |
| 371. | nBu | Cl | OPh |
| 372. | nBu | Cl | CH₂OCH₃ |
| 373. | nBu | Cl | OCF₃ |
| 374. | nBu | Cl | OCF₂CF₃ |
| 375. | nBu | Cl | Ph |
| 376. | nBu | Cl | N₃ |
| 377. | nBu | H | I |
| 378. | nBu | H | CH₃ |
| 379. | nBu | H | Bu |
| 380. | nBu | H | OCH₃ |
| 381. | nBu | H | OBu |
| 382. | nBu | H | SCH₃ |
| 383. | nBu | H | SBu |
| 384. | nBu | H | NHCH₃ |
| 385. | nBu | H | NHBu |
| 386. | nBu | H | N(CH₃)₂ |
| 387. | nBu | H | NBu₂ |
| 388. | nBu | H | NCH₃Bu |
| 389. | nBu | H | NEt₂ |
| 390. | nBu | H | NEtBu |
| 391. | nBu | H | CF₃ |
| 392. | nBu | H | OPh |
| 393. | nBu | H | CH₂OCH₃ |
| 394. | nBu | H | OCF₃ |
| 395. | nBu | H | OCF₂CF₃ |
| 396. | nBu | H | Ph |
| 397. | nBu | H | N₃ |
| 398. | nBu | CH₃ | CH₃ |
| 399. | nBu | CH₃ | Bu |
| 400. | nBu | CH₃ | OCH₃ |
| 401. | nBu | CH₃ | OBu |
| 402. | nBu | CH₃ | SCH₃ |
| 403. | nBu | CH₃ | SBu |
| 404. | nBu | CH₃ | NHCH₃ |
| 405. | nBu | CH₃ | NHBu |
| 406. | nBu | CH₃ | N(CH₃)₂ |
| 407. | nBu | CH₃ | NBu₂ |
| 408. | nBu | CH₃ | NCH₃Bu |
| 409. | nBu | CH₃ | NEt₂ |
| 410. | nBu | CH₃ | NEtBu |
| 411. | nBu | CH₃ | CF₃ |
| 412. | nBu | CH₃ | OPh |
| 413. | nBu | CH₃ | CH₂OCH₃ |
| 414. | nBu | CH₃ | OCF₃ |
| 415. | nBu | CH₃ | OCF₂CF₃ |
| 416. | nBu | CH₃ | Ph |
| 417. | nBu | CH₃ | N₃ |
| 418. | nBu | nBu | CH₃ |
| 419. | nBu | nBu | Bu |
| 420. | nBu | nBu | OCH₃ |
| 421. | nBu | nBu | OBu |
| 422. | nBu | nBu | SCH₃ |
| 423. | nBu | nBu | SBu |
| 424. | nBu | nBu | NHCH₃ |
| 425. | nBu | nBu | NHBu |
| 426. | nBu | nBu | N(CH₃)₂ |
| 427. | nBu | nBu | NBu₂ |
| 428. | nBu | nBu | NCH₃Bu |
| 429. | nBu | nBu | NEt₂ |
| 430. | nBu | nBu | NEtBu |
| 431. | nBu | nBu | CF₃ |
| 432. | nBu | nBu | OPh |
| 433. | nBu | nBu | CH₂OCH₃ |
| 434. | nBu | nBu | OCF₃ |
| 435. | nBu | nBu | OCF₂CF₃ |
| 436. | nBu | nBu | Ph |
| 437. | nBu | nBu | N3 |
| 438. | nBu | I | H |
| 439. | nBu | CH₃ | H |
| 440. | nBu | Bu | H |
| 441. | nBu | OCH₃ | H |
| 442. | nBu | OBu | H |
| 443. | nBu | SCH₃ | H |
| 444. | nBu | SBu | H |
| 445. | nBu | NHCH₃ | H |
| 446. | nBu | NHBu | H |
| 447. | nBu | N(CH₃)₂ | H |
| 448. | nBu | NBu₂ | H |
| 449. | nBu | NCH₃Bu | H |
| 450. | nBu | NEt₂ | H |
| 451. | nBu | NEtBu | H |
| 452. | nBu | CF₃ | H |
| 453. | nBu | OPh | H |
| 454. | nBu | CH₂OCH₃ | H |
| 455. | nBu | OCF₃ | H |
| 456. | nBu | OCF₂CF₃ | H |
| 457. | nBu | Ph | H |
| 458. | nBu | N₃ | H |
| 459. | nBu | CH₃ | CH₃ |
| 460. | nBu | Bu | CH₃ |
| 461. | nBu | OCH₃ | CH₃ |
| 462. | nBu | OBu | CH₃ |
| 463. | nBu | SCH₃ | CH₃ |
| 464. | nBu | SBu | CH₃ |
| 465. | nBu | NHCH₃ | CH₃ |
| 466. | nBu | NHBu | CH₃ |
| 467. | nBu | N(CH₃)₂ | CH₃ |
| 468. | nBu | NBu₂ | CH₃ |
| 469. | nBu | NCH₃Bu | CH₃ |
| 470. | nBu | NEt₂ | CH₃ |
| 471. | nBu | NEtBu | CH₃ |
| 472. | nBu | CF₃ | CH₃ |
| 473. | nBu | OPh | CH₃ |
| 474. | nBu | CH₂OCH₃ | CH₃ |
| 475. | nBu | OCF₃ | CH₃ |
| 476. | nBu | OCF₂CF₃ | CH₃ |
| 477. | nBu | Ph | CH₃ |
| 478. | nBu | N₃ | CH₃ |
| 479. | nBu | CH₃ | nBu |
| 480. | nBu | nBu | nBu |
| 481. | nBu | OCH₃ | nBu |
| 482. | nBu | OBu | nBu |
| 483. | nBu | SCH₃ | nBu |
| 484. | nBu | SBu | nBu |
| 485. | nBu | NHCH₃ | nBu |
| 486. | nBu | NHBu | nBu |
| 487. | nBu | N(CH₃)₂ | nBu |
| 488. | nBu | NBu₂ | nBu |
| 489. | nBu | NCH₃Bu | nBu |
| 490. | nBu | NEt₂ | nBu |
| 491. | nBu | NEtBu | nBu |
| 492. | nBu | CF₃ | nBu |
| 493. | nBu | OPh | nBu |
| 494. | nBu | CH₂OCH₃ | nBu |
| 495. | nBu | OCF₃ | nBu |
| 496. | nBu | OCF₂CF₃ | nBu |
| 497. | nBu | Ph | nBu |
| 498. | nBu | N₃ | nBu |
| 499. | nBu | NHCH₃ | Cl |
| 500. | nBu | NHBu | Cl |
| 501. | nBu | N(CH₃)₂ | Cl |
| 502. | nBu | NBu₂ | Cl |
| 503. | nBu | NCH₃Bu | Cl |
| 504. | nBu | NEt₂ | Cl |
| 505. | nBu | NEtBu | Cl |
| 506. | nBu | SCH₃ | Cl |
| 507. | nBu | SBu | Cl |
| 508. | nBu | OCH₃ | Cl |
| 509. | nBu | OBu | Cl |
| 510. | nBu | CF₃ | Cl |
| 511. | nBu | OPh | Cl |
| 512. | nBu | CH₂OCH₃ | Cl |
| 513. | nBu | OCF₃ | Cl |
| 514. | nBu | OCF₂CF₃ | Cl |
| 515. | nBu | Ph | Cl |
| 516. | nBu | N₃ | Cl |
| 517. | nBu | NHCH₃ | NHCH₃ |
| 518. | nBu | NHBu | NHBu |
| 519. | nBu | N(CH₃)₂ | N(CH₃)₂ |
| 520. | nBu | NBu₂ | NBu₂ |
| 521. | nBu | NCH₃Bu | NCH₃Bu |
| 522. | nBu | NEt₂ | NEt₂ |
| 523. | nBu | NEtBu | NEtBu |
| 524. | nBu | SCH₃ | SCH₃ |
| 525. | nBu | SBu | SBu |
| 526. | nBu | OCH₃ | OCH₃ |
| 527. | nBu | OBu | OBu |
| 528. | nBu | CF₃ | CF₃ |
| 529. | nBu | OPh | OPh |
| 530. | nBu | CH₂OCH₃ | CH₂OCH₃ |
| 531. | nBu | OCF₃ | OCF₃ |
| 532. | nBu | OCF₂CF₃ | OCF₂CF₃ |
| 533. | nBu | Ph | Ph |
| 534. | nBu | N₃ | N₃ |
| 535. | Ph | H | H |
| 536. | Ph | CH₃ | CH₃ |
| 537. | Ph | CH₃ | H |
| 538. | Ph | H | CH₃ |
| 539. | CH₂OCH₃ | H | H . |
| 540. | CH₂OCH₃ | CH₃ | CH₃ |
| 541. | CH₂OCH₃ | CH₃ | H |
| 542. | CH₂OCH₃ | H | CH₃ |
| 543. | CF₃ | H | H |
| 544. | CF₃ | CH₃ | CH₃ |
| 545. | CF₃ | CH₃ | H |
| 546. | CF₃ | H | CH₃ |
| 547. | OH | H | H |
| 548. | OH | CH₃ | CH₃ |
| 549. | OH | CH₃ | H |
| 550. | OH | H | CH₃ |
| 551. | OH | CHO | H |
| 552. | OH | CHO | CH₃ |
| 553. | OH | H | CF₃ |
| 554. | OH | CF₃ | H |
| 555. | OH | CF₃ | CF₃ |
| 556. | OH | CH₂OCH₃ | CH₃ |
| 557. | OH | CH₃ | CH₂OCH₃ |
| 558. | OH | CH₂OCH₃ | H |
| 559. | OH | H | CH₂OCH₃ |
| 560. | H | CHO | H |
| 561. | CH₃ | CHO | H |
| 562. | CF₃ | CHO | H |
| 563. | CH₂OCH₃ | CHO | H ' |
| 564. | nBu | CHO | H |
| 565. | H | CHO | CH₃ |
| 566. | CH₃ | CHO | CH₃ |
| 567. | CF₃ | CHO | CH₃ |
| 568. | CH₂OCH₃ | CHO | CH₃ |
| 569. | nBu | CHO | CH₃ |
| 570. | H | H | CHO |
| 571. | CH₃ | H | CHO |
| 572. | CF₃ | H | CHO |
| 573. | CH₂OCH₃ | H | CHO |
| 574. | nBu | H | CHO |
| 575. | H | CH₃ | CHO |
| 576. | CH₃ | CH₃ | CHO |
| 577. | CF₃ | CH₃ | CHO |
| 578. | CH₂OCH₃ | CH₃ | CHO |
| 579. | nBu | CH₃ | CHO |
| 580. | H | Cl | CH₃ |
| 581. | H | CH₃ | Cl |
| 582. | H | CF₃ | Cl |
| 583. | H | Cl | CF₃ |
| 584. | CH₃ | Cl | CH₃ |
| 585. | CH₃ | CH₃ | Cl |
| 586. | CH₃ | CF₃ | Cl |
| 587. | CH₃ | Cl | CF₃ |
| 588. | CF₃ | Cl | CH₃ |
| 589. | CF₃ | CH₃ | Cl |
| 590. | CF₃ | CF₃ | Cl |
| 591. | CF₃ | Cl | CF₃ |
| 592. | OCH₃ | H | H |
| 593. | OCH₃ | CH₃ | CH₃ |
| 594. | OCH₃ | CH₃ | H |
| 595. | OCH₃ | H | CH₃ |
| 596. | OCH₃ | CHO | H |
| 597. | OCH₃ | CHO | CH₃ |
| 598. | OCH₃ | H | CF₃ |
| 599. | OCH₃ | CF₃ | H |
| 600. | OCH₃ | CF₃ | CF₃ |
| 601. | OCH₃ | CH₂OCH₃ | CH₃ |
| 602. | OCH₃ | CH₃ | CH₂OCH₃ |
| 603. | OCH₃ | CH₂OCH₃ | H |
| 604. | OCH₃ | H | CH₂OCH₃ |
| 605. | H | Cl | OCH₂CH₃ |
| 606. | H | Cl | SCH₂CH₃ |
| 607. | H | Cl | Morpholin |
| 608. | H | Cl | Pyperidin |
| 609. | H | Pyperidin | Pyperidin |
| 610. | H | 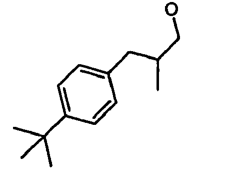 | Cl |
| 611. | H | 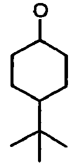 | Cl |
| 612. | H | CH₃(CH₂)₁₃O | Cl |
| 613. | H | OCH₂Ph-3-Cl | Cl |
| 614. | H | Cl | OCH₂Ph-3-Cl |
| 615. | H | O(CH₂)₂ C 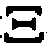 CH | Cl |
| 616. | H | OCH(CH₃)=CH₂ | Cl |
| 617. | H | O(CH₂)₂ CH=CH₂ | Cl |
| 618. | H | Cl | O(CH₂)₂CH=CH2 |
| 619. | H | 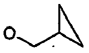 | Cl |
| 620. | H | O(CH₂)₂Ph-4-CN | Cl |
| 621. | H | Cl | O(CH₂)₂Ph-4-CN |
| 622. | H | OCH₂CH₂CH₃ | Cl |
| 623. | H | O(CH₂)₂O(CH₂)₂OCH₃ | Cl |
| 624. | H | CH₃CH₂CH(CH₃)O OCH(CH₃)CH₂CH₃ | Cl |
| 625. | H | OCH₂CH=CH₂ | Cl |
| 626. | H | Cl | OCH₂CH=CH₂ |
| 627. | H | Cl | OCH₂C 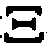 CH |
| 628. | H | O(CH₂)₂ C 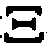 CCH₃ | Cl |
| 629. | H | OCH₂ C 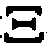 CCH₃ | Cl |
| 630. | H | Cl | OCH₂C 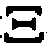 CCH₃ |
| 631. | H | OCH(CH₃)-cycloprop. | Cl |
| 632. | H | OCH₂C(CH₃)=CH₂ | Cl |
| 633. | H | Cl | OCH₂C(CH₃)=CH₂ |
| 634. | H | 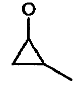 | Cl |
| 635. | H | Cl | 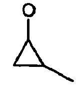 |
| 636. | H | O(CH₂)₂OPh-2-Cl | Cl |
| 637. | H | O-Cl, m-ClPhCH(CH₃)O | Cl |
| 638. | H | O(CH₂)₂SCH₂Ph-4-Cl | Cl |
| 639. | H | O(CH₂)₂Ph-2-Cl | Cl |
| 640. | H | O(CH₂)₂Ph-3-CF₃ | Cl |
| 641. | H | O(CH₂)₂Ph-4-CH₃ | Cl |
| 642. | H | 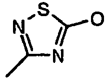 | Cl |
| 643. | H | 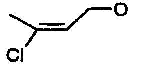 | Cl |
| 644. | H | O (CH₂)₂ CF₃ | Cl |
| 645. | H | 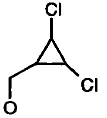 | Cl |
| 646. | H | O(CH₂)₁₁C(O)OCH₃ | Cl |
| 647. | H | 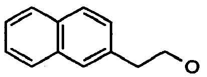 | Cl |
| 648. | H | O(CH₂)₂SCH₃ | Cl |
| 649. | H | O(CH₂)₇CH₃ | Cl |
| 650. | H | OCH₂Ph-3-OCH₃ | Cl |
| 651. | H | 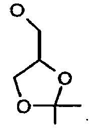 | Cl |
| 652. | H | OC₁₂H₂₄ | Cl |
| 653. | H | O(CH₂)₂O(CH₂)₅CH₃ | Cl |
| 654. | H | O C₁₀H₁₈ | Cl |
| 655. | H | 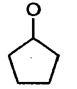 | Cl |
| 656. | H | O(CH₂)₂SCH₂CH₃ | Cl |
| 657. | H | 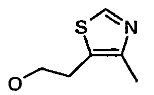 | Cl |
| 658. | H | OCH₂CH=CH(CH₂)₂CH₃ | Cl |
| 659. | H | O(CH₂)₂Ph-3,4-(OCH₃)₂ | Cl |
| 660. | H | O(CH₂)₂Ph-4-Cl | Cl |
| 661. | H | CF₃(CF₂)₅CH₂O | Cl |
| 662. | H | 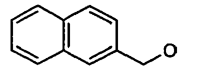 | Cl |
| 663. | H | OCH₂Ph-2-I | Cl |
| 664. | H | CH₃(CH₂)₂O(CH₂)₂O(CH ₂)₂O(CH₂)₂O | Cl |
| 665. | H | 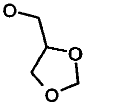 | Cl |
| 666. | H | O(CH₂)₃-4-(C₅H₄N) | Cl |
| 667. | H | 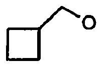 | Cl |
| 668. | H | 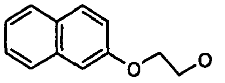 | Cl |
| 669. | H | O(CH₂)₁₁Br | Cl |
| 670. | H | O(CH₂)₂S Ph | Cl |
| 671. | H | 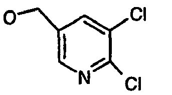 | Cl |
| 672. | H | 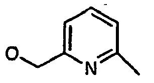 | Cl |
| 673. | H | O(CH₂)₆Ph | Cl |
| 674. | H | 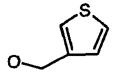 | Cl |
| 675. | H | O(CH₂)₉CH=CH₂ | Cl |
| 676. | H | 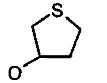 | Cl |
| 677. | H | 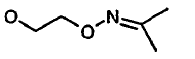 | Cl |
| 678. | H | OCH₂Ph-3-CF₃ | Cl |
| 679. | H | OCH₂-3-(C₅H₄N) | Cl |
| 680. | H | OCH₂Si(CH₃)₃ | Cl |
| 681. | H | O(CH₂)₄Cl | Cl |
| 682. | H | 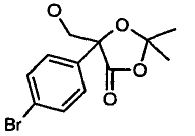 | Cl |
| 683. | H | 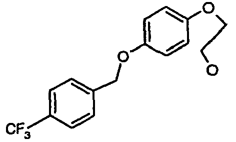 | Cl |
| 684. | H | 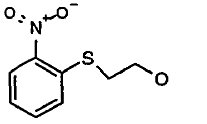 | Cl |
| 685. | H | SEt | SEt |
| 686. | H | Cl | OiPr |
| 687. | H | Cl | NH₂ |
| 688. | H | Cl | N(CH₃)NH₂ |
| 689. | H | 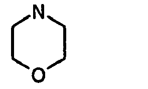 | 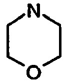 |
| 690. | H | Cl | NHPr |
| 691. | H | Cl | NHPh |
| 692. | H | Cl | NHCH(CH₂)₂ |
| 693. | H | NHPr | Cl |
| 694. | H | NHCH(CH₂)₂ | Cl |
| 695. | H | Cl | NH C₅H₉ |
| 696. | H | NH C₅H₉ | Cl |
| 697. | H | Cl | 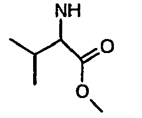 |
| 698. | H | Cl | N(CH₃)(OCH₃) |
| 699. | H | Cl | NHCH₂C 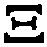 CH |
| 700. | H | Cl | NHCH(CH₃)CH₂OCH₃ |
| 701. | H | Cl | NHEt |
| 702. | H | NHCH₂C 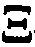 CH | Cl |
| 703. | H | NHEt | Cl |
| 704. | H | Br | Br |
| 705. | H | iPr | Br |
| 706. | H | OBu | Ph |
| 707. | H | Ph-3,5-(CF₃)₂ | Ph-3,5-(CF₃)₂ |
| 708. | H | Ph-4-CH₃ | Ph-4-CH₃ |
| 709. | H | OiPr | Ph |
| 710. | H | OiPr | Ph-4-CH₃ |
| 711. | H | OiPr | Ph-3,5-(CF₃)₂ |
| 712. | H | OiPr | Ph-4- Si (CH₃)₃ |
| 713. | H | Ph-4- Si (CH₃)₃ | Ph-4- Si (CH₃)₃ |
| 714. | H | OCH₂C 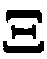 CH | Cl |
| 715. | H | NHCH₂Ph | Cl |
| 716. | H | Cl | NHCH₂Ph |
| 717. | H | NH₂ | Cl |
| 718. | H | 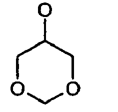 | Cl |
| 719. | H | 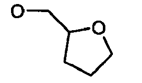 | Cl |
| 720. | H | 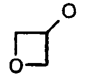 | Cl |
| 721. | H | Cl | 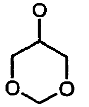 |
| 722. | H | Cl | 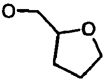 |
| 723. | H | OiPr | Cl |
| 724. | H | OEt | Cl |
| 725. | H | NHCH(CH₃)CH₂OCH₃ | Cl |
| 726. | H | SCH₂CH₃ | Cl |

**Table B-7**

| | | | | | |
|---|---|---|---|---|---|
| 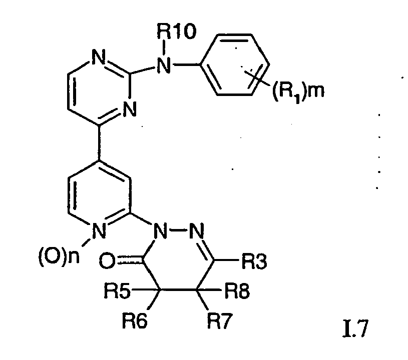 | | | | | |

| No | R₃ | R₅ | R₆ | R₇ | R₈ |
|---|---|---|---|---|---|
| 1. | H | H | H | H | H |
| 2. | H | CH₃ | H | H | H |
| 3. | H | nBu | H | H | H |
| 4. | H | CF₃ | H | H | H |
| 5. | H | CF₂CF₃ | H | H | H |
| 6. | H | CH₂OCH₃ | H | H | H |
| 7. | H | OCH₃ | H | H | H |
| 8. | H | Ph | H | H | H |
| 9. | H | -CH₂Ph | H | H | H |
| 10. | H | H | H | CH₃ | H |
| 11. | H | H | H | nBu | H |
| 12. | H | H | H | CF₃ | H |
| 13. | H | H | H | CF₂CF₃ | H |
| 14. | H | H | H | CH₂OCH₃ | H |
| 15. | H | H | H | OCH₃ | H |
| 16. | H | H | H | Ph | H |
| 17. | H | H | H | -CH₂Ph | H |
| 18. | H | CH₃ | CH₃ | H | H |
| 19. | H | nBu | CH₃ | H | H |
| 20. | H | CF₃ | CH₃ | H | H |
| 21. | H | CF₂CF₃ | CH₃ | H | H |
| 22. | H | CH₂OCH₃ | CH₃ | H | H |
| 23. | H | OCH₃ | CH₃ | H | H |
| 24. | H | Ph | CH₃ | H | H |
| 25. | H | -CH₂Ph | CH₃ | H | H |
| 26. | H | H | H | CH₃ | CH₃ |
| 27. | H | H | H | CH₃ | nBu |
| 28. | H | H | H | CH₃ | CF₃ |
| 29. | H | H | H | CH₃ | CF₂CF₃ |
| 30. | H | H | H | CH₃ | CH₂OCH₃ |
| 31. | H | H | H | CH₃ | OCH₃ |
| 32. | H | H | H | CH₃ | Ph |
| 33. | H | H | H | CH₃ | -CH₂Ph |
| 34. | H | CH₃ | H | H | CH₃ |
| 35. | H | nBu | H | H | CH₃ |
| 36. | H | CF₃ | H | H | CH₃ |
| 37. | H | CF₂CF₃ | H | H | CH₃ |
| 38. | H | CH₂OCH₃ | H | H | CH₃ |
| 39. | H | OCH₃ | H | H | CH₃ |
| 40. | H | Ph | H | H | CH₃ |
| 41. | H | -CH₂Ph | H | H | CH₃ |
| 42. | H | H | CH₃ | nBu | H |
| 43. | H | H | CH₃ | CF₃ | H |
| 44. | H | H | CH₃ | CF₂CF₃ | H |
| 45. | H | H | CH₃ | CH₂OCH₃ | H |
| 46. | H | H | CH₃ | OCH₃ | H |
| 47. | H | H | CH₃ | Ph | H |
| 48. | H | H | CH₃ | -CH₂Ph | H |
| 49. | H | CH₃ | H | CH₃ | CH₃ |
| 50. | H | nBu | H | CH₃ | CH₃ |
| 51. | H | CF₃ | H | CH₃ | CH₃ |
| 52. | H | CF₂CF₃ | H | CH₃ | CH₃ |
| 53. | H | CH₂OCH₃ | H | CH₃ | CH₃ |
| 54. | H | OCH₃ | H | CH₃ | CH₃ |
| 55. | H | Ph | H | CH₃ | CH₃ |
| 56. | H | -CH₂Ph | H | CH₃ | CH₃ |
| 57. | H | CH₃ | CH₃ | CH₃ | H |
| 58. | H | CH₃ | CH₃ | nBu | H |
| 59. | H | CH₃ | CH₃ | CF₃ | H |
| 60. | H | CH₃ | CH₃ | CF₂CF₃ | H |
| 61. | H | CH₃ | CH₃ | CH₂OCH₃ | H |
| 62. | H | CH₃ | CH₃ | OCH₃ | H |
| 63. | H | CH₃ | CH₃ | Ph | H |
| 64. | H | CH₃ | CH₃ | -CH₂Ph | H |
| 65. | H | nBu | CH₃ | CH₃ | H |
| 66. | H | CF₃ | CH₃ | CH₃ | H |
| 67. | H | CF₂CF₃ | CH₃ | CH₃ | H |
| 68. | H | CH₂OCH₃ | CH₃ | CH₃ | H |
| 69. | H | OCH₃ | CH₃ | CH₃ | H |
| 70. | H | Ph | CH₃ | CH₃ | H |
| 71. | H | -CH₂Ph | CH₃ | CH₃ | H |
| 72. | H | CH₃ | H | CH₃ | nBu |
| 73. | H | CH₃ | H | CH₃ | CF₃ |
| 74. | H | CH₃ | H | CH₃ | CF₂CF₃ |
| 75. | H | CH₃ | H | CH₃ | CH₂OCH₃ |
| 76. | H | CH₃ | H | CH₃ | OCH₃ |
| 77. | H | CH₃ | H | CH₃ | Ph |
| 78. | H | CH₃ | H | CH₃ | -CH₂Ph |
| 79. | H | CH₃ | CH₃ | CH₃ | CH₃ |
| 80. | H | nBu | CH₃ | CH₃ | CH₃ |
| 81. | H | CF₃ | CH₃ | CH₃ | CH₃ |
| 82. | H | CF₂CF₃ | CH₃ | CH₃ | CH₃ |
| 83. | H | CH₂OCH₃ | CH₃ | CH₃ | CH₃ |
| 84. | H | OCH₃ | CH₃ | CH₃ | CH₃ |
| 85. | H | Ph | CH₃ | CH₃ | CH₃ |
| 86. | H | -CH₂Ph | CH₃ | CH₃ | CH₃ |
| 87. | H | CH₃ | CH₃ | CH₃ | nBu |
| 88. | H | CH₃ | CH₃ | CH₃ | CF₃ |
| 89. | H | CH₃ | CH₃ | CH₃ | CF₂CF₃ |
| 90. | H | CH₃ | CH₃ | CH₃ | CH₂OCH₃ |
| 91. | H | CH₃ | CH₃ | CH₃ | OCH₃ |
| 92. | H | CH₃ | CH₃ | CH₃ | Ph |
| 93. | H | CH₃ | CH₃ | CH₃ | -CH₂Ph |
| 94. | H | nBnu | nBu | H | H |
| 95. | H | CF₃ | nBu | H | H |
| 96. | H | CF₂CF₃ | nBu | H | H |
| 97. | H | CH₂OCH | nBu | H | H |
| 98. | H | OCH₃ | nBu | H | H |
| 99. | H | Ph | nBu | H | H |
| 100 | H | -CH₂Ph | nBu | H | H |
| 101 | H | H | H | Bu | nBu |
| 102 | H | H | H | CF₃ | nBu |
| 103 | H | H | H | CF₂CF₃ | nBu |
| 104 | H | H | H | CH₂OCH₃ | nBu |
| 105 | H | H | H | OCH₃ | nBu |
| 106 | H | H | H | Ph | nBu |
| 107 | H | H | H | -CH₂Ph | nBu |
| 108 | H | nBu | H | H | H |
| 109 | H | nBu | H | H | CH₃ |
| 110 | H | nBu | H | H | nBu |
| 111 | H | nBu | H | H | CF₃ |
| 112 | H | nBu | H | H | CF₂CF₃ |
| 113 | H | nBu | H | H | CH₂OCH₃ |
| 114 | H | nBu | H | H | OCH₃ |
| 115 | H | nBu | H | H | Ph |
| 116 | H | nBu | H | H | -CH₂Ph |
| 117 | H | H | H | nBu | H |
| 118 | H | H | CH₃ | nBu | H |
| 119 | H | H | nBu | nBu | H |
| 120 | H | H | CF₃ | nBu | H |
| 121 | H | H | CF₂CF₃ | nBu | H |
| 122 | H | H | CH₂OCH₃ | nBu | H |
| 123 | H | H | OCH₃ | nBu | H |
| 124 | H | H | Ph | nBu | H |
| 125 | H | H | -CH₂Ph | nBu | H |
| 126 | H | CH₃ | nBu | nBu | H |
| 127 | H | nBu | nBu | nBu | H |
| 128 | H | CF₃ | nBu | nBu | H |
| 129 | H | CF₂CF₃ | nBu | nBu | H |
| 130 | H | CH₂OCH₃ | nBu | nBu | H |
| 131 | H | OCH₃ | nBu | nBu | H |
| 132 | H | Ph | nBu | Bu | H |
| 133 | H | -CH₂Ph | nBu | nBu | H |
| 134 | H | CH₃ | H | nBu | nBu |
| 135 | H | nBu | H | nBu | nBu |
| 136 | H | CF₃ | H | nBu | nBu |
| 137 | H | CF₂CF₃ | H | nBu | nBu |
| 138 | H | CH₂OCH₃ | H | nBu | nBu |
| 139 | H | OCH₃ | H | nBu | nBu |
| 140 | H | Ph | H | nBu | nBu |
| 141 | H | -CH₂Ph | H | nBu | nBu |
| 142 | H | nBu | nBu | CH₃ | H |
| 143 | H | nBu | nBu | CF₃ | H |
| 144 | H | nBu | nBu | CF₂CF₃ | H |
| 145 | H | nBu | nBu | CH₂OCH₃ | H |
| 146 | H | nBu | nBu | OCH₃ | H |
| 147 | H | nBu | nBu | Ph | H |
| 148 | H | nBu | nBu | -CH₂Ph | H |
| 149 | H | nBu | H | CH₃ | nBu |
| 150 | H | nBu | H | CF₃ | nBu |
| 151 | H | nBu | H | CF₂CF₃ | nBu |
| 152 | H | nBu | H | CH₂OCH₃ | nBu |
| 153 | H | nBu | H | OCH₃ | nBu |
| 154 | H | nBu | H | Ph | nBu |
| 155 | H | nBu | H | -CH₂Ph | nBu |
| 156 | H | CH₃ | nBu | nBu | nBu |
| 157 | H | Bu | nBu | nBu | nBu |
| 158 | H | CF₃ | nBu | nBu | nBu |
| 159 | H | CF₂CF₃ | nBu | nBu | nBu |
| 160 | H | CH₂OCH₃ | nBu | nBu | nBu |
| 161 | H | OCH₃ | nBu | nBu | nBu |
| 162 | H | Ph | nBu | nBu | nBu |
| 163 | H | -CH₂Ph | nBu | nBu | nBu |
| 164 | H | nBu | nBu | nBu | CH₃ |
| 165 | H | nBu | nBu | nBu | CF₃ |
| 166 | H | nBu | nBu | nBu | CF₂CF₃ |
| 167 | H | nBu | nBu | nBu | CH₂OCH₃ |
| 168 | H | nBu | nBu | nBu | OCH₃ |
| 169 | H | nBu | nBu | nBu | Ph |
| 170 | H | nBu | Bu | nBu | -CH₂Ph |
| 171 | H | nBu | CH₃ | nBu | CH₃ |
| 172 | H | nBu | CH₃ | nBu | CF₃ |
| 173 | H | nBu | CH₃ | nBu | CF₂CF₃ |
| 174 | H | nBu | CH₃ | nBu | CH₂OCH₃ |
| 175 | H | nBu | CH₃ | nBu | OCH₃ |
| 176 | H | nBu | CH₃ | nBu | Ph |
| 177 | H | nBu | CH₃ | nBu | -CH₂Ph |
| 178 | H | CF₃ | nBu | nBu | CH₃ |
| 179 | H | CF₂CF₃ | nBu | nBu | CH₃ |
| 180 | H | CH₂OCH₃ | nBu | nBu | CH₃ |
| 181 | H | OCH₃ | nBu | nBu | CH₃ |
| 182 | H | Ph | nBu | nBu | CH₃ |
| 183 | H | -CH₂Ph | nBu | nBu | CH₃ |
| 184 | H | CH₃ | CH₃ | nBu | nBu |
| 185 | H | CF₃ | CH₃ | nBu | nBu |
| 186 | H | CF₂CF₃ | CH₃ | nBu | nBu |
| 187 | H | CH₂OCH₃ | CH₃ | nBu | nBu |
| 188 | H | OCH₃ | CH₃ | nBu | nBu |
| 189 | H | Ph | CH₃ | nBu | nBu |
| 190 | H | -CH₂Ph | CH₃ | nBu | nBu |
| 191 | H | nBu | nBu | CH₃ . | CH₃ |
| 192 | H | nBu | nBu | CF₃ | CH₃ |
| 193 | H | nBu | nBu | CF₂CF₃ | CH₃ |
| 194 | H | nBu | nBu | CH₂OCH₃ | CH₃ |
| 195 | H | nBu | nBu | OCH₃ | CH₃ |
| 196 | H | nBu | nBu | Ph | CH₃ |
| 197 | H | nBu | nBu | -CH₂Ph | CH₃ |
| 198 | H | nBu | CH₃ | CH₃ | CF₃ |
| 199 | H | nBu | CH₃ | CH₃ | CF₂CF₃ |
| 200 | H | nBu | CH₃ | CH₃ | CH₂OCH₃ |
| 201 | H | nBu | CH₃ | CH₃ | OCH₃ |
| 202 | H | nBu | CH₃ | CH₃ | Ph |
| 203 | H | nBu | CH₃ | CH₃ | -CH₂Ph |
| 204 | H | CF₃ | CH₃ | CH₃ | nBu |
| 205 | H | CF₂CF₃ | CH₃ | CH₃ | nBu |
| 206 | H | CH₂OCH₃ | CH₃ | CH₃ | nBu |
| 207 | H | OCH₃ | CH₃ | CH₃ | nBu |
| 208 | H | Ph | CH₃ | CH₃ | nBu |
| 209 | H | -CH₂Ph | CH₃ | CH₃ | nBu |
| 210 | H | CF₃ | nBu | CH₃ | CH₃ |
| 211 | H | CF₂CF₃ | nBu | CH₃ | CH₃ |
| 212 | H | CH₂OCH₃ | nBu | CH₃ | CH₃ |
| 213 | H | OCH₃ | nBu | CH₃ | CH₃ |
| 214 | H | Ph | nBu | CH₃ | CH₃ |
| 215 | H | -CH₂Ph | nBu | CH₃ | CH₃ |
| 216 | H | CH₃ | CH₃ | CF₃ | nBu |
| 217 | H | CH₃ | CH₃ | CF₂CF₃ | nBu |
| 218 | H | CH₃ | CH₃ | CH₂OCH₃ | nBu |
| 219 | H | CH₃ | CH₃ | OCH₃ | nBu |
| 220 | H | CH₃ | CH₃ | Ph | nBu |
| 221 | H | CH₃ | CH₃ | -CH₂Ph | nBu |
| 222 | H | CF₃ | nBu | H | CH₃ |
| 223 | H | CF₂CF₃ | nBu | H | CH₃ |
| 224 | H | CH₂OCH₃ | nBu | H | CH₃ |
| 225 | H | OCH₃ | nBu | H | CH₃ |
| 226 | H | Ph | nBu | H | CH₃ |
| 227 | H | -CH₂Ph | nBu | H | CH₃ |
| 228 | H | H | CH₃ | CF₃ | nBu |
| 229 | H | H | CH₃ | CF₂CF₃ | nBu |
| 230 | H | H | CH₃ | CH₂OCH₃ | nBu |
| 231 | H | H | CH₃ | OCH₃ | nBu |
| 232 | H | H | CH₃ | Ph | nBu |
| 233 | H | H | CH₃ | -CH₂Ph | nBu |
| 234 | H | nBu | H | CH₃ | CF₃ |
| 235 | H | nBu | H | CH₃ | CF₂CF₃ |
| 236 | H | nBu | H | CH₃ | CH₂OCH₃ |
| 237 | H | nBu | H | CH₃ | OCH₃ |
| 238 | H | nBu | H | CH₃ | Ph |
| 239 | H | nBu | H | CH₃ | -CH₂Ph |
| 240 | H | CF₃ | CH₃ | nBu | H |
| 241 | H | CF₂CF₃ | CH₃ | nBu | H |
| 242 | H | CH₂OCH₃ | CH₃ | nBu | H |
| 243 | H | OCH₃ | CH₃ | nBu | H |
| 244 | H | Ph | CH₃ | nBu | H |
| 245 | H | -CH₂Ph | CH₃ | nBu | H |
| 246 | H | CF₃ | Bu | CH₃ | H |
| 247 | H | CF₂CF₃ | Bu | CH₃ | H |
| 248 | H | CH₂OCH₃ | Bu | CH₃ | H |
| 249 | H | OCH₃ | Bu | CH₃ | H |
| 250 | H | Ph | Bu | CH₃ | H |
| 251 | H | -CH₂Ph | Bu | CH₃ | H |
| 252 | H | CH₃ | H | CF₃ | nBu |
| 253 | H | CH₃ | H | CF₂CF₃ | nBu |
| 254 | H | CH₃ | H | CH₂OCH₃ | nBu |
| 255 | H | CH₃ | H | OCH₃ | nBu |
| 256 | H | CH₃ | H | Ph | nBu |
| 257 | H | CH₃ | H | -CH₂Ph | nBu |
| 258 | H | nBu | CH₃ | H | CF₃ |
| 259 | H | nBu | CH₃ | H | CF₂CF₃ |
| 260 | H | nBu | CH₃ | H | CH₂OCH₃ |
| 261 | H | nBu | CH₃ | H | OCH₃ |
| 262 | H | nBu | CH₃ | H | Ph |
| 263 | H | nBu | CH₃ | H | -CH₂Ph |
| 264 | H | CF₃ | H | CH₃ | nBu |
| 265 | H | CF₂CF₃ | H | CH₃ | nBu |
| 266 | H | CH₂OCH₃ | H | CH₃ | nBu |
| 267 | H | OCH₃ | H | CH₃ | nBu |
| 268 | H | Ph | H | CH₃ | nBu |
| 269 | H | -CH₂Ph | H | CH₃ | nBu |
| 270 | CH₃ | H | H | H | H |
| 271 | CH₃ | CH₃ | H | H | H |
| 272 | CH₃ | nBu | H | H | H |
| 273 | CH₃ | CF₃ | H | H | H |
| 274 | CH₃ | CF₂CF₃ | H | H | H |
| 275 | CH₃ | CH₂OCH | H | H | H |
| 276 | CH₃ | OCH₃ | H | H | H |
| 277 | CH₃ | Ph | H | H | H |
| 278 | CH₃ | -CH₂Ph | H | H | H |
| 279 | CH₃ | H | H | CH₃ | H |
| 280 | CH₃ | H | H | nBu | H |
| 281 | CH₃ | H | H | CF₃ | H |
| 282 | CH₃ | H | H | CF₂CF₃ | H |
| 283 | CH₃ | H | H | CH₂OCH₃ | H |
| 284 | CH₃ | H | H | OCH₃ | H |
| 285 | CH₃ | H | H | Ph | H |
| 286 | CH₃ | H | H | -CH₂Ph | H |
| 287 | CH₃ | CH₃ | CH₃ | H | H |
| 288 | CH₃ | nBu | CH₃ | H | H |
| 289 | CH₃ | CF₃ | CH₃ | H | H |
| 290 | CH₃ | CF₂CF₃ | CH₃ | H | H |
| 291 | CH₃ | CH₂OCH₃ | CH₃ | H | H |
| 292 | CH₃ | OCH₃ | CH₃ | H | H |
| 293 | CH₃ | Ph | CH₃ | H | H |
| 294 | CH₃ | -CH₂Ph | CH₃ | H | H |
| 295 | CH₃ | H | H | CH₃ | CH₃ |
| 296 | CH₃ | H | H | CH₃ | nBu |
| 297 | CH₃ | H | H | CH₃ | CF₃ |
| 298 | CH₃ | H | H | CH₃ | CF₂CF₃ |
| 299 | CH₃ | H | H | CH₃ | CH₂OCH₃ |
| 300 | CH₃ | H | H | CH₃ | OCH₃ |
| 301 | CH₃ | H | H | CH₃ | Ph |
| 302 | CH₃ | H | H | CH₃ | -CH₂Ph |
| 303 | CH₃ | CH₃ | H | H | CH₃ |
| 304 | CH₃ | Bu | H | H | CH₃ |
| 305 | CH₃ | CF₃ | H | H | CH₃ |
| 306 | CH₃ | CF₂CF₃ | H | H | CH₃ |
| 307 | CH₃ | CH₂OCH₃ | H | H | CH₃ |
| 308 | CH₃ | OCH₃ | H | H | CH₃ |
| 309 | CH₃ | Ph | H | H | CH₃ |
| 310 | CH₃ | -CH₂Ph | H | H | CH₃ |
| 311 | CH₃ | H | CH₃ | nBu | H |
| 312 | CH₃ | H | CH₃ | CF₃ | H |
| 313 | CH₃ | H | CH₃ | CF₂CF₃ | H |
| 314 | CH₃ | H | CH₃ | CH₂OCH₃ | H |
| 315 | CH₃ | H | CH₃ | OCH₃ | H |
| 316 | CH₃ | H | CH₃ | Ph | H |
| 317 | CH₃ | H | CH₃ | -CH₂Ph | H |
| 318 | CH₃ | CH₃ | H | CH₃ | CH₃ |
| 319 | CH₃ | nBu | H | CH₃ | CH₃ |
| 320 | CH₃ | CF₃ | H | CH₃ | CH₃ |
| 321 | CH₃ | CF₂CF₃ | H | CH₃ | CH₃ |
| 322 | CH₃ | CH₂OCH₃ | H | CH₃ | CH₃ |
| 323 | CH₃ | OCH₃ | H | CH₃ | CH₃ |
| 324 | CH₃ | Ph | H | CH₃ | CH₃ |
| 325 | CH₃ | -CH₂Ph | H | CH₃ | CH₃ |
| 326 | CH₃ | CH₃ | CH₃ | CH₃ | H |
| 327 | CH₃ | CH₃ | CH₃ | nBu | H |
| 328 | CH₃ | CH₃ | CH₃ | CF₃ | H |
| 329 | CH₃ | CH₃ | CH₃ | CF₂CF₃ | H |
| 330 | CH₃ | CH₃ | CH₃ | CH₂OCH₃ | H |
| 331 | CH₃ | CH₃ | CH₃ | OCH₃ | H |
| 332 | CH₃ | CH₃ | CH₃ | Ph | H |
| 333 | CH₃ | CH₃ | CH₃ | -CH₂Ph | H |
| 334 | CH₃ | nBu | CH₃ | CH₃ | H |
| 335 | CH₃ | CF₃ | CH₃ | CH₃ | H |
| 336 | CH₃ | CF₂CF₃ | CH₃ | CH₃ | H |
| 337 | CH₃ | CH₂OCH₃ | CH₃ | CH₃ | H |
| 338 | CH₃ | OCH₃ | CH₃ | CH₃ | H |
| 339 | CH₃ | Ph | CH₃ | CH₃ | H |
| 340 | CH₃ | -CH₂Ph | CH₃ | CH₃ | H |
| 341 | CH₃ | CH₃ | H | CH₃ | nBu |
| 342 | CH₃ | CH₃ | H | CH₃ | CF₃ |
| 343 | CH₃ | CH₃ | H | CH₃ | CF₂CF₃ |
| 344 | CH₃ | CH₃ | H | CH₃ | CH₂OCH₃ |
| 345 | CH₃ | CH₃ | H | CH₃ | OCH₃ |
| 346 | CH₃ | CH₃ | H | CH₃ | Ph |
| 347 | CH₃ | CH₃ | H | CH₃ | -CH₂Ph |
| 348 | CH₃ | CH₃ | CH₃ | CH₃ | CH₃ |
| 349 | CH₃ | nBu | CH₃ | CH₃ | CH₃ |
| 350 | CH₃ | CF₃ | CH₃ | CH₃ | CH₃ |
| 351 | CH₃ | CF₂CF₃ | CH₃ | CH₃ | CH₃ |
| 352 | CH₃ | CH₂OCH₃ | CH₃ | CH₃ | CH₃ |
| 353 | CH₃ | OCH₃ | CH₃ | CH₃ | CH₃ |
| 354 | CH₃ | Ph | CH₃ | CH₃ | CH₃ |
| 355 | CH₃ | -CH₂Ph | CH₃ | CH₃ | CH₃ |
| 356 | CH₃ | CH₃ | CH₃ | CH₃ | nBu |
| 357 | CH₃ | CH₃ | CH₃ | CH₃ | CF₃ |
| 358 | CH₃ | CH₃ | CH₃ | CH₃ | CF₂CF₃ |
| 359 | CH₃ | CH₃ | CH₃ | CH₃ | CH₂OCH₃ |
| 360 | CH₃ | CH₃ | CH₃ | CH₃ | OCH₃ |
| 361 | CH₃ | CH₃ | CH₃ | CH₃ | Ph |
| 362 | CH₃ | CH₃ | CH₃ | CH₃ | -CH₂Ph |
| 363 | CH₃ | nBu | nBu | H | H |
| 364 | CH₃ | CF₃ | nBu | H | H |
| 365 | CH₃ | CF₂CF₃ | nBu | H | H |
| 366 | CH₃ | CH₂OCH₃ | nBu | H | H |
| 367 | CH₃ | OCH₃ | nBu | H | H |
| 368 | CH₃ | Ph | nBu | H | H |
| 369 | CH₃ | -CH₂Ph | nBu | H | H |
| 370 | CH₃ | H | H | nBu | nBu |
| 371 | CH₃ | H | H | CF₃ | nBu |
| 372 | CH₃ | H | H | CF₂CF₃ | nBu |
| 373 | CH₃ | H | H | CH₂OCH₃ | nBu |
| 374 | CH₃ | H | H | OCH₃ | nBu |
| 375 | CH₃ | H | H | Ph | nBu |
| 376 | CH₃ | H | H | -CH₂Ph | nBu |
| 377 | CH₃ | nBu | H | H | H |
| 378 | CH₃ | nBu | H | H | CH₃ |
| 379 | CH₃ | nBu | H | H | nBu |
| 380 | CH₃ | nBu | H | H | CF₃ |
| 381 | CH₃ | nBu | H | H | CF₂CF₃ |
| 382 | CH₃ | nBu | H | H | CH₂OCH₃ |
| 383 | CH₃ | nBu | H | H | OCH₃ |
| 384 | CH₃ | nBu | H | H | Ph |
| 385 | CH₃ | nBu | H | H | -CH₂Ph |
| 386 | CH₃ | H | H | nBu | H |
| 387 | CH₃ | H | CH₃ | nBu | H |
| 388 | CH₃ | H | Bu | nBu | H |
| 389 | CH₃ | H | CF₃ | nBu | H |
| 390 | CH₃ | H | CF₂CF₃ | nBu | H |
| 391 | CH₃ | H | CH₂OCH₃ | nBu | H |
| 392 | CH₃ | H | OCH₃ | nBu | H |
| 393 | CH₃ | H | Ph | nBu | H |
| 394 | CH₃ | H | -CH₂Ph | nBu | H |
| 395 | CH₃ | CH₃ | nBu | nBu | H |
| 396 | CH₃ | nBu | nBu | nBu | H |
| 397 | CH₃ | CF₃ | nBu | nBu | H |
| 398 | CH₃ | CF₂CF₃ | nBu | nBu | H |
| 399 | CH₃ | CH₂OCH₃ | nBu | nBu | H |
| 400 | CH₃ | OCH₃ | nBu | nBu | H |
| 401 | CH₃ | Ph | nBu | nBu | H |
| 402 | CH₃ | -CH₂Ph | nBu | nBu | H |
| 403 | CH₃ | CH₃ | H | nBu | nBu |
| 404 | CH₃ | nBu | H | nBu | nBu |
| 405 | CH₃ | CF₃ | H | nBu | nBu |
| 406 | CH₃ | CF₂CF₃ | H | nBu | nBu |
| 407 | CH₃ | CH₂OCH₃ | H | nBu | nBu |
| 408 | CH₃ | OCH₃ | H | nBu | nBu |
| 409 | CH₃ | Ph | H | nBu | nBu |
| 410 | CH₃ | -CH₂Ph | H | nBu | nBu |
| 411 | CH₃ | nBu | nBu | CH₃ | H |
| 412 | CH₃ | nBu | nBu | CF₃ | H |
| 413 | CH₃ | nBu | nBu | CF₂CF₃ | H |
| 414 | CH₃ | nBu | nBu | CH₂OCH₃ | H |
| 415 | CH₃ | nBu | nBu | OCH₃ | H |
| 416 | CH₃ | nBu | nBu | Ph | H |
| 417 | CH₃ | nBu | nBu | -CH₂Ph | H |
| 418 | CH₃ | nBu | H | CH₃ | nBu |
| 419 | CH₃ | nBu | H | CF₃ | nBu |
| 420 | CH₃ | nBu | H | CF₂CF₃ | nBu |
| 421 | CH₃ | nBu | H | CH₂OCH₃ | nBu |
| 422 | CH₃ | nBu | H | OCH₃ | nBu |
| 423 | CH₃ | nBu | H | Ph | nBu |
| 424 | CH₃ | nBu | H | -CH₂Ph | nBu |
| 425 | CH₃ | CH₃ | nBu | nBu | nBu |
| 426 | CH₃ | Bu | nBu | nBu | nBu |
| 427 | CH₃ | CF₃ | nBu | nBu | nBu |
| 428 | CH₃ | CF₂CF₃ | nBu | nBu | nBu |
| 429 | CH₃ | CH₂OCH₃ | nBu | nBu | nBu |
| 430 | CH₃ | OCH₃ | nBu | nBu | nBu |
| 431 | CH₃ | Ph | nBu | nBu | nBu |
| 432 | CH₃ | -CH₂Ph | nBu | nBu | nBu |
| 433 | CH₃ | nBu | nBu | nBu | CH₃ |
| 434 | CH₃ | nBu | nBu | nBu | CF₃ |
| 435 | CH₃ | nBu | nBu | nBu | CF₂CF₃ |
| 436 | CH₃ | nBu | nBu | nBu | CH₂OCH₃ |
| 437 | CH₃ | nBu | nBu | nBu | OCH₃ |
| 438 | CH₃ | nBu | nBu | nBu | Ph |
| 439 | CH₃ | nBu | nBu | nBu | -CH₂Ph |
| 440 | CH₃ | nBu | CH₃ | nBu | CH₃ |
| 441 | CH₃ | nBu | CH₃ | nBu | CF₃ |
| 442 | CH₃ | nBu | CH₃ | nBu | CF₂CF₃ |
| 443 | CH₃ | nBu | CH₃ | nBu | CH₂OCH₃ |
| 444 | CH₃ | nBu | CH₃ | nBu | OCH₃ |
| 445 | CH₃ | nBu | CH₃ | nBu | Ph |
| 446 | CH₃ | nBu | CH₃ | nBu | -CH₂Ph |
| 447 | CH₃ | CF₃ | nBu | nBu | CH₃ |
| 448 | CH₃ | CF₂CF₃ | nBu | nBu | CH₃ |
| 449 | CH₃ | CH₂OCH₃ | nBu | nBu | CH₃ |
| 450 | CH₃ | OCH₃ | nBu | nBu | CH₃ |
| 451 | CH₃ | Ph | nBu | nBu | CH₃ |
| 452 | CH₃ | -CH₂Ph | nBu | nBu | CH₃ |
| 453 | CH₃ | CH₃ | CH₃ | nBu | nBu |
| 454 | CH₃ | CF₃ | CH₃ | nBu | nBu |
| 455 | CH₃ | CF₂CF₃ | CH₃ | nBu | nBu |
| 456 | CH₃ | CH₂OCH₃ | CH₃ | nBu | nBu |
| 457 | CH₃ | OCH₃ | CH₃ | nBu | nBu |
| 458 | CH₃ | Ph | CH₃ | nBu | nBu |
| 459 | CH₃ | -CH₂Ph | CH₃ | nBu | nBu |
| 460 | CH₃ | nBu | nBu | CH₃ | CH₃ |
| 461 | CH₃ | nBu | nBu | CF₃ | CH₃ |
| 462 | CH₃ | nBu | nBu | CF₂CF₃ | CH₃ |
| 463 | CH₃ | nBu | nBu | CH₂OCH₃ | CH₃ |
| 464 | CH₃ | nBu | nBu | OCH₃ | CH₃ |
| 465 | CH₃ | nBu | nBu | Ph | CH₃ |
| 466 | CH₃ | nBu | nBu | -CH₂Ph | CH₃ |
| 467 | CH₃ | nBu | CH₃ | CH₃ | CF₃ |
| 468 | CH₃ | nBu | CH₃ | CH₃ | CF₂CF₃ |
| 469 | CH₃ | nBu | CH₃ | CH₃ | CH₂OCH₃ |
| 470 | CH₃ | nBu | CH₃ | CH₃ | OCH₃ |
| 471 | CH₃ | nBu | CH₃ | CH₃ | Ph |
| 472 | CH₃ | nBu | CH₃ | CH₃ | -CH₂Ph |
| 473 | CH₃ | CF₃ | CH₃ | CH₃ | nBu |
| 474 | CH₃ | CF₂CF₃ | CH₃ | CH₃ | nBu |
| 475 | CH₃ | CH₂OCH₃ | CH₃ | CH₃ | nBu |
| 476 | CH₃ | OCH₃ | CH₃ | CH₃ | nBu |
| 477 | CH₃ | Ph | CH₃ | CH₃ | nBu |
| 478 | CH₃ | -CH₂Ph | CH₃ | CH₃ | nBu |
| 479 | CH₃ | CF₃ | nBu | CH₃ | CH₃ |
| 480 | CH₃ | CF₂CF₃ | nBu | CH₃ | CH₃ |
| 481 | CH₃ | CH₂OCH₃ | nBu | CH₃ | CH₃ |
| 482 | CH₃ | OCH₃ | nBu | CH₃ | CH₃ |
| 483 | CH₃ | Ph | nBu | CH₃ | CH₃ |
| 484 | CH₃ | -CH₂Ph | nBu | CH₃ | CH₃ |
| 485 | CH₃ | CH₃ | CH₃ | CF₃ | nBu |
| 486 | CH₃ | CH₃ | CH₃ | CF₂CF₃ | nBu |
| 487 | CH₃ | CH₃ | CH₃ | CH₂OCH₃ | nBu |
| 488 | CH₃ | CH₃ | CH₃ | OCH₃ | nBu |
| 489 | CH₃ | CH₃ | CH₃ | Ph | nBu |
| 490 | CH₃ | CH₃ | CH₃ | -CH₂Ph | nBu |
| 491 | CH₃ | CF₃ | nBu | H | CH₃ |
| 492 | CH₃ | CF₂CF₃ | nBu | H | CH₃ |
| 493 | CH₃ | CH₂OCH₃ | nBu | H | CH₃ |
| 494 | CH₃ | OCH₃ | nBu | H | CH₃ |
| 495 | CH₃ | Ph | nBu | H | CH₃ |
| 496 | CH₃ | -CH₂Ph | nBu | H | CH₃ |
| 497 | CH₃ | H | CH₃ | CF₃ | nBu |
| 498 | CH₃ | H | CH₃ | CF₂CF₃ | nBu |
| 499 | CH₃ | H | CH₃ | CH₂OCH₃ | nBu |
| 500 | CH₃ | H | CH₃ | OCH₃ | nBu |
| 501 | CH₃ | H | CH₃ | Ph | nBu |
| 502 | CH₃ | H | CH₃ | -CH₂Ph | nBu |
| 503 | CH₃ | nBu | H | CH₃ | CF₃ |
| 504 | CH₃ | nBu | H | CH₃ | CF₂CF₃ |
| 505 | CH₃ | nBu | H | CH₃ | CH₂OCH₃ |
| 506 | CH₃ | nBu | H | CH₃ | OCH₃ |
| 507 | CH₃ | nBu | H | CH₃ | Ph |
| 508 | CH₃ | nBu | H | CH₃ | -CH₂Ph |
| 509 | CH₃ | CF₃ | CH₃ | nBu | H |
| 510 | CH₃ | CF₂CF₃ | CH₃ | nBu | H |
| 511 | CH₃ | CH₂OCH₃ | CH₃ | nBu | H |
| 512 | CH₃ | OCH₃ | CH₃ | nBu | H |
| 513 | CH₃ | Ph | CH₃ | nBu | H |
| 514 | CH₃ | -CH₂Ph | CH₃ | nBu | H |
| 515 | CH₃ | CF₃ | nBu | CH₃ | H |
| 516 | CH₃ | CF₂CF₃ | nBu | CH₃ | H |
| 517 | CH₃ | CH₂OCH₃ | nBu | CH₃ | H |
| 518 | CH₃ | OCH₃ | nBu | CH₃ | H |
| 519 | CH₃ | Ph | nBu | CH₃ | H |
| 520 | CH₃ | -CH₂Ph | nBu | CH₃ | H |
| 521 | CH₃ | CH₃ | H | CF₃ | nBu |
| 522 | CH₃ | CH₃ | H | CF₂CF₃ | nBu |
| 523 | CH₃ | CH₃ | H | CH₂OCH₃ | nBu |
| 524 | CH₃ | CH₃ | H | OCH₃ | nBu |
| 525 | CH₃ | CH₃ | H | Ph | nBu |
| 526 | CH₃ | CH₃ | H | -CH₂Ph | nBu |
| 527 | CH₃ | nBu | CH₃ | H | CF₃ |
| 528 | CH₃ | nBu | CH₃ | H | CF₂CF₃ |
| 529 | CH₃ | nBu | CH₃ | H | CH₂OCH₃ |
| 530 | CH₃ | nBu | CH₃ | H | OCH₃ |
| 531 | CH₃ | nBu | CH₃ | H | Ph |
| 532 | CH₃ | nBu | CH₃ | H | -CH₂Ph |
| 533 | CH₃ | CF₃ | H | CH₃ | nBu |
| 534 | CH₃ | CF₂CF₃ | H | CH₃ | nBu |
| 535 | CH₃ | CH₂OCH₃ | H | CH₃ | nBu |
| 536 | CH₃ | OCH₃ | H | CH₃ | nBu |
| 537 | CH₃ | Ph | H | CH₃ | nBu |
| 538 | CH₃ | -CH₂Ph | H | CH₃ | nBu |
| 539 | CF₃ | H | H | H | H |
| 54C | CH₃ | CH₃ | H | H | H |
| 541 | CF₃ | nBu | H | H | H |
| 542 | CF₃ | CF₃ | H | H | H |
| 543 | CF₃ | CF₂CF₃ | H | H | H |
| 544 | CF₃ | CH₂OCH₃ | H | H | H |
| 545 | CF₃ | OCH₃ | H | H | H |
| 546 | CF₃ | Ph | H | H | H |
| 547 | CF₃ | -CH₂Ph | H | H | H |
| 548 | CF₃ | H | H | CH₃ | H |
| 549 | CF₃ | H | H | nBu | H |
| 550 | CF₃ | H | H | CF₃ | H |
| 551 | CF₃ | H | H | CF₂CF₃ | H |
| 552 | CF₃ | H | H | CH₂OCH₃ | H |
| 553 | CF₃ | H | H | OCH₃ | H |
| 554 | CF₃ | H | H | Ph | H |
| 555 | CF₃ | H | H | -CH₂Ph | H |
| 556 | CF₃ | CH₃ | CH₃ | H | H |
| 557 | CF₃ | nBu | CH₃ | H | H |
| 558 | CF₃ | CF₃ | CH₃ | H | H |
| 559 | CF₃ | CF₂CF₃ | CH₃ | H | H |
| 560 | CF₃ | CH₂OCH₃ | CH₃ | H | H |
| 561 | CF₃ | OCH₃ | CH₃ | H | H |
| 562 | CF₃ | Ph | CH₃ | H | H |
| 563 | CF₃ | -CH₂Ph | CH₃ | H | H |
| 564 | CF₃ | H | H | CH₃ | CH₃ |
| 565 | CF₃ | H | H | CH₃ | nBu |
| 566 | CF₃ | H | H | CH₃ | CF₃ |
| 567 | CF₃ | H | H | CH₃ | CF₂CF₃ |
| 568 | CF₃ | H | H | CH₃ | CH₂OCH₃ |
| 569 | CF₃ | H | H | CH₃ | OCH₃ |
| 570 | CF₃ | H | H | CH₃ | Ph |
| 571 | CF₃ | H | H | CH₃ | -CH₂Ph |
| 572 | CF₃ | CH₃ | H | H | CH₃ |
| 573 | CF₃ | nBu | H | H | CH₃ |
| 574 | CF₃ | CF₃ | H | H | CH₃ |
| 575 | CF₃ | CF₂CF₃ | H | H | CH₃ |
| 576 | CF₃ | CH₂OCH₃ | H | H | CH₃ |
| 577 | CF₃ | OCH₃ | H | H | CH₃ |
| 578 | CF₃ | Ph | H | H | CH₃ |
| 579 | CF₃ | -CH₂Ph | H | H | CH₃ |
| 580 | CF₃ | H | CH₃ | nBu | H |
| 581 | CF₃ | H | CH₃ | CF₃ | H |
| 582 | CF₃ | H | CH₃ | CF₂CF₃ | H |
| 583 | CF₃ | H | CH₃ | CH₂OCH₃ | H |
| 584 | CF₃ | H | CH₃ | OCH₃ | H |
| 585 | CF₃ | H | CH₃ | Ph | H |
| 586 | CF₃ | H | CH₃ | -CH₂Ph | H |
| 587 | CF₃ | CH₃ | H | CH₃ | CH₃ |
| 588 | CF₃ | nBu | H | CH₃ | CH₃ |
| 589 | CF₃ | CF₃ | H | CH₃ | CH₃ |
| 590 | CF₃ | CF₂CF₃ | H | CH₃ | CH₃ |
| 591 | CF₃ | CH₂OCH₃ | H | CH₃ | CH₃ |
| 592 | CF₃ | OCH₃ | H | CH₃ | CH₃ |
| 593 | CF₃ | Ph | H | CH₃ | CH₃ |
| 594 | CF₃ | -CH₂Ph | H | CH₃ | CH₃ |
| 595 | CF₃ | CH₃ | CH₃ | CH₃ | H |
| 596 | CF₃ | CH₃ | CH₃ | nBu | H |
| 597 | CF₃ | CH₃ | CH₃ | CF₃ | H |
| 598 | CF₃ | CH₃ | CH₃ | CF₂CF₃ | H |
| 599 | CF₃ | CH₃ | CH₃ | CH₂OCH₃ | H |
| 600 | CF₃ | CH₃ | CH₃ | OCH₃ | H |
| 601 | CF₃ | CH₃ | CH₃ | Ph | H |
| 602 | CF₃ | CH₃ | CH₃ | -CH₂Ph | H - |
| 603 | CF₃ | nBu | CH₃ | CH₃ | H |
| 604 | CF₃ | CF₃ | CH₃ | CH₃ | H |
| 605 | CF₃ | CF₂CF₃ | CH₃ | CH₃ | H |
| 606 | CF₃ | CH₂OCH₃ | CH₃ | CH₃ | H |
| 607 | CF₃ | OCH₃ | CH₃ | CH₃ | H |
| 608 | CF₃ | Ph | CH₃ | CH₃ | H |
| 609 | CF₃ | -CH₂Ph | CH₃ | CH₃ | H |
| 61C | CF₃ | CH₃ | H | CH₃ | nBu |
| 611 | CF₃ | CH₃ | H | CH₃ | CF₃ |
| 612 | CF₃ | CH₃ | H | CH₃ | CF₂CF₃ |
| 613 | CF₃ | CH₃ | H | CH₃ | CH₂OCH₃ |
| 614 | CF₃ | CH₃ | H | CH₃ | OCH₃ |
| 615 | CF₃ | CH₃ | H | CH₃ | Ph |
| 616 | CF₃ | CH₃ | H | CH₃ | -CH₂Ph |
| 617 | CF₃ | CH₃ | CH₃ | CH₃ | CH₃ |
| 618 | CF₃ | nBu | CH₃ | CH₃ | CH₃ |
| 619 | CF₃ | CF₃ | CH₃ | CH₃ | CH₃ |
| 620 | CF₃ | CF₂CF₃ | CH₃ | CH₃ | CH₃ |
| 621 | CF₃ | CH₂OCH₃ | CH₃ | CH₃ | CH₃ |
| 622 | CF₃ | OCH₃ | CH₃ | CH₃ | CH₃ |
| 623 | CF₃ | Ph | CH₃ | CH₃ | CH₃ |
| 624 | CF₃ | -CH₂Ph | CH₃ | CH₃ | CH₃ |
| 625 | CF₃ | CH₃ | CH₃ | CH₃ | nBu |
| 626 | CF₃ | CH₃ | CH₃ | CH₃ | CF₃ |
| 627 | CF₃ | CH₃ | CH₃ | CH₃ | CF₂CF₃ |
| 628 | CF₃ | CH₃ | CH₃ | CH₃ | CH₂OCH₃ |
| 629 | CF₃ | CH₃ | CH₃ | CH₃ | OCH₃ |
| 630 | CF₃ | CH₃ | CH₃ | CH₃ | Ph |
| 631 | CF₃ | CH₃ | CH₃ | CH₃ | -CH₂Ph |
| 632 | CF₃ | Bu | nBu | H | H |
| 633 | CF₃ | CF₃ | nBu | H | H |
| 634 | CF₃ | CF₂CF₃ | nBu | H | H |
| 635 | CF₃ | CH₂OCH₃ | nBu | H | H |
| 636 | CF₃ | OCH₃ | nBu | H | H |
| 637 | CF₃ | Ph | nBu | H | H |
| 638 | CF₃ | -CH₂Ph | nBu | H | H |
| 639 | CF₃ | H | H | nBu | nBu |
| 640 | CF₃ | H | H | CF₃ | nBu |
| 641 | CF₃ | H | H | CF₂CF₃ | nBu |
| 642 | CF₃ | H | H | CH₂OCH₃ | nBu |
| 643 | CF₃ | H | H | OCH₃ | nBu |
| 644 | CF₃ | H | H | Ph | nBu |
| 645 | CF₃ | H . | H | -CH₂Ph | nBu |
| 646 | CF₃ | nBu | H | H | H |
| 647 | CF₃ | nBu | H | H | CH₃ |
| 648 | CF₃ | nBu | H | H | Bu |
| 649 | CF₃ | nBu | H | H | CF₃ |
| 650 | CF₃ | nBu | H | H | CF₂CF₃ |
| 651 | CF₃ | nBu | H | H | CH₂OCH₃ |
| 652 | CF₃ | nBu | H | H | OCH₃ |
| 653 | CF₃ | nBu | H | H | Ph |
| 654 | CF₃ | nBu | H | H | -CH₂Ph |
| 655 | CF₃ | H | H | nBu | H |
| 656 | CF₃ | H | CH₃ | nBu | H |
| 657 | CF₃ | H | Bu | nBu | H |
| 658 | CF₃ | H | CF₃ | nBu | H |
| 659 | CF₃ | H | CF₂CF₃ | nBu | H |
| 660 | CF₃ | H | CH₂OCH₃ | nBu | H |
| 661 | CF₃ | H | OCH₃ | nBu | H |
| 662 | CF₃ | H | Ph | nBu | H |
| 663 | CF₃ | H | -CH₂Ph | nBu | H |
| 664 | CF₃ | CH₃ | nBu | nBu | H |
| 665 | CF₃ | Bu | nBu | nBu | H |
| 666 | CF₃ | CF₃ | nBu | nBu | H |
| 667 | CF₃ | CF₂CF₃ | nBu | nBu | H |
| 668 | CF₃ | CH₂OCH₃ | nBu | nBu | H |
| 669 | CF₃ | OCH₃ | nBu | nBu | H |
| 670 | CF₃ | Ph | nBu | nBu | H |
| 671 | CF₃ | -CH₂Ph | nBu | nBu | H |
| 672 | CF₃ | CH₃ | H | nBu | nBu |
| 673 | CF₃ | nBu | H | nBu | nBu |
| 674 | CF₃ | CF₃ | H | nBu | nBu |
| 675 | CF₃ | CF₂CF₃ | H | nBu | nBu |
| 676 | CF₃ | CH₂OCH₃ | H | nBu | nBu |
| 677 | CF₃ | OCH₃ | H | nBu | nBu |
| 678 | CF₃ | Ph | H | nBu | nBu |
| 679 | CF₃ | -CH₂Ph | H | nBu | nBu |
| 680 | CF₃ | nBu | nBu | CH₃ | H |
| 681 | CF₃ | nBu | nBu | CF₃ | H |
| 682 | CF₃ | nBu | nBu | CF₂CF₃ | H |
| 683 | CF₃ | nBu | nBu | CH₂OCH₃ | H |
| 684 | CF₃ | nBu | nBu | OCH₃ | H |
| 685 | CF₃ | nBu | nBu | Ph | H |
| 686 | CF₃ | nBu | nBu | -CH₂Ph | H |
| 687 | CF₃ | nBu | H | CH₃ | nBu |
| 688 | CF₃ | nBu | H | CF₃ | nBu |
| 689 | CF₃ | nBu | H | CF₂CF₃ | nBu |
| 690 | CF₃ | nBu | H | CH₂OCH₃ | nBu |
| 691 | CF₃ | nBu | H | OCH₃ | nBu |
| 692 | CF₃ | nBu | H | Ph | nBu |
| 693 | CF₃ | nBu | H | -CH₂Ph | nBu |
| 694 | CF₃ | CH₃ | nBu | nBu | nBu |
| 695 | CF₃ | nBu | nBu | nBu | nBu |
| 696 | CF₃ | CF₃ | nBu | nBu | nBu |
| 697 | CF₃ | CF₂CF₃ | nBu | nBu | nBu |
| 698 | CF₃ | CH₂OCH₃ | nBu | nBu | nBu |
| 699 | CF₃ | OCH₃ | nBu | nBu | nBu |
| 700 | CF₃ | Ph | nBu | nBu | nBu |
| 701 | CF₃ | -CH₂Ph | nBu | nBu | nBu |
| 702 | CF₃ | nBu | nBu | nBu | CH₃ |
| 703 | CF₃ | nBu | nBu | nBu | CF₃ |
| 704 | CF₃ | nBu | nBu | nBu | CF₂CF₃ |
| 705 | CF₃ | nBu | nBu | nBu | CH₂OCH₃ |
| 706 | CF₃ | nBu | nBu | nBu | OCH₃ |
| 707 | CF₃ | nBu | nBu | nBu | Ph |
| 708 | CF₃ | nBu | nBu | nBu | -CH₂Ph |
| 709 | CF₃ | nBu | CH₃ | nBu | CH₃ |
| 710 | CF₃ | nBu | CH₃ | nBu | CF₃ |
| 711 | CF₃ | nBu | CH₃ | nBu | CF₂CF₃ |
| 712 | CF₃ | nBu | CH₃ | nBu | CH₂OCH₃ |
| 713 | CF₃ | nBu | CH₃ | nBu | OCH₃ |
| 714 | CF₃ | nBu | CH₃ | nBu | Ph |
| 715 | CF₃ | nBu | CH₃ | nBu | -CH₂Ph |
| 716 | CF₃ | CF₃ | nBu | nBu | CH₃ |
| 717 | CF₃ | CF₂CF₃ | nBu | nBu | CH₃ |
| 718 | CF₃ | CH₂OCH₃ | nBu | nBu | CH₃ |
| 719 | CF₃ | OCH₃ | nBu | nBu | CH₃ |
| 720 | CF₃ | Ph | nBu | nBu | CH₃ |
| 721 | CF₃ | -CH₂Ph | nBu | nBu | CH₃ |
| 722 | CF₃ | CH₃ | CH₃ | nBu | nBu |
| 723 | CF₃ | CF₃ | CH₃ | nBu | nBu |
| 724 | CF₃ | CF₂CF₃ | CH₃ | nBu | nBu |
| 725 | CF₃ | CH₂OCH₃ | CH₃ | nBu | nBu |
| 726 | CF₃ | OCH₃ | CH₃ | nBu | nBu |
| 727 | CF₃ | Ph | CH₃ | nBu | nBu |
| 728 | CF₃ | -CH₂Ph | CH₃ | nBu | nBu |
| 729 | CF₃ | nBu | nBu | CH₃ | CH₃ |
| 730 | CF₃ | nBu | nBu | CF₃ | CH₃ |
| 731 | CF₃ | nBu | nBu | CF₂CF₃ | CH₃ |
| 732 | CF₃ | nBu | nBu | CH₂OCH₃ | CH₃ |
| 733 | CF₃ | nBu | nBu | OCH₃ | CH₃ |
| 734 | CF₃ | nBu | nBu | Ph | CH₃ |
| 735 | CF₃ | nBu | nBu | -CH₂Ph | CH₃ |
| 736 | CF₃ | nBu | CH₃ | CH₃ | CF₃ |
| 737 | CF₃ | nBu | CH₃ | CH₃ | CF₂CF₃ |
| 738 | CF₃ | nBu | CH₃ | CH₃ | CH₂OCH₃ |
| 739 | CF₃ | nBu | CH₃ | CH₃ | OCH₃ |
| 740 | CF₃ | nBu | CH₃ | CH₃ | Ph |
| 741 | CF₃ | nBu | CH₃ | CH₃ | -CH₂Ph |
| 742 | CF₃ | CF₃ | CH₃ | CH₃ | nBu |
| 743 | CF₃ | CF₂CF₃ | CH₃ | CH₃ | nBu |
| 744 | CF₃ | CH₂OCH₃ | CH₃ | CH₃ | nBu |
| 745 | CF₃ | OCH₃ | CH₃ | CH₃ | nBu |
| 746 | CF₃ | Ph | CH₃ | CH₃ | nBu |
| 747 | CF₃ | -CH₂Ph | CH₃ | CH₃ | nBu |
| 748 | CF₃ | CF₃ | nBu | CH₃ | CH₃ |
| 749 | CF₃ | CF₂CF₃ | nBu | CH₃ | CH₃ |
| 750 | CF₃ | CH₂OCH₃ | nBu | CH₃ | CH₃ |
| 751 | CF₃ | OCH₃ | nBu | CH₃ | CH₃ |
| 752 | CF₃ | Ph | nBu | CH₃ | CH₃ |
| 753 | CF₃ | -CH₂Ph | nBu | CH₃ | CH₃ |
| 754 | CF₃ | CH₃ | CH₃ | CF₃ | nBu |
| 755 | CF₃ | CH₃ | CH₃ | CF₂CF₃ | nBu |
| 756 | CF₃ | CH₃ | CH₃ | CH₂OCH₃ | nBu |
| 757 | CF₃ | CH₃ | CH₃ | OCH₃ | nBu |
| 758 | CF₃ | CH₃ | CH₃ | Ph | nBu |
| 759 | CF₃ | CH₃ | CH₃ | -CH₂Ph | nBu |
| 760 | CF₃ | CF₃ | nBu | H | CH₃ |
| 761 | CF₃ | CF₂CF₃ | nBu | H | CH₃ |
| 762 | CF₃ | CH₂OCH₃ | nBu | H | CH₃ |
| 763 | CF₃ | OCH₃ | nBu | H | CH₃ |
| 764 | CF₃ | Ph | nBu | H | CH₃ |
| 765 | CF₃ | -CH₂Ph | nBu | H | CH₃ |
| 766 | CF₃ | H | CH₃ | CF₃ | nBu |
| 767 | CF₃ | H | CH₃ | CF₂CF₃ | nBu |
| 768 | CF₃ | H | CH₃ | CH₂OCH₃ | nBu |
| 769 | CF₃ | H | CH₃ | OCH₃ | nBu |
| 770 | CF₃ | H | CH₃ | Ph | nBu |
| 771 | CF₃ | H | CH₃ | -CH₂Ph | nBu |
| 772 | CF₃ | nBu | H | CH₃ | CF₃ |
| 773 | CF₃ | nBu | H | CH₃ | CF₂CF₃ |
| 774 | CF₃ | nBu | H | CH₃ | CH₂OCH₃ |
| 775 | CF₃ | nBu | H | CH₃ | OCH₃ |
| 776 | CF₃ | nBu | H | CH₃ | Ph |
| 777 | CF₃ | nBu | H | CH₃ | -CH₂Ph |
| 778 | CF₃ | CF₃ | CH₃ | nBu | H |
| 779 | CF₃ | CF₂CF₃ | CH₃ | nBu | H |
| 780 | CF₃ | CH₂OCH₃ | CH₃ | nBu | H |
| 781 | CF₃ | OCH₃ | CH₃ | nBu | H |
| 782 | CF₃ | Ph | CH₃ | nBu | H |
| 783 | CF₃ | -CH₂Ph | CH₃ | nBu | H |
| 784 | CF₃ | CF₃ | nBu | CH₃ | H |
| 785 | CF₃ | CF₂CF₃ | nBu | CH₃ | H |
| 786 | CF₃ | CH₂OCH₃ | nBu | CH₃ | H |
| 787 | CF₃ | OCH₃ | nBu | CH₃ | H |
| 788 | CF₃ | Ph | nBu | CH₃ | H |
| 789 | CF₃ | -CH₂Ph | nBu | CH₃ | H |
| 790 | CF₃ | CH₃ | H | CF₃ | nBu |
| 791 | CF₃ | CH₃ | H | CF₂CF₃ | nBu |
| 792 | CF₃ | CH₃ | H | CH₂OCH₃ | nBu |
| 793 | CF₃ | CH₃ | H | OCH₃ | nBu |
| 794 | CF₃ | CH₃ | H | Ph | nBu |
| 795 | CF₃ | CH₃ | H | -CH₂Ph | nBu |
| 796 | CF₃ | nBu | CH₃ | H | CF₃ |
| 797 | CF₃ | nBu | CH₃ | H | CF₂CF₃ |
| 798 | CF₃ | nBu | CH₃ | H | CH₂OCH₃ |
| 799 | CF₃ | nBu | CH₃ | H | OCH₃ |
| 800 | CF₃ | nBu | CH₃ | H | Ph |
| 801 | CF₃ | nBu | CH₃ | H | -CH₂Ph |
| 802 | CF₃ | CF₃ | H | CH₃ | nBu |
| 803 | CF₃ | CF₂CF₃ | H | CH₃ | nBu |
| 804 | CF₃ | CH₂OCH₃ | H | CH₃ | nBu |
| 805 | CF₃ | OCH₃ | H | CH₃ | nBu |
| 806 | CF₃ | Ph | H | CH₃ | nBu |
| 807 | CF₃ | -CH₂Ph | H | CH₃ | nBu |
| 808 | Ph | H | H | H | H |
| 809 | Ph | CH₃ | H | H | H |
| 810 | Ph | H | H | CH₃ | H |
| 811 | Ph | CH₃ | CH₃ | H | H |
| 812 | Ph | H | H | CH₃ | CH₃ |
| 813 | Ph | CF₃ | H | H | H |
| 814 | Ph | H | H | CF₃ | H |
| 815 | OH | H | H | H | H |
| 816 | OH | CH₃ | H | H | H |
| 817 | OH | H | H | CH₃ | H |
| 818 | OH | CH₃ | CH₃ | H | H |
| 819 | OH | H | H | CH₃ | CH₃ |
| 820 | OH | H | H | CF₃ | H |
| 821 | OH | CF₃ | H | H | CH₃ |
| 822 | OH | CF₃ | H | H | H |
| 823 | OH | CH₃ | H | CF₃ | H |
| 824 | OH | H | H | CH₂OCH₃ | H |
| 825 | OH | CH₂OCH₃ | H | H | H |
| 826 | OH | CH₂OCH₃ | H | CH₃ | H |
| 827 | OH | H | CH₃ | CH₂OCH₃ | H |
| 828 | OH | CH₂OCH₃ | H | CH₃ | H |
| 829 | OH | H | CH₃ | CH₂OCH₃ | H |
| 830 | CH₃ | H | (CH₂)₄ | | H |

**Table B-8**

| | | | | | |
|---|---|---|---|---|---|
| 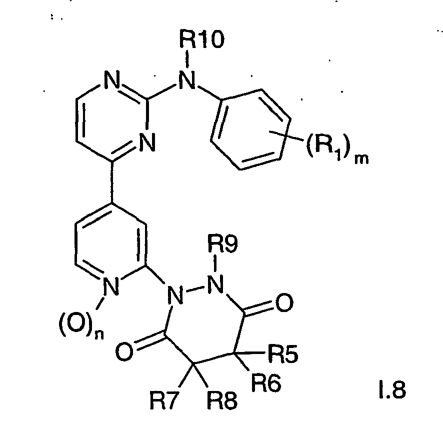 | | | | | |

| | R₅ | R₆ | R₇ | R₈ | R₉ |
|---|---|---|---|---|---|
| 1. | H | H | H | H | H |
| 2. | CH₃ | H | H | H | H |
| 3. | n-Bu | H | H | H | H |
| 4. | CF₃ | H | H | H | H |
| 5. | CF₂CF₃ | H | H | H | H |
| 6. | CH₂OCH₃ | H | H | H | H |
| 7. | OCH₃ | H | H | H | H |
| 8. | Ph | H | H | H | H |
| 9. | -CH₂Ph | H | H | H | H |
| 10. | H | H | CH₃ | H | H |
| 11. | H | H | n-Bu | H | H |
| 12. | H | H | CF₃ | H | H |
| 13. | H | H | CF₂CF₃ | H | H |
| 14. | H | H | CH₂OCH₃ | H | H |
| 15. | H | H | OCH₃ | H | H |
| 16. | H | H | Ph | H | H |
| 17. | H | H | -CH₂Ph | H | H |
| 18. | CH₃ | CH₃ | H | H | H |
| 19. | n-Bu | CH₃ | H | H | H |
| 20. | CF₃ | CH₃ | H | H | H |
| 21. | CF₂CF₃ | CH₃ | H | H | H |
| 22. | CH₂OCH₃ | CH₃ | H | H | H |
| 23. | OCH₃ | CH₃ | H | H | H |
| 24. | Ph | CH₃ | H | H | H |
| 25. | -CH₂Ph | CH₃ | H | H | H |
| 26. | H | H | CH₃ | CH₃ | H |
| 27. | H | H | CH₃ | n-Bu | H |
| 28. | H | H | CH₃ | CF₃ | H |
| 29. | H | H | CH₃ | CF₂CF₃ | H |
| 30. | H | H | CH₃ | CH₂OCH₃ | H |
| 31. | H | H | CH₃ | OCH₃ | H |
| 32. | H | H | CH₃ | Ph | H |
| 33. | H | H | CH₃ | CH₂Ph | H |
| 34. | CH₃ | H | H | CH₃ | H |
| 35. | n-Bu | H | H | CH₃ | H |
| 36. | CF₃ | H | H | CH₃ | H |
| 37. | CF₂CF₃ | H | H | CH₃ | H |
| 38. | CH₂OCH₃ | H | H | CH₃ | H |
| 39. | OCH₃ | H | H | CH₃ | H |
| 40. | Ph | H | H | CH₃ | H |
| 41. | -CH₂Ph | H | H | CH₃ | H |
| 42. | H | CH₃ | n-Bu | H | H |
| 43. | H | CH₃ | CF₃ | H | H |
| 44. | H | CH₃ | CF₂CF₃ | H | H |
| 45. | H | CH₃ | CH₂OCH₃ | H | H |
| 46. | H | CH₃ | OCH₃ | H | H |
| 47. | H | CH₃ | Ph | H | H |
| 48. | H | CH₃ | -CH₂Ph | H | H |
| 49. | CH₃ | H | CH₃ | CH₃ | H |
| 50. | n-Bu | H | CH₃ | CH₃ | H |
| 51. | CF₃ | H | CH₃ | CH₃ | H |
| 52. | CF₂CF₃ | H | CH₃ | CH₃ | H |
| 53. | CH₂OCH₃ | H | CH₃ | CH₃ | H |
| 54. | OCH₃ | H | CH₃ | CH₃ | H |
| 55. | Ph | H | CH₃ | CH₃ | H |
| 56. | CH₂Ph | H | CH₃ | CH₃ | H |
| 57. | CH₃ | CH₃ | CH₃ | H | H |
| 58. | CH₃ | CH₃ | n-Bu | H | H |
| 59. | CH₃ | CH₃ | CF₃ | H | H |
| 60. | CH₃ | CH₃ | CF₂CF₃ | H | H |
| 61. | CH₃ | CH₃ | CH₂OCH₃ | H | H |
| 62. | CH₃ | CH₃ | OCH₃ | H | H |
| 63. | CH₃ | CH₃ | Ph | H | H |
| 64. | CH₃ | CH₃ | -CH₂Ph | II | H |
| 65. | n-Bu | CH₃ | CH₃ | H | H |
| 66. | CF₃ | CH₃ | CH₃ | H | H |
| 67. | CF₂CF₃ | CH₃ | CH₃ | H | H |
| 68. | CH₂OCH₃ | CH₃ | CH₃ | H | H |
| 69. | OCH₃ | CH₃ | CH₃ | H | H |
| 70. | Ph | CH₃ | CH₃ | H | H |
| 71. | CH₂Ph | CH₃ | CH₃ | H | H |
| 72. | CH₃ | H | CH₃ | n-Bu | H |
| 73. | CH₃ | H | CH₃ | CF₃ | H |
| 74. | CH₃ | H | CH₃ | CF₂CF₃ | H |
| 75. | CH₃ | H | CH₃ | CH₂OCH₃ | H |
| 76. | CH₃ | H | CH₃ | OCH₃ | H |
| 77. | CH₃ | H | CH₃ | Ph | H |
| 78. | CH₃ | H | CH₃ | CH₂Ph | H |
| 79. | CH₃ | CH₃ | CH₃ | CH₃ | H |
| 80. | n-Bu | CH₃ | CH₃ | CH₃ | H |
| 81. | CF₃ | CH₃ | CH₃ | CH₃ | H |
| 82. | CF₂CF₃ | CH₃ | CH₃ | CH₃ | H |
| 83. | CH₂OCH₃ | CH₃ | CH₃ | CH₃ | H |
| 84. | OCH₃ | CH₃ | CH₃ | CH₃ | H |
| 85. | Ph | CH₃ | CH₃ | CH₃ | H |
| 86. | CH₂Ph | CH₃ | CH₃ | CH₃ | H |
| 87. | CH₃ | CH₃ | CH₃ | n-Bu | H |
| 88. | CH₃ | CH₃ | CH₃ | CF₃ | H |
| 89. | CH₃ | CH₃ | CH₃ | CF₂CF₃ | H |
| 90. | CH₃ | CH₃ | CH₃ | CH₂OCH₃ | H |
| 91. | CH₃ | CH₃ | CH₃ | OCH₃ | H |
| 92. | CH₃ | CH₃ | CH₃ | Ph | H |
| 93. | CH₃ | CH₃ | CH₃ | CH₂Ph | H |
| 94. | n-Bu | n-Bu | H | H | H |
| 95. | CF₃ | n-Bu | H | H | H |
| 96. | CF₂CF₃ | n-Bu | H | H | H |
| 97. | CH₂OCH₃ | n-Bu | H | H | H |
| 98. | OCH₃ | n-Bu | H | H | H |
| 99. | Ph | n-Bu | H | H | H |
| 100. | CH₂Ph | n-Bu | H | H | H |
| 101. | H | H | n-Bu | n-Bu | H |
| 102. | H | H | CF₃ | n-Bu | H |
| 103. | H | H | CF₂CF₃ | n-Bu | H |
| 104. | H | H | CH₂OCH₃ | n-Bu | H |
| 105. | H | H | OCH₃ | n-Bu | H |
| 106. | H | H | Ph | n-Bu | H |
| 107. | H | H | -CH₂Ph | n-Bu | H |
| 108. | n-Bu | H | H | H | H |
| 109. | n-Bu | H | H | CH₃ | H |
| 110. | n-Bu | H | H | v | H |
| 111. | n-Bu | H | H | CF₃ | H |
| 112. | n-Bu | H | H | CF₂CF₃ | H |
| 113. | n-Bu | H | H | CH₂OCH₃ | H |
| 114. | n-Bu | H | H | OCH₃ | H |
| 115. | n-Bu | H | H | Ph | H |
| 116. | n-Bu | H | H | -CH₂Ph | H |
| 117. | H | H | n-Bu | H | H |
| 118. | H | CH₃ | n-Bu | H | H |
| 119. | H | n-Bu | n-Bu | H | H |
| 120. | H | CF₃ | n-Bu | H | H |
| 121. | H | CF₂CF₃ | n-Bu | H | H |
| 122. | H | CH₂OCH₃ | n-Bu | H | H |
| 123. | H | OCH₃ | n-Bu | H | H |
| 124. | H | Ph | n-Bu | H | H |
| 125. | H | -CH₂Ph | n-Bu | H | H |
| 126. | CH₃ | n-Bu | n-Bu | H | H |
| 127. | n-Bu | n-Bu | n-Bu | H | H |
| 128. | CF₃ | n-Bu | n-Bu | H | H |
| 129. | CF₂CF₃ | n-Bu | n-Bu | H | H |
| 130. | CH₂OCH₃ | n-Bu | n-Bu | H | H |
| 131. | OCH₃ | n-Bu | n-Bu | H | H |
| 132. | Ph | n-Bu | n-Bu | H | H |
| 133. | -CH₂Ph | n-Bu | n-Bu | H | H |
| 134. | CH₃ | H | n-Bu | n-Bu | H |
| 135. | n-Bu | H | n-Bu | n-Bu | H |
| 136. | CF₃ | H | n-Bu | n-Bu | H |
| 137. | CF₂CF₃ | H | n-Bu | n-Bu | H |
| 138. | CH₂OCH₃ | H | n-Bu | n-Bu | H |
| 139. | OCH₃ | H | n-Bu | n-Bu | H |
| 140. | Ph | H | n-Bu | n-Bu | H |
| 141. | -CH₂Ph | H | n-Bu | n-Bu | H |
| 142. | n-Bu | n-Bu | CH₃ | H | H |
| 143. | n-Bu | n-Bu | CF₃ | H | H |
| 144. | n-Bu | n-Bu | CF₂CF₃ | H | H |
| 145. | n-Bu | n-Bu | CH₂OCH₃ | H | H |
| 146. | n-Bu | n-Bu | OCH₃ | H | H |
| 147. | n-Bu | n-Bu | Ph | H | H |
| 148. | n-Bu | n-Bu | -CH₂Ph | H | H |
| 149. | n-Bu | H | CH₃ | n-Bu | H |
| 150. | n-Bu | H | CF₃ | n-Bu | H |
| 151. | n-Bu | H | CF₂CF₃ | n-Bu | H |
| 152. | n-Bu | H | CH₂OCH₃ | n-Bu | H |
| 153. | n-Bu | H | OCH₃ | n-Bu | H |
| 154. | n-Bu | H | Ph | n-Bu | H |
| 155. | n-Bu | H | -CH₂Ph | n-Bu | H |
| 156. | CH₃ | n-Bu | n-Bu | n-Bu | H |
| 157. | n-Bu | n-Bu | n-Bu | n-Bu | H |
| 158. | CF₃ | n-Bu | n-Bu | n-Bu | H |
| 159. | CF₂CF₃ | n-Bu | n-Bu | n-Bu | H |
| 160. | CH₂OCH₃ | n-Bu | n-Bu | n-Bu | H |
| 161. | OCH₃ | n-Bu | n-Bu | n-Bu | H |
| 162. | Ph | n-Bu | n-Bu | n-Bu | H |
| 163. | -CH₂Ph | n-Bu | n-Bu | n-Bu | H |
| 164. | n-Bu | n-Bu | n-Bu | CH₃ | H |
| 165. | n-Bu | n-Bu | n-Bu | CF₃ | H |
| 166. | n-Bu | n-Bu | n-Bu | CF₂CF₃ | H |
| 167. | n-Bu | n-Bu | n-Bu | CH₂OCH₃ | H |
| 168. | n-Bu | n-Bu | n-Bu | OCH₃ | H |
| 169. | n-Bu | n-Bu | n-Bu | Ph | H |
| 170. | n-Bu | n-Bu | n-Bu | -CH₂Ph | H |
| 171. | n-Bu | CH₃ | n-Bu | CH₃ | H |
| 172. | n-Bu | CH₃ | n-Bu | CF₃ | H |
| 173. | n-Bu | CH₃ | n-Bu | CF₂CF₃ | H |
| 174. | n-Bu | CH₃ | n-Bu | CH₂OCH₃ | H |
| 175. | n-Bu | CH₃ | n-Bu | OCH₃ | H |
| 176. | n-Bu | CH₃ | n-Bu | Ph | H |
| 177. | n-Bu | CH₃ | n-Bu | -CH₂Ph | H |
| 178. | CF₃ | n-Bu | n-Bu | CH₃ | H |
| 179. | CF₂CF₃ | n-Bu | n-Bu | CH₃ | H |
| 180. | CH₂OCH₃ | n-Bu | n-Bu | CH₃ | H |
| 181. | OCH₃ | n-Bu | n-Bu | CH₃ | H |
| 182. | Ph | n-Bu | n-Bu | CH₃ | H |
| 183. | -CH₂Ph | n-Bu | n-Bu | CH₃ | H |
| 184. | CH₃ | CH₃ | n-Bu | n-Bu | H |
| 185. | CF₃ | CH₃ | n-Bu | n | H |
| 186. | CF₂CF₃ | CH₃ | n-Bu | n | H |
| 187. | CH₂OCH₃ | CH₃ | n-Bu | n | H |
| 188. | OCH₃ | CH₃ | n-Bu | n | H |
| 189. | Ph | CH₃ | n-Bu | n | H |
| 190. | -CH₂Ph | CH₃ | n-Bu | n | H |
| 191. | n-Bu | n-Bu | CH₃ | CH₃ | H |
| 192. | n-Bu | n-Bu | CF₃ | CH₃ | H |
| 193. | n-Bu | n-Bu | CF₂CF₃ | CH₃ | H |
| 194. | n-Bu | n-Bu | CH₂OCH₃ | CH₃ | H |
| 195. | n-Bu | n-Bu | OCH₃ | CH₃ | H |
| 196. | n-Bu | n-Bu | Ph | CH₃ | H |
| 197. | n-Bu | n-Bu | -CH₂Ph | CH₃ | H |
| 198. | n-Bu | CH₃ | CH₃ | CF₃ | H |
| 199. | n-Bu | CH₃ | CH₃ | CF₂CF₃ | H |
| 200. | n-Bu | CH₃ | CH₃ | CH₂OCH₃ | H |
| 201. | n-Bu | CH₃ | CH₃ | OCH₃ | H |
| 202. | n-Bu | CH₃ | CH₃ | Ph | H |
| 203. | n-Bu | CH₃ | CH₃ | -CH₂Ph | H |
| 204. | CF₃ | CH₃ | CH₃ | n-Bu | H |
| 205. | CF₂CF₃ | CH₃ | CH₃ | n-Bu | H |
| 206. | CH₂OCH₃ | CH₃ | CH₃ | n-Bu | H |
| 207. | OCH₃ | CH₃ | CH₃ | n-Bu | H |
| 208. | Ph | CH₃ | CH₃ | n-Bu | H |
| 209. | -CH₂Ph | CH₃ | CH₃ | n-Bu | H |
| 210. | CF₃ | n-Bu | CH₃ | CH₃ | H |
| 211. | CF₂CF₃ | n-Bu | CH₃ | CH₃ | H |
| 212. | CH₂OCH₃ | n-Bu | CH₃ | CH₃ | H |
| 213. | OCH₃ | n-Bu | CH₃ | CH₃ | H |
| 214. | Ph | n-Bu | CH₃ | CH₃ | H |
| 215. | -CH₂Ph | n-Bu | CH₃ | CH₃ | H |
| 216. | CH₃ | CH₃ | CF₃ | n-Bu | H |
| 217. | CH₃ | CH₃ | CF₂CF₃ | n-Bu | H |
| 218. | CH₃ | CH₃ | CH₂OCH₃ | n-Bu | H |
| 219. | CH₃ | CH₃ | OCH₃ | n-Bu | H |
| 220. | CH₃ | CH₃ | Ph | n-Bu | H |
| 221. | CH₃ | CH₃ | -CH₂Ph | n-Bu | H |
| 222. | CF₃ | n-Bu | H | CH₃ | H |
| 223. | CF₂CF₃ | n-Bu | H | CH₃ | H |
| 224. | CH₂OCH₃ | n-Bu | H | CH₃ | H |
| 225. | OCH₃ | n-Bu | H | CH₃ | H |
| 226. | Ph | n-Bu | H | CH₃ | H |
| 227. | -CH₂Ph | n-Bu | H | CH₃ | H |
| 228. | H | CH₃ | CF₃ | n-Bu | H |
| 229. | H | CH₃ | CF₂CF₃ | n-Bu | H |
| 230. | H | CH₃ | CH₂OCH | n-Bu | H |
| 231. | H | CH₃ | OCH₃ | n-Bu | H |
| 232. | H | CH₃ | Ph | n-Bu | H |
| 233. | H | CH₃ | -CH₂Ph | n-Bu | H |
| 234. | n-Bu | H | CH₃ | CF₃ | H |
| 235. | n-Bu | H | CH₃ | CF₂CF₃ | H |
| 236. | n-Bu | H | CH₃ | CH₂OCH₃ | H |
| 237. | n-Bu | H | CH₃ | OCH₃ | H |
| 238. | n-Bu | H | CH₃ | Ph | H |
| 239. | n-Bu | H | CH₃ | -CH₂Ph | H |
| 240. | CF₃ | CH₃ | n-Bu | H | H |
| 241. | CF₂CF₃ | CH₃ | n-Bu | H | H |
| 242. | CH₂OCH₃ | CH₃ | n-Bu | H | H |
| 243. | OCH₃ | CH₃ | n-Bu | H | H |
| 244. | Ph | CH₃ | n-Bu | H | H |
| 245. | -CH₂Ph | CH₃ | n-Bu | H | H |
| 246. | CF₃ | n-Bu | CH₃ | H | H |
| 247. | CF₂CF₃ | n-Bu | CH₃ | H | H |
| 248. | CH₂OCH₃ | n-Bu | CH₃ | H | H |
| 249. | OCH₃ | n-Bu | CH₃ | H | H |
| 250. | Ph | n-Bu | CH₃ | H | H |
| 251. | -CH₂Ph | n-Bu | CH₃ | H | H |
| 252. | CH₃ | H | CF₃ | n-Bu | H |
| 253. | CH₃ | H | CF₂CF₃ | n-Bu | H |
| 254. | CH₃ | H | CH₂OCH₃ | n-Bu | H |
| 255. | CH₃ | H | OCH₃ | n-Bu | H |
| 256. | CH₃ | H | Ph | n-Bu | H |
| 257. | CH₃ | H | -CH₂Ph | n-Bu | H |
| 258. | n-Bu | CH₃ | H | CF₃ | H |
| 259. | n-Bu | CH₃ | H | CF₂CF₃ | H |
| 260. | n-Bu | CH₃ | H | CH₂OCH₃ | H |
| 261. | n-Bu | CH₃ | H | OCH₃ | H |
| 262. | n-Bu | CH₃ | H | Ph | H |
| 263. | n-Bu | CH₃ | H | -CH₂Ph | H |
| 264. | CF₃ | H | CH₃ | n-Bu | H |
| 265. | CF₂CF₃ | H | CH₃ | n-Bu | H |
| 266. | CH₂OCH₃ | H | CH₃ | n-Bu | H |
| 267. | OCH₃ | H | CH₃ | n-Bu | H |
| 268. | Ph | H | CH₃ | n-Bu | H |
| 269. | -CH₂Ph | H | CH₃ | n-Bu | H |
| 270. | H | H | H | H | CH₃ |
| 271. | CH₃ | H | H | H | CH₃ |
| 272. | n-Bu | H | H | H | CH₃ |
| 273. | CF₃ | H | H | H | CH₃ |
| 274. | CF₂CF₃ | H | H | H | CH₃ |
| 275. | CH₂OCH₃ | H | H | H | CH₃ |
| 276. | OCH₃ | H | H | H | CH₃ |
| 277. | Ph | H | H | H | CH₃ |
| 278. | -CH₂Ph | H | H | H | CH₃ |
| 279. | H | H | CH₃ | H | CH₃ |
| 280. | H | H | n-Bu | H | CH₃ |
| 281. | H | H | CF₃ | H | CH₃ |
| 282. | H | H | CF₂CF₃ | H | CH₃ |
| 283. | H | H | CH₂OCH₃ | H | CH₃ |
| 284. | H | H | OCH₃ | H | CH₃ |
| 285. | H | H | Ph | H | CH₃ |
| 286. | H | H | -CH₂Ph | H | CH₃ |
| 287. | CH₃ | CH₃ | H | H | CH₃ |
| 288. | n-Bu | CH₃ | H | H | CH₃ |
| 289. | CF₃ | CH₃ | H | H | CH₃ |
| 290. | CF₂CF₃ | CH₃ | H | H | CH₃ |
| 291. | CH₂OCH₃ | CH₃ | H | H | CH₃ |
| 292. | OCH₃ | CH₃ | H | H | CH₃ |
| 293. | Ph | CH₃ | H | H | CH₃ |
| 294. | -CH₂Ph | CH₃ | H | H | CH₃ |
| 295. | H | H | CH₃ | CH₃ | CH₃ |
| 296. | H | H | CH₃ | n-Bu | CH₃ |
| 297. | H | H | CH₃ | CF₃ | CH₃ |
| 298. | H | H | CH₃ | CF₂CF₃ | CH₃ |
| 299. | H | H | CH₃ | CH₂OCH₃ | CH₃ |
| 300. | H | H | CH₃ | OCH₃ | CH₃ |
| 301. | H | H | CH₃ | Ph | CH₃ |
| 302. | H | H | CH₃ | -CH₂Ph | CH₃ |
| 303. | CH₃ | H | H | CH₃ | CH₃ |
| 304. | n-Bu | H | H | CH₃ | CH₃ |
| 305. | CF₃ | H | H | CH₃ | CH₃ |
| 306. | CF₂CF₃ | H | H | CH₃ | CH₃ |
| 307. | CH₂OCH₃ | H | H | CH₃ | CH₃ |
| 308. | OCH₃ | H | H | CH₃ | CH₃ |
| 309. | Ph | H | H | CH₃ | CH₃ |
| 310. | -CH₂Ph | H | H | CH₃ | CH₃ |
| 311. | H | CH₃ | n-Bu | H | CH₃ |
| 312. | H | CH₃ | CF₃ | H | CH₃ |
| 313. | H | CH₃ | CF₂CF₃ | H | CH₃ |
| 314. | H | CH₃ | CH₂OCH₃ | H | CH₃ |
| 315. | H | CH₃ | OCH₃ | H | CH₃ |
| 316. | H | CH₃ | Ph | H | CH₃ |
| 317. | H | CH₃ | -CH₂Ph | H | CH₃ |
| 318. | CH₃ | H | CH₃ | CH₃ | CH₃ |
| 319. | n-Bu | H | CH₃ | CH₃ | CH₃ |
| 320. | CF₃ | H | CH₃ | CH₃ | CH₃ |
| 321. | CF₂CF₃ | H | CH₃ | CH₃ | CH₃ |
| 322. | CH₂OCH₃ | H | CH₃ | CH₃ | CH₃ |
| 323. | OCH₃ | H | CH₃ | CH₃ | CH₃ |
| 324. | Ph | H | CH₃ | CH₃ | CH₃ |
| 325. | -CH₂Ph | H | CH₃ | CH₃ | CH₃ |
| 326. | CH₃ | CH₃ | CH₃ | H | CH₃ |
| 327. | CH₃ | CH₃ | n-Bu | H | CH₃ |
| 328. | CH₃ | CH₃ | CF₃ | H | CH₃ |
| 329. | CH₃ | CH₃ | CF₂CF₃ | H | CH₃ |
| 330. | CH₃ | CH₃ | CH₂OCH₃ | H | CH₃ |
| 331. | CH₃ | CH₃ | OCH₃ | H | CH₃ |
| 332. | CH₃ | CH₃ | Ph | H | CH₃ |
| 333. | CH₃ | CH₃ | -CH₂Ph | H | CH₃ |
| 334. | n-Bu | CH₃ | CH₃ | H | CH₃ |
| 335. | CF₃ | CH₃ | CH₃ | H | CH₃ |
| 336. | CF₂CF₃ | CH₃ | CH₃ | H | CH₃ |
| 337. | CH₂OCH₃ | CH₃ | CH₃ | H | CH₃ |
| 338. | OCH₃ | CH₃ | CH₃ | H | CH₃ |
| 339. | Ph | CH₃ | CH₃ | H | CH₃ |
| 340. | -CH₂Ph | CH₃ | CH₃ | H | CH₃ |
| 341. | CH₃ | H | CH₃ | n-Bu | CH₃ |
| 342. | CH₃ | H | CH₃ | CF₃ | CH₃ |
| 343. | CH₃ | H | CH₃ | CF₂CF₃ | CH₃ |
| 344. | CH₃ | H | CH₃ | CH₂OCH₃ | CH₃ |
| 345. | CH₃ | H | CH₃ | OCH₃ | CH₃ |
| 346. | CH₃ | H | CH₃ | Ph | CH₃ |
| 347. | CH₃ | H | CH₃ | -CH₂Ph | CH₃ |
| 348. | CH₃ | CH₃ | CH₃ | CH₃ | CH₃ |
| 349. | n-Bu | CH₃ | CH₃ | CH₃ | CH₃ |
| 350. | CF₃ | CH₃ | CH₃ | CH₃ | CH₃ |
| 351. | CF₂CF₃ | CH₃ | CH₃ | CH₃ | CH₃ |
| 352. | CH₂OCH₃ | CH₃ | CH₃ | CH₃ | CH₃ |
| 353. | OCH₃ | CH₃ | CH₃ | CH₃ | CH₃ |
| 354. | Ph | CH₃ | CH₃ | CH₃ | CH₃ |
| 355. | -CH₂Ph | CH₃ | CH₃ | CH₃ | CH₃ |
| 356. | CH₃ | CH₃ | CH₃ | n-Bu | CH₃ |
| 357. | CH₃ | CH₃ | CH₃ | CF₃ | CH₃ |
| 358. | CH₃ | CH₃ | CH₃ | CF₂CF₃ | CH₃ |
| 359. | CH₃ | CH₃ | CH₃ | CH₂OCH₃ | CH₃ |
| 360. | CH₃ | CH₃ | CH₃ | OCH₃ | CH₃ |
| 361. | CH₃ | CH₃ | CH₃ | Ph | CH₃ |
| 362. | CH₃ | CH₃ | CH₃ | -CH₂Ph | CH₃ |
| 363. | n-Bu | n-Bu | H | H | CH₃ |
| 364. | CF₃ | n-Bu | H | H | CH₃ |
| 365. | CF₂CF₃ | n-Bu | H | H | CH₃ |
| 366. | CH₂OCH₃ | n-Bu | H | H | CH₃ |
| 367. | OCH₃ | n-Bu | H | H | CH₃ |
| 368. | Ph | n-Bu | H | H | CH₃ |
| 369. | -CH₂Ph | n-Bu | H | H | CH₃ |
| 370. | H | H | n-Bu | n-Bu | CH₃ |
| 371. | H | H | CF₃ | n-Bu | CH₃ |
| 372. | H | H | CF₂CF₃ | n-Bu | CH₃ |
| 373. | H | H | CH₂OCH₃ | n-Bu | CH₃ |
| 374. | H | H | OCH₃ | n-Bu | CH₃ |
| 375. | H | H | Ph | n-Bu | CH₃ |
| 376. | H | H | -CH₂Ph | n-Bu | CH₃ |
| 377. | n-Bu | H | H | H | CH₃ |
| 378. | n-Bu | H | H | CH₃ | CH₃ |
| 379. | n-Bu | H | H | n-Bu | CH₃ |
| 380. | n-Bu | H | H | CF₃ | CH₃ |
| 381. | n-Bu | H | H | CF₂CF₃ | CH₃ |
| 382. | n-Bu | H | H | CH₂OCH₃ | CH₃ |
| 383. | n-Bu | H | H | OCH₃ | CH₃ |
| 384. | n-Bu | H | H | Ph | CH₃ |
| 385. | n-Bu | H | H | -CH₂Ph | CH₃ |
| 386. | H | H | n-Bu | H | CH₃ |
| 387. | H | CH₃ | n-Bu | H | CH₃ |
| 388. | H | n-Bu | n-Bu | H | CH₃ |
| 389. | H | CF₃ | n-Bu | H | CH₃ |
| 390. | H | CF₂CF₃ | n-Bu | H | CH₃ |
| 391. | H | CH₂OCH₃ | n-Bu | H | CH₃ |
| 392. | H | OCH₃ | n-Bu | H | CH₃ |
| 393. | H | Ph | n-Bu | H | CH₃ |
| 394. | H | -CH₂Ph | n-Bu | H | CH₃ |
| 395. | CH₃ | n-Bu | n-Bu | H | CH₃ |
| 396. | n-Bu | n-Bu | n-Bu | H | CH₃ |
| 397. | CF₃ | n-Bu | n-Bu | H | CH₃ |
| 398. | CF₂CF₃ | n-Bu | n-Bu | H | CH₃ |
| 399. | CH₂OCH₃ | n-Bu | n-Bu | H | CH₃ |
| 400. | OCH₃ | n-Bu | n-Bu | H | CH₃ |
| 401. | Ph | n-Bu | n-Bu | H | CH₃ |
| 402. | -CH₂Ph | n-Bu | n-Bu | H | CH₃ |
| 403. | CH₃ | H | n-Bu | n-Bu | CH₃ |
| 404. | n-Bu | H | n-Bu | n-Bu | CH₃ |
| 405. | CF₃ | H | n-Bu | n-Bu | CH₃ |
| 406. | CF₂CF₃ | H | n-Bu | n-Bu | CH₃ |
| 407. | CH₂OCH₃ | H | n-Bu | n-Bu | CH₃ |
| 408. | OCH₃ | H | n-Bu | n-Bu | CH₃ |
| 409. | Ph | H | n-Bu | n-Bu | CH₃ |
| 410. | -CH₂Ph | H | n-Bu | n-Bu | CH₃ |
| 411. | n-Bu | n-Bu | CH₃ | H | CH₃ |
| 412. | n-Bu | n-Bu | CF₃ | H | CH₃ |
| 413. | n-Bu | n-Bu | CF₂CF₃ | H | CH₃ |
| 414. | n-Bu | n-Bu | CH₂OCH | H | CH₃ |
| 415. | n-Bu | n-Bu | OCH₃ | H | CH₃ |
| 416. | n-Bu | n-Bu | Ph | H | CH₃ |
| 417. | n-Bu | n-Bu | -CH₂Ph | H | CH₃ |
| 418. | n-Bu | H | CH₃ | n-Bu | CH₃ |
| 419. | n-Bu | H | CF₃ | n-Bu | CH₃ |
| 420. | n-Bu | H | CF₂CF₃ | n-Bu | CH₃ |
| 421. | n-Bu | H | CH₂OCH₃ | n-Bu | CH₃ |
| 422. | n-Bu | H | OCH₃ | n-Bu | CH₃ |
| 423. | n-Bu | H | Ph | n-Bu | CH₃ |
| 424. | n-Bu | H | -CH₂Ph | n-Bu | CH₃ |
| 425. | CH₃ | n-Bu | n-Bu | n-Bu | CH₃ |
| 426. | n-Bu | n-Bu | n-Bu | n-Bu | CH₃ |
| 427. | CF₃ | n-Bu | n-Bu | n-Bu | CH₃ |
| 428. | CF₂CF₃ | n-Bu | n-Bu | n-Bu | CH₃ |
| 429. | CH₂OCH₃ | n-Bu | n-Bu | n-Bu | CH₃ |
| 430. | OCH₃ | n-Bu | n-Bu | n-Bu | CH₃ |
| 431. | Ph | n-Bu | n-Bu | n-Bu | CH₃ |
| 432. | -CH₂Ph | n-Bu | n-Bu | n-Bu | CH₃ |
| 433. | n-Bu | n-Bu | n-Bu | CH₃ | CH₃ |
| 434. | n-Bu | n-Bu | n-Bu | CF₃ | CH₃ |
| 435. n-Bu | | n-Bu | n-Bu | CF₂CF₃ | CH₃ |
| 436. | n-Bu | n-Bu | n-Bu | CH₂OCH₃ | CH₃ |
| 437. | n-Bu | n-Bu | n-Bu | OCH₃ | CH₃ |
| 438. | n-Bu | n-Bu | n-Bu | Ph | CH₃ |
| 439. | n-Bu | n-Bu | n-Bu | -CH₂Ph | CH₃ |
| 440. | n-Bu | CH₃ | n-Bu | CH₃ | CH₃ |
| 441. | n-Bu | CH₃ | n-Bu | CF₃ | CH₃ |
| 442. | n-Bu | CH₃ | n-Bu | CF₂CF₃ | CH₃ |
| 443. | n-Bu | CH₃ | n-Bu | CH₂OCH₃ | CH₃ |
| 444. | n-Bu | CH₃ | n-Bu | OCH₃ | CH₃ |
| 445. | n-Bu | CH₃ | n-Bu | Ph | CH₃ |
| 446. | n-Bu | CH₃ | n-Bu | -CH₂Ph | CH₃ |
| 447. | CF₃ | n-Bu | n-Bu | CH₃ | CH₃ |
| 448. | CF₂CF₃ | n-Bu | n-Bu | CH₃ | CH₃ |
| 449. | CH₂OCH₃ | n-Bu | n-Bu | CH₃ | CH₃ |
| 450. | OCH₃ | n-Bu | n-Bu | CH₃ | CH₃ |
| 451. | Ph | n-Bu | n-Bu | CH₃ | CH₃ |
| 452. | -CH₂Ph | n-Bu | n-Bu | CH₃ | CH₃ |
| 453. | CH₃ | CH₃ | n-Bu | n-Bu | CH₃ |
| 454. | CF₃ | CH₃ | n-Bu | n-Bu | CH₃ |
| 455. | CF₂CF₃ | CH₃ | n-Bu | n-Bu | CH₃ |
| 456. | CH₂OCH₃ | CH₃ | n-Bu | n-Bu | CH₃ |
| 457. | OCH₃ | CH₃ | n-Bu | n-Bu | CH₃ |
| 458. | Ph | CH₃ | n-Bu | n-Bu | CH₃ |
| 459. | -CH₂Ph | CH₃ | n-Bu | n-Bu | CH₃ |
| 460. | n-Bu | n-Bu | CH₃ | CH₃ | CH₃ |
| 461. | n-Bu | n-Bu | CF₃ | CH₃ | CH₃ |
| 462. | n-Bu | n-Bu | CF₂CF₃ | CH₃ | CH₃ |
| 463. | n-Bu | n-Bu | CH₂OCH₃ | CH₃ | CH₃ |
| 464. | n-Bu | n-Bu | OCH₃ | CH₃ | CH₃ |
| 465. | n-Bu | n-Bu | Ph | CH₃ | CH₃ |
| 466. | n-Bu | n-Bu | -CH₂Ph | CH₃ | CH₃ |
| 467. | n-Bu | CH₃ | CH₃ | CF₃ | CH₃ |
| 468. | n-Bu | CH₃ | CH₃ | CF₂CF₃ | CH₃ |
| 469. | n-Bu | CH₃ | CH₃ | CH₂OCH₃ | CH₃ |
| 470. | n-Bu | CH₃ | CH₃ | OCH₃ | CH₃ |
| 471. | n-Bu | CH₃ | CH₃ | Ph | CH₃ |
| 472. | n-Bu | CH₃ | CH₃ | -CH₂Ph | CH₃ |
| 473. | CF₃ | CH₃ | CH₃ | n-Bu | CH₃ |
| 474. | CF₂CF₃ | CH₃ | CH₃ | n-Bu | CH₃ |
| 475. | CH₂OCH₃ | CH₃ | CH₃ | n-Bu | CH₃ |
| 476. | OCH₃ | CH₃ | CH₃ | n-Bu | CH₃ |
| 477. | Ph | CH₃ | CH₃ | n-Bu | CH₃ |
| 478. | -CH₂Ph | CH₃ | CH₃ | n-Bu | CH₃ |
| 479. | CF₃ | n-Bu | CH₃ | CH₃ | CH₃ |
| 480. | CF₂CF₃ | n-Bu | CH₃ | CH₃ | CH₃ |
| 481. | CH₂OCH₃ | n-Bu | CH₃ | CH₃ | CH₃ |
| 482. | OCH₃ | n-Bu | CH₃ | CH₃ | CH₃ |
| 483. | Ph | n-Bu | CH₃ | CH₃ | CH₃ |
| 484. | -CH₂Ph | n-Bu | CH₃ | CH₃ | CH₃ |
| 485. | CH₃ | CH₃ | CF₃ | n-Bu | CH₃ |
| 486. | CH₃ | CH₃ | CF₂CF₃ | n-Bu | CH₃ |
| 487. | CH₃ | CH₃ | CH₂OCH₃ | n-Bu | CH₃ |
| 488. | CH₃ | CH₃ | OCH₃ | n-Bu | CH₃ |
| 489. | CH₃ | CH₃ | Ph | n-Bu | CH₃ |
| 490. | CH₃ | CH₃ | -CH₂Ph | n-Bu | CH₃ |
| 491. | CF₃ | n-Bu | H | CH₃ | CH₃ |
| 492. | CF₂CF₃ | n-Bu | H | CH₃ | CH₃ |
| 493. | CH₂OCH₃ | n-Bu | H | CH₃ | CH₃ |
| 494. | OCH₃ | n-Bu | H | CH₃ | CH₃ |
| 495. | Ph | n-Bu | H | CH₃ | CH₃ |
| 496. | -CH₂Ph | n-Bu | H | CH₃ | CH₃ |
| 497. | H | CH₃ | CF₃ | n-Bu | CH₃ |
| 498. | H | CH₃ | CF₂CF₃ | n-Bu | CH₃ |
| 499. | H | CH₃ | CH₂OCH₃ | n-Bu | CH₃ |
| 500. | H | CH₃ | OCH₃ | n-Bu | CH₃ |
| 501. | H | CH₃ | Ph | n-Bu | CH₃ |
| 502. | H | CH₃ | -CH₂Ph | n-Bu | CH₃ |
| 503. | n-Bu | H | CH₃ | CF₃ | CH₃ |
| 504. | n-Bu | H | CH₃ | CF₂CF₃ | CH₃ |
| 505. | n-Bu | H | CH₃ | CH₂OCH₃ | CH₃ |
| 506. | n-Bu | H | CH₃ | OCH₃ | CH₃ |
| 507. | n-Bu | H | CH₃ | Ph | CH₃ |
| 508. | n-Bu | H | CH₃ | -CH₂Ph | CH₃ |
| 509. | CF₃ | CH₃ | n-Bu | H | CH₃ |
| 510. | CF₂CF₃ | CH₃ | n-Bu | H | CH₃ |
| 511. | CH₂OCH₃ | CH₃ | n-Bu | H | CH₃ |
| 512. | OCH₃ | CH₃ | n-Bu | H | CH₃ |
| 513. | Ph | CH₃ | n-Bu | H | CH₃ |
| 514. | -CH₂Ph | CH₃ | n-Bu | H | CH₃ |
| 515. | CF₃ | n-Bu | CH₃ | H | CH₃ |
| 516. | CF₂CF₃ | n-Bu | CH₃ | H | CH₃ |
| 517. | CH₂OCH₃ | n-Bu | CH₃ | H | CH₃ |
| 518. | OCH₃ | n-Bu | CH₃ | H | CH₃ |
| 519. | Ph | n-Bu | CH₃ | H | CH₃ |
| 520. | -CH₂Ph | n-Bu | CH₃ | H | CH₃ |
| 521. | CH₃ | H | CF₃ | n-Bu | CH₃ |
| 522. | CH₃ | H | CF₂CF₃ | n-Bu | CH₃ |
| 523. | CH₃ | H | CH₂OCH₃ | n-Bu | CH₃ |
| 524. | CH₃ | H | OCH₃ | n-Bu | CH₃ |
| 525. | CH₃ | H | Ph | n-Bu | CH₃ |
| 526. | CH₃ | H | -CH₂Ph | n-Bu | CH₃ |
| 527. | n-Bu | CH₃ | H | CF₃ | CH₃ |
| 528. | n-Bu | CH₃ | H | CF₂CF₃ | CH₃ |
| 529. | n-Bu | CH₃ | H | CH₂OCH₃ | CH₃ |
| 530. | n-Bu | CH₃ | H | OCH₃ | CH₃ |
| 531. | n-Bu | CH₃ | H | Ph | CH₃ |
| 532. | n-Bu | CH₃ | H | -CH₂Ph | CH₃ |
| 533. | CF₃ | H | CH₃ | n-Bu | CH₃ |
| 534. | CF₂CF₃ | H | CH₃ | n-Bu | CH₃ |
| 535. | CH₂OCH₃ | H | CH₃ | n-Bu | CH₃ |
| 536. | OCH₃ | H | CH₃ | n-Bu | CH₃ |
| 537. | Ph | H | CH₃ | n-Bu | CH₃ |
| 538. | -CH₂Ph | H | CH₃ | n-Bu | CH₃ |
| 539. | H | -CH₂- | | H | CH₃ |
| 540. | H | -(CH₂)₄- | | H | CH₃ |

For the following example compounds physico-chemical data have been obtained and are displayed in order to illustrate the working of the present invention, including the outlined methods of synthesis. The number of given data may not be interpreted as a limitation of the invention. Analysis of compounds 6.610 to 6.684 : Reversed-phase was performed on a Waters Alliance 2790 LC equiped with a Waters996 UV detector using a YMC CombiScreen ODS-AQ cartridge (30x4.6 mm, S-5 □m, 12 um) Mobile phase: A: H₂O/CH₃CN 10/TFA, B: CH₃CN/TFA 0.1, C : MeOH.Gradient : 89% A 11% B, 0-3.5 min; 90% B 10% C 0.5 min.

**Table C**

| Comp. No. from | | Melting point [°C] or |
|---|---|---|
| Table B | Table A | ¹H-NMR [δ in ppm] |
| 1.001 | 028 | 122-131 |
| 1.002 | 028 | 199-201 |
| 1.003 | 028 | (DMSO); 0.60(t,3H), 1.19(s,3H), 1.67(q,2H), 2.02(s,3H), 6.93(dd,1H), 7.26(t,1H), 7.47(d,1H), 7.76(dd,1H), 7.83(dd,1H), 7.93(dd,1H), 8.48(d,1H), 8.55(d,1H), 8.63(d,1H), 10.00(s,NH); |
| 1.004 | 028 | 187-192 |
| 1.005 | 028 | (CDCl₃); 1.80(s,3H), 2.14(s,3H), 7.00(dd,1H), 7.22-7.29(m,7H), 7.39(dd,1H), 7.72(s,1H), 7.84(s,1H), 8.52(d,1H), 8.70(dd,1H), 8.77(s,NH); |
| 1.006 | 028 | 167-168 |
| 1.007 | 028 | 90-92 |
| 1.008 | 028 | 95-99 |
| 1.009 | 028 | (DMSO); 1.41(s,3H), 2.18(s,3H), 3.10(s,2H), 7.04(d,1H), 7.14(s,5H), 7.38(t,1H), 7.50(d,1H), 7.85(d,1H), 7.92(d,1H), 7.98(s,1H), 8.42(s,1H), 8.55(d,1H), 8.71(d,1H), 10.09(s,NH); |
| 1.010 | 028 | 165-168 |
| 1.011 | 028 | 215-219 |
| 1.012 | 028 | 210 |
| 1.050 | 028 | 202-205 |
| 1.051 | 028 | 164-167 |
| 1.052 | 028 | 167-170 |
| 1.053 | 028 | 189-192 |
| 2.002 | 028 | 181-185 |
| 2.003 | 028 | 204-208 |
| 2.004 | 028 | 210 |
| 2.005 | 028 | 190-192 |
| 2.006 | 028 | 199-203 |
| 2.007 | 028 | 180-182 |
| 2.008 | 048 | 127-135 |
| 2.009 | 028 | 87-83 |
| 2.010 | 028 | 195-197 |
| 2.011 | 028 | 187-189 |
| 2.012 | 0.28 | 218-220 |
| 3.001 | 028 | 163-166 |
| 3.002 | 028 | 189-191 |
| 3.003 | 028 | 158 |
| 3.011 | 028 | (DMSO); 3.32(s,3H), 4.35(s,2H), 5.66(s,1H), 7.03(dd,1H), 7.35(t,1H), 7.62(m, 1H), 7.77(m, 1H), 8.00(m, 1H), 8.20(m,1H), 8.48(m,1H), 8.62(d,1H), 8.74(d,1H), 10.12(s,NH), 12.25(s, 1H) |
| 3.012 | 028 | 158-159 |
| 3.013 | 028 | 167 |
| 3.014 | 028 | 141-150 |
| 3.015 | 028 | (DMSO); 1.74(s,3H), 2.15(s,3H), 7.01(dd,1H), 7.37(t,1H), 7.46(s,1H), 7.82(s,1H), 7.93(d,2H),8.55(d,1H), 8.63(d,1H), 9.21(s,1H), 10.07(s,NH), 11.5/12.0(s,1H); |
| 3.016 | 028 | (DMSO); 1.85(s,3H), 7.02(dd,1H), 7.35-7.79(m,7H), 8.61(d,1H), 8.74(d,1H), 10.12(s,NH), 11.7/11.9(s,1H); |
| 3.017 | 028 | 185-188 |
| 3.018 | 028 | 171-174 |
| 3.019 | 028 | 149-150 |
| 3.020 | 028 | 155-157 |
| 3.027 | 028 | 178-180 |
| 3.028 | 028 | 181-184 |
| 3.029 | 028 | 199-201 |
| 3.030 | 028 | 120-125 |
| 3.031 | 028 | 169-170 |
| 3.032 | 028 | 184 |
| 3.033 | 028 | 171-175 |
| 3.034 | 028 | 163-167 |
| 3.035 | 028 | 152-161 |
| 3.036 | 028 | 115-119 |
| 3.037 | 028 | 182-185 |
| 3.038 | 028 | 160-163 |
| 3.039 | 028 | 210 |
| 3.040 | 028 | 184 |
| 3.041 | 028 | 210 |
| 5.001 | 028 | 143-144 |
| 5.002 | 028 | 151-153 |
| 5.003 | 028 | 166-168 |
| 5.004 | 028 | 200-202 |
| 1.001 | 048 | (DMSO); 1.31(s,6H), 2.14(s,3H), 3.42(s,3H), 5.49(s,2H), 7.37-7.60(m,5H), 7.88(dd,1H), 8.54(s,1H), 8.61(d,1H), 8.64(d,1H); |
| 6.002 | 028 | 238-240 |
| 6.003 | 028 | 120-125 |
| 6.012 | 028 | 229-231 |
| 6.015 | 028 | 173-175 |
| 6.020 | 028 | 184-186 |
| 6.152 | 028 | 213-215 |
| 6.153 | 028 | 118-127 |
| 6.177 | 028 | 184-186 |
| 6.179 | 028 | 187-189 |
| 6.605 | 028 | 196-198 |
| 6.606 | 028 | 79-84 |
| 6.607 | 028 | 153-156 |
| 6.608 | 028 | 110-120 |
| 6.609 | 028 | 213-216 |
| 6.610 | 028 | RT 3.3 |
| | | MS 614.2(Area MS 100%, AreaUV 100%) |
| 6.611 | 028 | RT 3.23 |
| | | MS 564.2 (Area MS 100 %, AreaUV 100%) |
| 6.612 | 028 | RT 3.9 |
| | | MS 622.3(Area MS 100%, AreaUV 100%) |
| 6.613 | 028 | RT 2.37 |
| | | MS 550(Area MS 100%, AreaUV 100%) |
| 6.614 | 028 | RT 2.37 |
| | | MS 550(Area MS 100%, AreaUV 100%) |
| 6.615 | 048 | RT 2.15 |
| | | MS 478.1(Area MS 100%, AreaUV 100%) |
| 6.616 | 028 | RT 2.4 |
| | | MS 480.1 (Area MS 100%, AreaUV 84%) |
| 6.617 | 028 | RT 2.37 |
| | | MS 480.1 (Area MS 100%, AreaUV 100%) |
| 6.618 | 028 | RT 2.1 |
| | | MS 480.1(Area MS 100 %, AreaUV 88%) |
| 6.619 | 028 | RT 2.32 |
| | | MS 480.1 (Area MS 100%, AreaUV 100%) |
| 6.620 | 028 | RT 2.1 |
| | | MS 555.1 (Area MS 100%, AreaUV 90%) |
| 6.621 | 028 | RT 2.1 |
| | | MS 555.1 (Area MS 100%, AreaUV 90%) |
| 6.622 | 028 | RT2.1 |
| | | MS 468.1(Area MS 100%, AreaUV 85%) |
| 6.623 | 028 | RT 1.54 |
| | | MS 572.1(Area MS 100%, AreaUV 89%) |
| 6.624 | 028 | RT 2.5 |
| | | MS 482. 1 (Area MS 100%, AreaUV 100%) |
| 6.625 | 028 | RT 2.24 |
| | | MS 466.1 (Area MS 100%, AreaUV 100%) |
| 6.626 | 028 | RT 1.95 |
| | | MS 466.1(Area MS 100%, AreaUV 100%) |
| 6.627 | 028 | RT 1.85 |
| | | MS 464.1 (Area MS 100%, AreaUV 100%) |
| 6.628 | 028 | RT 2.1 |
| | | MS 492.1(Area MS 100%, AreaUV 92%) |
| 6.629 | 028 | RT 2 |
| | | MS 478.1(Area MS 100%, AreaUV 100%) |
| 6.630 | 028 | RT 2 |
| | | MS 478.1 (Area MS 100%, AreaUV 100%) |
| 6.631 | 028 | RT 2.5 |
| | | MS 494.1(Area MS 100%, AreaUV 93%) |
| 6.632 | 028 | RT 2.1 |
| | | MS 480.1(Area MS 100%, AreaUV 74%) |
| 6.633 | 028 | RT 2.1 |
| | | MS 480.1 (Area MS 100%, AreaUV 74%) |
| 6.634 | 028 | RT 2.24 |
| | | MS 494.1(Area MS 100%, AreaUV 100%) |
| 6.635 | 028 | RT 2.24 |
| | | MS 494.1(Area MS 100%, AreaUV 100%) |
| 6.636 | 028 | RT 3.1;3.2 |
| | | MS 580.1(Area MS 70%, AreaUV 62%) |
| 6.637 | 028 | RT 4 |
| | | MS 598(Area MS 77%, AreaUV 100%) |
| 6.638 | 028 | RT 3.47 |
| | | MS 610.1(Area MS 58%, AreaUV 100%) |
| 6.639 | 028 | RT 3.5 |
| | | MS 564.1(Area MS 67%, AreaUV 100%) |
| 6.640 | 028 | RT 3.4 |
| | | MS 598.1(Area MS 79%, AreaUV 80%) |
| 6.641 | 028 | RT 3.22 |
| | | MS 560.1(Area MS 69%, AreaUV 100%) |
| 6.642 | 028 | RT 3.07 |
| | | MS 660.1(Area MS 100%, AreaUV 100%) |
| 6.643 | 028 | RT 3.1 |
| | | MS 514(Area MS 59%, AreaUV 100%) |
| 6.644 | 028 | RT 2.8 |
| | | MS 522.1 (Area MS 72%, AreaUV 100%) |
| 6.645 | 028 | RT 3.07 |
| | | MS 548(Area MS 80%, AreaUV 100%) |
| 6.646 | 028 | RT 4.4 |
| | | MS 638.2(Area MS 90%, AreaUV 62%) |
| 6.647 | 028 | RT 3.5 |
| | | MS 580.1(Area MS 57%, AreaUV 100%) |
| 6.648 | 028 | RT 2.81;2.8 |
| | | MS 500.1(Area MS 63%, AreaUV 100%) |
| 6.649 | 028 | RT 4 |
| | | MS 538.2(Area MS 84%, AreaUV 100%) |
| 6.650 | 028 | RT 3.11 |
| | | MS 546.1(Area MS 53%, AreaUV 80%) |
| 6.651 | 028 | RT 2.7;2.8 |
| | | MS 540.1(Area MS 56%, AreaUV 74%) |
| 6.652 | 028 | RT 4.5 |
| | | MS 592.2(Area MS 62%, AreaUV 100%) |
| 6.653 | 028 | RT 3.5 |
| | | MS 554.2(Area MS 96%, AreaUV 100%) |
| 6.654 | 028 | RT 4.3 |
| | | MS 562.2(Area MS 71 %, AreaUV 100%) |
| 6.655 | 028 | RT 3.47 |
| | | MS 494.1(Area MS 100%, AreaUV 100%) |
| 6.656 | 028 | RT 3 |
| | | MS 514.1(Area MS 86%, AreaUV 100%) |
| 6.657 | 028 | RT 2.2 |
| | | MS 551.1(AreaMS 74%, AreaUV 100%) |
| 6.658 | 028 | RT 3.36 |
| | | MS 508.1(Area MS 100%, AreaUV 100%) |
| 6.659 | 028 | RT 3.22 |
| | | MS 590.1(Area MS 84%, AreaUV 100%) |
| 6.660 | 028 | RT 3.3 |
| | | MS 564.1(Area MS 69%, AreaUV 74%) |
| 6.661 | 028 | RT 3.8 |
| | | MS 758(Area MS 42%, AreaUV 100%) |
| 6.662 | 028 | RT 3.4 |
| | | MS 566.1(Area MS 78%, AreaUV 100%) |
| 6.663 | 028 | RT 3.4 |
| | | MS 642(Area MS 80%, AreaUV 100%) , |
| 6.664 | 028 | RT 3 |
| | | MS 614.2(Area MS 82%, AreaUV 100%) |
| 6.665 | 028 | RT 2.4 |
| | | MS 512.1(Area MS 92%, AreaUV 82%) |
| 6.666 | 028 | RT 2.0;2.3 |
| | | MS 545. 1 (Area MS 82%, AreaUV 100%) |
| 6.667 | 028 | RT 3.2 |
| | | MS 494.1 (Area MS 74%, AreaUV 100%) |
| 6.668 | 028 | RT 3.4 |
| | | MS 596. 1 (Area MS 75%, AreaUV 100%) |
| 6.669 | 028 | RT 4.4 |
| | | MS 658.1(Area MS 66%, AreaUV 100%) |
| 6.670 | 028 | RT 3.3 |
| | | MS 562. 1 (Area MS 81 %, AreaUV 100%) |
| 6.671 | 028 | RT 3.1 |
| | | MS 585(Area MS 70%, AreaUV 100%) |
| 6.672 | 028 | RT 2.04;2.1 |
| | | MS 531.1(Area MS 84%, AreaUV 100%) |
| 6.673 | 028 | RT 3.9 |
| | | MS 586.2(Area MS 88%, AreaUV 100%) |
| 6.674 | 028 | RT 3 |
| | | MS 522(Area MS 91 %, AreaUV 100%) |
| 6.675 | 028 | RT 4.3 |
| | | MS 578.2(Area MS 88%, AreaUV 100%) |
| 6.676 | 028 | RT 2.78;2.8 |
| | | MS 512.1(Area MS 100%, AreaUV 100%) |
| 6.677 | 028 | RT 2.7 |
| | | MS 525.1(Area MS 95%, AreaUV 100%) |
| 6.678 | 028 | RT 3.3 |
| | | MS 584.1 (Area MS 91%, AreaUV 100%) |
| 6.679 | 028 | RT 1.8;2.1 |
| | | MS 517.1(Area MS 72%, AreaUV 100%) |
| 6.680 | 028 | RT 3.7 |
| | | MS 512.1 (Area MS 96%, AreaUV 100%) |
| 6.681 | 028 | RT 3 |
| | | MS 516.1(Area MS 54%, AreaUV 38%) |
| 6.682 | 028 | RT 3.5 |
| | | MS 708(Area MS 71%, AreaUV 100%) |
| 6.683 | 028 | RT 3.7 |
| | | MS 720.1(Area MS 81%, AreaUV 100%) |
| 6.684 | 028 | RT 3.1 |
| | | MS 607.1(Area MS 88%, AreaUV 100%) |
| 6.685 | 028 | 80-100 |
| 6.686 | 028 | 183-186 |
| 6.687 | 028 | 212-215 |
| 6.688 | 028 | 176-178 |
| 6.689 | 028 | 183-185 |
| 6.690 | 028 | 110-115 |
| 6.691 | 028 | 119-123 |
| 6.692 | 028 | 117-120 |
| 6.693 | 028 | 83-89 |
| 6.694 | 028 | 90-100 |
| 6.695 | 028 | 73-76 |
| 6.696 | 028 | 110-120 |
| 6.697 | 028 | 145-160 |
| 6.698 | 028 | 84-90 |
| 6.699 | 028 | 239-242 |
| 6.700 | 028 | 90-105 |
| 6.701 | 028 | 232-235 |
| 6.702 | 028 | 178-182 |
| 6.703 | 028 | 142-148 |
| 6.704 | 028 | 222-225 |
| 6.705 | 028 | 75-85 |
| 6.706 | 028 | 142-144 |
| 6.707 | 028 | 235-240 |
| 6.708 | 028 | 141-144 |
| 6.709 | 028 | 80-82 |
| 6.710 | 028 | 82-84 |
| 6.711 | 028 | 174-176 |
| 6.712 | 028 | 201-203 |
| 6.713 | 028 | 120-125 |
| 6.714 | 028 | 198-200 |
| | | RT 2.06 |
| | | MS 464.1(Area MS 100%, AreaUV 100%) |
| 6.715 | 028 | 85-90 |
| 6.716 | 028 | 87-97 |
| 6.717 | 028 | 251-253 |
| 6.718 | 028 | RT 2.41 |
| | | MS 512.1 (Area MS 100%, AreaUV 0%) |
| 6.719 | 028 | RT 2.63 |
| | | MS 510.1(Area MS 88%, AreaUV 100%) |
| 6.720 | 028 | RT 2.4 |
| | | MS 482.1 (Area MS 90%, AreaUV 82%) |
| 6.721 | 028 | 92-96 |
| 6.722 | 028 | 90-100 |
| 6.723 | 028 | 110-115 |
| 6.724 | 028 | 188-190 |
| 6.725 | 028 | 70-80 |
| 6.726 | 028 | 182-184 |
| 7.001 | 028 | 110-130 |
| 7.270 | 028 | 189-192 |
| 7.271 | 028 | 207-209 |
| 7.277 | 028 | 89-93 |
| 7.303 | 028 | 177-179 |
| 7.808 | 028 | 165-167 |
| 7.830 | 028 | 90-95 |
| 8.270 | 028 | 201-204 |
| 8.271 | 028 | 193-195 |
| 8.277 | 028 | 105-115 |
| 8.279 | 028 | 95-100 |
| 8.285 | 028 | 98-105 |
| 8.303 | 028 | 105-110 |
| 8.539 | 028 | 80-85 |
| 8.540 | 028 | 95-100 |

In the following, examples of test systems in plant protection are provided which can demonstrate the efficiency of the compounds of the formula I (designated as "active ingredient" or "test compounds"):

### Biological Examples

### Example B-1: Effect against Puccinia graminis on wheat (brownrust on wheat)

### a) Residual protective activity

1 week old wheat plants cv. Arina are treated with the formulated test-compound (0.02 % active substance) in a spray chamber. Two days after application wheat plants are inoculated by spraying a spore suspension (1 x 10⁵ ureidospores/ml) on the test plants. After an incubation period of 1 day at +20°C and 95% relative atmospheric humidity (r. h.) plants are kept for 9 days at +20°C and 60% r.h. in a greenhouse. The disease incidence is assessed 10 days after inoculation.

At the indicated concentration compounds 1.01/028; 2.02/028; 1.03/028; 1.07/028; 2.03/028; 2.05/028; 2.06/028 exhibited over 70% control of the fungal infection in this test.

### Example B-2: Effect against Phytophthora infestans on tomatoes (late blight on potato)

### a) Residual protective activity

3 week old tomato plants cv. Roter Gnom are treated with the formulated test compound (0.02 % active substance) in a spray chamber. Two day after application the plants are inoculated by spraying a sporangia suspension (2 x 10⁴ sporangia/ml) on the test plants. After an incubation period of 4 days at +18°C and 95% r. h. in a growth chamber the disease incidence is assessed.

At the indicated concentration compounds 1.01/028; 1.03/028; 1.04/028; 1.07/028 exhibited over 70% control of the fungal infection in this test.

### Example B-3: Effect against Phytophthora infestans.l potato (late blight on potato)

5 week old potato plants cv. Bintje are treated with the formulated test compound (0.02 % active substance) in a spray chamber. Two days after application the plants are inoculated by spraying a sporangia suspension (1.4 x 10⁵ sporangia/ml) on the test plants. After an incubation period of 4 days at +18°C and 95% r. h. in a growth chamber the disease incidence is assessed.

### Example B-4: Effect against Plasmopara viticola on grapevine (grape downy mildew)

5 week old grape seedlings cv. Gutedel are treated with the formulated test compound (0.02 % active substance) in a spray chamber. One day after application grape plants are inoculated by spraying a sporangia suspension (4 x 10⁴ sporangia/ml) on the lower leaf side of the test plants. After an incubation period of 6 days at +22°C and 95% r. h. in a greenhouse the disease incidence is assessed.

At the indicated concentration compounds 1.01/028;3.01/028; 1.04/028 exhibited over 70% control of the fungal infection in this test.

### Example B-5: Residual protective activity against Venturia inaequalis on apples (scab on apple)

4 week old apple seedlings cv. McIntosh are treated with the formulated test compound (0.02 % active substance) in a spray chamber. One day after application apple plants are inoculated by spraying a spore suspension (4 x 10⁵ conidia/ml) on the test plants. After an incubation period of 4 days at +20°C and 95% r. h. the plants are transferred to standard greenhouse conditions at 20 and 60% r.h. where they stayed for 2 days. After another 4 day incubation period at +20°C and 95% r. h. the disease incidence is assessed. At the indicated concentration compounds 2.03/028; 1.001/028 exhibited over 70% control of the fungal infection in this test.

### Example B-6: Effect against Erysiphe graminis on barley (powdery mildew on barley)

### a) Residual protective activity

Barley plants, cv. Regina of approximately 8 cm height were treated with the formulated test compound (0.02% active substance) in a spray chamber and duste 2 days after inoculation with conidia of the fungus. The infected plants are placed in a greenhouse at +20°C. 6 days after infection, the fungal attack was evaluated.

At the indicated concentration compounds 1.01/028; 1.03%028; 1.04/028, 2.05/028; 2.09/028; 3.014/028; 3.030/028 exhibited over 70% control of the fungal infection in this test.

### Example B-7: Botrytis cinerea / grape (botrytis on grapes)

5 week old grape seedlings cv. Gutedel are treated with the formulated test compound (0.02% active substance) in a spray chamber. Two days after application grape plants are inoculated by spraying a spore suspension (1.5 x 10⁵ conidia/ml) on the test plants. After an incubation period of 3 days at +21°C and 95% r. h. in a greenhouse the disease incidence is assessed.

At the indicated concentration compounds 1.01/028; 1.03/028; 1.04/028, 1.05/028; 1.06/028,1.07/028;2.03/028; 2.05/028; 2.08/048; 2.09/028; 3.012/028;

3.013/028;3.014/028; 2.012/028 exhibited over 70% control of the fungal infection in this test.

### Example B-8: Effect against Botrytis cinerea / tomato (botrytis on tomatoes)

4 week old tomato plants cv. Roter Gnom are treated with the formulated test compound 0.02 % active substance) in a spray chamber. Two days after application tomato plants are inoculated by spraying a spore suspension (1 x 10⁵ conidia/ml) on the test plants. After an incubation period of 4 days at +20°C and 95% r. h. in a greenhouse the disease incidence is assessed.

At the indicated concentration compounds 1.01/028; 2.02/028; 3.01/028; 1.04/028; 1.06/028; 2.06/028; 2.05/028; 2.08/048; 4.02/028; 7.270/028 exhibited over 70% control of the fungal infection in this test.

### Example B-9: Effect against Pyricularia oryzae / rice (rice blast)

3 week old rice plants cv. Sasanishiki are treated with the formulated test compound (0.02 % active substance) in a spray chamber. Two days after application rice plants are inoculated by spraying a spore suspension (1 x 10⁵ conidia/ml) on the test plants. After an incubation period of 6 days at +25°C and 95% r. h. the disease incidence is assessed. At the indicated concentration compounds 1.02/028; 1.04/028; 2.03/028; 2.06/028; 2.07/028 exhibited over 70% control of the fungal infection in this test.

### Example B-10: Effect against Pyrenophora teres (Helminthosporium) / barley (net blotch on barley)

1 week old barley plants cv. Regina are treated with a formulated test compound (0.02 % active substance) in a spray chamber. Two days after application barley plants are inoculated by spraying a spore suspension (3 x 10⁴ conidia/ml) on the test plants. After an incubation period of 2 days at +20°C and 95% r.h. the disease incidence is assessed. At the indicated concentration compounds 1.01/028; 2.02/028; 3.01/028; 5.01/028; 1.03/028; 1.04/028, 1.01/048; 1.06/028,1.07/028:1.08/028; 2.03/028; 2.05/028; 2.07/028; 2.08/048; 2.09/028; 3.012/028; 3.013/028;3.014/028; 2.012/028; 2.011/028; 3.016/028; 3.017/0283.027/028; 3.028/028; 7.270/028 exhibited over 70% control of the fungal infection in this test.

### Example B-11: Effect against Fusarium culmorum/wheat (fusarium head blight on wheat)

A conidia suspension of *F. culmorum* (7 x 10⁵ conidia/ml) is mixed with the formulated test compound (0.002 % active substance).. The mixture is applied into a pouch which has been equipped before with a filter paper. After the application wheat seeds (cv. Orestis) are sown into the upper fault of the filter paper. The prepared pouches are then incubated for 11 days at approx. +10°C to +18°C and a relative humidity of 100% with a light period of 14 hours. The evaluation is made by assessing the degree of disease occurrence in the form of brown lesions on the roots.

### Example B-12: Effect against Septoria nodorum / wheat (septoria leaf spot on wheat)

1 week old wheat plants cv. Arina are treated with a formulated test compound (0.02 % active substance) in a spray chamber. One day after application wheat plants are inoculated by spraying a spore suspension (6 x 10⁵ conidia/ml) on the test plants. After an incubation period of 1 day at +22°C and 95% r.h. plants are kept for 7 days at +22°C and 60% r.h. in a greenhouse. The disease incidence is assessed 8 days after inoculation. At the indicated concentration compounds 1.01/028; 2.02/028; 3.01/028; 5.01/028; 1.03/028; 1.06/028,1.07/028; 2.03/028;2.04/028; 2.05/028; 2.06/028; 2.09/028; 3.012/028; 2.012/028; 3.028/028 exhibited over 70% control of the fungal infection in this test.

## Claims

1. A compound of formula I 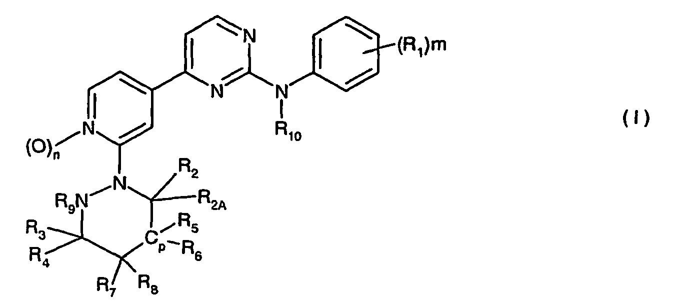 wherein
m is 0, 1, 2 or 3;
n and p are independently of each other 0 or 1;
R₁ is halogen, optionally substituted alkyl, optionally substituted alkoxy, optionally substituted alkenyloxy, optionally substituted alkynyloxy, optionally substituted thioalkyl optionally substituted aryl, COOR₁₁, CONR₁₂R₁₃, S(O)_{q}R₁₄, SO₂NR₁₅R₁₆ or NR₁₅ₐR₁₆ₐ; when there is more than on R₁ group, they may be the same or different;
q is 1 or 2;
R₂, R₂ₐ, R₃, R₄, R₅, R₆, R₇, R₈ are each independently hydrogen, optionally substituted alkyl, COR₁₇, COOR₁₈ or optionally substituted aryl, and in addition R₂ and R₃ may also independently be optionally substituted alkoxy, optionally substituted alkenyloxy, optionally substituted alkynyloxy, or optionally substituted alkylthio, COOR₁₉, CONR₂₀R₂₁, OH or SH;
R₆ and R₇ may also be independently halogen, optionally substituted alkoxy, optionally substituted alkenyloxy, optionally substituted alkynyloxy, optionally substituted alkenylamino, optionally substituted alkynylamino, optionally substituted alkylthio, optionally substituted cycloalkyl, optionally substituted heteroaryl, optionally substituted hetrocyclyl, optionally substituted cycloalkyloxy, OH, SH, N₃, NR₂₂R₂₃ or N(R₂₄)COR₂₅;
or the ring members CR₃R₄ or CR₂R_{2A} are independently of each other a carbonyl group (C=O) or a thonyl group (C=S);
or one or two of the adjacent pairs of groups R₉ and R₄, R₄ and R₈, R₅ and R₈, or, if p is zero, R_{2A} and R₈ may form a bond, provided that if there are 2 double bonds in the ring the double bonds are not adjacent each other;
or the pair of groups R₇ and R₈ or the pair of groups R₆ and R₇ together with the atom to which they are attached form a C₃-C₇ saturated ring;
R₉ is hydrogen, optionally substituted alkyl, optionally substituted alkenyl or optionally substituted alkynyl;
R₁₀ is hydrogen, C₁-C₄-alkyl, C₃-C₄-alkenyl, C₃-C₄-alkynyl, -CH₂OR₂₆, CH₂SR₂₇, - C(O)R₂₈, -C(O)OR₂₉. SO₂R₃₀, SOR₃₁ or SR₃₂;
R₂₆, R₂₇, R₂₈, R₂₉, R₃₀, R₃₁, R₃₂ are independently C₁-C₈-alkyl C₁-C₈-alkoxyalkyl, C₁-C₈ haloalkyl or phenylC₁-C₂-alkyl wherein the phenyl may be substituted by up to three groups selected from halo or C₁-C₄-alkyl,
R₁₁, R₁₂, R₁₃, R₁₄, R₁₅, R₁₆ R₁₅ₐ, R₁₆ₐ, R₁₇, R₁₈, R₁₉, R₂₀, R₂₁, R₂₂, R₂₃, R₂₄, and R₂₅ are independently H or optionally substituted alkyl; or a salt thereof.

2. A compound according to claim 1, wherein the moiety 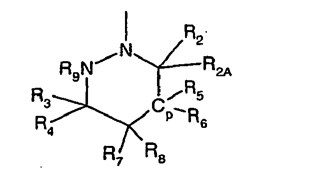 is a 5- and 6-membered ring selected from 2,4-dihydro-pyrazol-3-ones, 2,4-dihydropyrazole-3-thione, 1H-pyrazoles, 2H-pyridazin-3-ones, 4,5-dihydro-2H-pyridazin-3-ones, 1,2-dihydro-pyrazol-3-ones, 1,2-dihydro-pyrazole-3-thione, pyrazolidin-3-one, pyrazolidine-3-thione, 2H-pyridazin-3-thione and 4,5-dihydro-2H-pyridazin-3-thione.

3. A compound according to claims 1 or 2, wherein R₁ is halogen, C₁₋₃ haloalkoxy, CH(OH)R, COR, SO₂NRR', CH(NR'R")R, COORa or CONRbRc where Ra, Rb, Rc, R, R', R" are independently H or lower alkyl.

4. A compound according to any one of claims 1 to 3, wherein R₂, R_{2A}, R₃, R₄, R₅, R₆, R₇, R₈ and R₉ independently of each other are hydrogen or methyl.

5. A compound according to any one of claims 1 to 4, wherein n is zero.

6. A compound according to any one of claims 1 to 5, wherein m is 1 and the R₁ group is at the 3- or 4- position of the phenyl ring.

7. A compound according to any one of claims 1 to 6, wherein R₇ is hydrogen, methyl, ethyl, allyl, propargyl, methoxymethyl, thiomethoxymethyl or ethoxymethyl, more preferably hydrogen or methoxymethyl.

8. A compound according to any one of claims 1 to 7 where R₁₀ is hydrogen, methyl, ethyl, allyl, propargyl, methoxymethyl, thiomethoxymethyl or ethoxymethyl, preferably hydrogen or methoxymethyl.

9. A compound according to any one of claims 1 to 8, wherein the compound is selected from (3-Chloro-phenyl)-14-[2-(3,4,5-tirimethyl-pyrazol-1-yl)-pyiidin-4-yl]-pyrimidin-2-yl}-amine;
(3-Chloro-phenyl)-{4-[2-(5-methoxy-3-methoxymethyl-pyrazol-1-yl)-pyridin-4-yl]-pyrimidin-2-yl }-amine;
(3-Chloro-phenyl)-{4-[2-(5-methoxy-3-methoxymethyl-4-methyl-pyrazol-1-yl)-pyridin-4-yl]-pyrimidin-2-yl}-amine;
(3-Chloro-phenyl)- {4-[2-(5-methoxy-4-methyl-pyrazol-1-yl)-pyridin-4-yl]-pyrimidin-2-yl }-amine;
(3-Chloro-phenyl)-{ 4-[2-(5-ethoxy-3,4-dimethyl-pyrazol-1-yl)-pyridin-4-yl]-pyrimidin-2-yl }-amine;
2-{4-[2-(3-Chloro-phenylamino)-pyrimidin-4-yl]-pyridin-2-yl}-5-methoxymethyl-1,4-dimethyl-1,2-dihydro-pyrazol-3-one;
2-(4-{2-[(3-Chloro-phenyl)-methoxymethyl-amino]-pyrimidin-4-yl}-pyridin-2-yl)-1,5-dimethyl-1,2-dihydro-pyrazol-3-one;
2-{4-[2-(3-Chloro-phenylamino)-pyrimidin-4-yl]-pyridin-2-yl}-1-ethyl-4,5-dimethyl-1,2-dihydro-pyrazol-3-one;
2-{4-[2-(3-Chloro-phenylamino)-pyrimidin-4-yl]-pyridin-2-yl}-1,4-dimethyl-1,2-dihydro-pyrazol-3-one;
2-{4-[2-(3-Chloro-phenylamino)-pyrimidin-4-yl]-pyridin-2-yl}-1,5-dimethyl-1,2-dihydro-pyrazol-3-one;
2-{4-[2-(3-Chloro-phenylamino)-pyrimidin-4-yl]-pyridin-2-yl}-5-methoxymethyl-4,4-dimethyl-2,4-dihydro-pyrazol-3-one;
2-{4-[2-(3-Chloro-phenylamino)-pyrimidin-4-yl]-pyridin-2-yl}-4,4-dimethyl-2,4-dihydro-pyrazol-3-one;
2-{4-[2-(3-Chloro-phenylamino)-pyrimidin-4-yl]-pyridin-2-yl}-4,4,5-trimethyl-2,4-dihydro-pyrazole-3-thione;
5-{4-[2-(3-Chloro-phenylamino)-pyrimidin-4-yl]-pyridin-2-yl}-7-methyl-5,6-diazaspiro[2.4]hept-6-en-4-one;
2-{4-[2-(3-Chloro-phenylamino)-pyrimidin-4-yl]-pyridin-2-yl}-4-ethyl-4,5-dimethyl-2,4-dihydro-pyrazol-3-one;
(3-Chloro-phenyl)-{4-[2-(5-methoxy-3-methyl-pyrazol-1-yl)-pyridin-4-yl]-pyrimidin-2-yl}-amine;
2-{4-[2-(3-Chloro-phenylamino)-pyrimidin-4-yl]-pyridin-2-yl}-1,4,5-trimethyl-1,2-dihydro-pyrazol-3-one;
2-{4-[2-(3-Chloro-phenylamino)-pyrimidin-4-yl]-pyridin-2-yl}-4,4,5-trimethyl-2,4-dihydro-pyrazol-3-one;
2-{4-[2-(3-Chloro-phenylamino)-pyrimidin-4-yl]-pyridin-2-yl}-1,5-dimethyl-1,2-dihydro-pyrazol-3-one;
4,5-Dichloro-2-{4-[2-(3-chloro-phenylamino)-pyrimidin-4-yl]-pyridin-2-yl}-2H-pyridazin-3-one;
2-{4-[2-(3-Chloro-phenylamino)-pyrimidin-4-yl]-pyridin-2-yl}-6-methyl-2H-pyridazin-3-one;
2-{4-[2-(3-Chloro-phenylamino)-pyrimidin-4-yl]-pyridin-2-yl}-6-methyl-4,5-dihydro-2H-pyridazin-3-one;
2-{4-[2-(3-Chloro-phenylamino)-pyrimidin-4-yl]-pyridin-2-yl}-6-Phenyl-4,5-dihydro-2H-pyridazin-3-one;
4-Chloro-2-{4-[2-(3-chloro-phenylamino)-pyrimidin-4-yl]-pyridin-2-yl}-5-ethoxy-2H-pyridazin-3-one;
4-Chloro-2-{4-[2-(3-chloro-phenylamino)-pyrimidin-4-yl]-pyridin-2-yl}-5-ethylsulfanyl-2H-pyridazin-3-one;
5-Azido-4-chloro-2-{4-[2-(3-chloro-phenylamino)-pyrimidin-4-yl]-pyridin-2-yl}-2H-pyridazin-3-one;
1-{4-[2-(3-Chloro-phenylamino)-pyrimidin-4-yl]-pyridin-2-yl}-2-methylpyrazolidin-3-one;
(3-Chloro-phenyl)-{4-[2-(5-methoxy-3,4-dimethyl-pyrazol-1-yl)-pyridin-4-yl]-pyrimidin-2-yl}-amine;
2-{4-[2-(3-Chloro-phenylamino)-pyrimidin-4-yl]-pyridin-2-yl}-5-methoxymethyl-1-methyl-1,2-dihydro-pyrazol-3-one;
2-{4-[2-(3-Chloro-phenylamino)-pyrimidin-4-yl]-pyridin-2-yl}-1,5-dimethyl-3-oxo-2,3-dihydro-1H-pyrazole-4-carbaldehyde;
5-Chloro-2-{4-[2-(3-chloro-phenylamino)-pyrimidin-4-yl]-pyridin-2-yl}-4-(oxetan-3-yloxy)-2H-pyridazin-3-one; and
4-Chloro-2-{4-[2-(3-chloro-phenylamino)-pyrimidin-4-yl]-pyridin-2-yl}-5-(tetrahydro-furan-2-ylmethoxy)-2H-pyridazin-3-one.

10. A composition for controlling and protecting against phytopathogenic microorganisms, comprising a compound of formula I according to claim 1 as active ingredient together with a suitable carrier.

11. The use of a compound of formulae according to claim 1 in protecting plants against infestation by phytopathogenic microorganisms.

12. A method of controlling and preventing an infestation of crop plants by phytopathogenic microorganisms, which comprises the application of a compound of formula I according to claim 1 as active ingredient to the plant, to parts of plants or to the locus thereof.

13. A method according to claim 12, wherein the phytopathogenic microorganisms are fungal organisms.

## Patentansprüche

1. Verbindung der Formel (I): 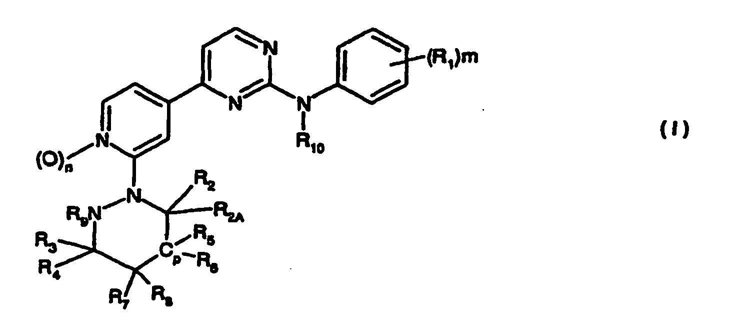 worin
m 0, 1, 2 oder 3 ist;
n und p unabhängig voneinander 0 oder 1 sind;
R₁ Halogen, gegebenenfalls substituiertes Alkyl, gegebenenfalls substituiertes Alkoxy, gegebenenfalls substituiertes Alkenyloxy, gegebenenfalls substituiertes Alkinyloxy, gegebenenfalls substituiertes Thioalkyl, gegebenenfalls substituiertes Aryl, COOR₁₁, CONR₁₂R₁₃, S(O)_{q}R₁₄, SO₂NR₁₅R₁₆ oder NR₁₅ₐR₁₆ₐ ist; wenn es mehr als eine R₁-Gruppe gibt, sie gleich oder verschieden sein können;
q 1 oder 2 ist;
R₂, R₂ₐ, R₃, R₄, R₅, R₆, R₇ und R₈ jeweils unabhängig Wasserstoff, gegebenenfalls substituiertes Alkyl, COR₁₇, COOR₁₈ oder gegebenenfalls substituiertes Aryl sind und zusätzlich R₂ und R₃ auch unabhängig gegebenenfalls substituiertes Alkoxy, gegebenenfalls substituiertes Alkenyloxy, gegebenenfalls substituiertes Alkinyloxy oder gegebenenfalls substituiertes Alkylthio, COOR₁₉, CONR₂₀R₂₁, OH oder SH sein können;
R₆ und R₇ auch unabhängig Halogen, gegebenenfalls substituiertes Alkoxy, gegebenenfalls substituiertes Alkenyloxy, gegebenenfalls substituiertes Alkinyloxy, gegebenenfalls substituiertes Alkenylamino, gegebenenfalls substituiertes Alkinylamino, gegebenenfalls substituiertes Alkylthio, gegebenenfalls substituiertes Cycloalkyl, gegebenenfalls substituiertes Heteroaryl, gegebenenfalls substituiertes Heterocyclyl, gegebenenfalls substituiertes Cycloalkyloxy, OH, SH, N₃, NR₂₂R₂₃ oder N(R₂₄)COR_{2S} sein können;
oder die Ringglieder CR₃R₄ oder CR₂R_{2A} unabhängig voneinander eine Carbonyl-Gruppe (C=O) oder Thionyl-Gruppe (C=S) sind;
oder eines oder zwei der benachbarten Paare von Gruppen R₉ und R₄, R₄ und R₈, R₅ und R₈ oder, falls p Null ist, R_{2A} und R₈ eine Bindung bilden können, mit der Maßgabe, daß dann, wenn es zwei Doppelbindungen im Ring gibt, die Doppelbindungen nicht zueinander benachbart sind;
oder das Paar der Gruppen R₇ und R₈ oder das Paar der Gruppen R₆ und R₇ zusammen mit dem Atom, an das sie gebunden sind, einen gesättigten C₃₋₇-Ring bildet;
R₉ Wasserstoff, gegebenenfalls substituiertes Alkyl, gegebenenfalls substituiertes Alkenyl oder gegebenenfalls substituiertes Alkinyl ist;
R₁₀ Wasserstoff, C₁₋₄-Alkyl, C₃₋₄-Alkenyl, C₃₋₄-Alkinyl, -CH₂OR₂₆, -CH₂SR₂₇, -C(O)R₂₈, -C(O)OR₂₉, SO₂R₃₀, SOR₃₁ oder SR₃₂ ist;
R₂₆, R₂₇, R₂₈, R₂₉, R₃₀, R₃₁ oder R₃₂ unabhängig C₁₋₈-Alkyl, C₁₋₈-Alkoxyalkyl, C₁₋₈-Halogenalkyl oder Phenyl-C₁₋₂-alkyl sind, worin das Phenyl mit bis zu drei Gruppen substituiert sein kann, die aus Halogen oder C₁₋₄-Alkyl ausgewählt sind,
R₁₁, R₁₂, R₁₃, R₁₄, R₁₅, R₁₆, R₁₅ₐ, R₁₆ₐ, R₁₇, R₁₈, R₁₉, R₂₀, R₂₁, R₂₂, R₂₃, R₂₄ und R₂₅ unabhängig H oder gegebenenfalls substituiertes Alkyl sind; oder ein Salz davon.

2. Verbindung gemäß Anspruch 1, worin die Einheit: 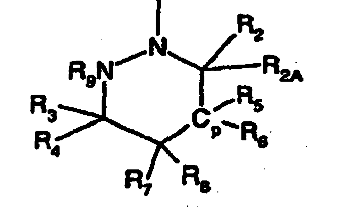 ein 5- und 6-gliedriger Ring ist, ausgewählt aus 2,4-Dihydro-pyrazol-3-onen, 2,4-Dihydro-pyrazol-3-thion, 1H-Pyrazolen, 2H-Pyridazin-3-onen, 4,5-Dihydro-2H-pyridazin-3-onen, 1,2-Dihydro-pyrazol-3-onen, 1,2-Dihydro-pyrazol-3-thion, Pyrazolidin-3-on, Pyrazolidin-3-thion, 2H-Pyridazin-3-thion und 4,5-Dihydro-2H-pyridazin-3-thion.

3. Verbindung gemäß Ansprüchen 1 oder 2, worin R₁ Halogen, C₁₋₃-Halogenalkoxy, CH(OH)R, COR, SO₂NRR', CH(NR'R")R, COORa oder CONRbRc ist, worin Ra, Rb, Rc, R, R', R" unabhängig H oder Niederalkyl sind.

4. Verbindung gemäß einem der Ansprüche 1 bis 3, worin R₂, R_{2A}, R₃, R₄, R₅, R₆, R₇, R₈ und R₉ unabhängig voneinander Wasserstoff oder Methyl sind.

5. Verbindung gemäß einem der Ansprüche 1 bis 4, worin n Null ist.

6. Verbindung gemäß einem der Ansprüche 1 bis 5, worin m 1 ist und die R₁-Gruppe in der 3- oder 4-Stellung des Phenylrings ist.

7. Verbindung gemäß einem, der Ansprüche 1 bis 6, worin R₇ Wasserstoff, Methyl, Ethyl, Allyl, Propargyl, Methoxymethyl, Thiomethoxymethyl oder Ethoxymethyl ist, besonders bevorzugt Wasserstoff oder Methoxymethyl.

8. Verbindung gemäß einem der Ansprüche 1 bis 7, worin R₁₀ Wasserstoff, Methyl, Ethyl, Allyl, Propargyl, Methoxymethyl, Thiomethoxymethyl oder Ethoxymethyl ist, bevorzugt Wasserstoff oder Methoxymethyl.

9. Verbindung gemäß einem der Ansprüche 1 bis 8, worin die Verbindung ausgewählt ist aus:
(3-Chlor-phenyl)-{4-[2-(3,4,5-trimethyl-pyrazol-1-yl)-pyridin-4-yl]-pyrimidin-2-yl}-amin;
(3-Chlor-phenyl)-{4-[2-(5-methoxy-3-methoxymethylpyrazol-1-yl)-pyridin-4-yl]-pyrimidin-2-yl}-amin;
(3-Chlor-phenyl)-{4-[2-(5-methoxy-3-methoxymethyl-4-methyl-pyrazol-1-yl)-pyridin-4-yl]-pyrimidin-2-yl}-amin;
(3-Chlor-phenyl)-{4-[2-(5-methoxy-4-methyl-pyrazol-1-yl)-pyridin-4-yl]-pyrimidin-2-yl}-amin;
(3-Chlor-phenyl)-{4-[2-(5-ethoxy-3,4-dimethylpyrazol-1-yl)-pyridin-4-yl]-pyrimidin-2-yl}-amin;
2-{4-[2-(3-Chlor-phenylamino)-pyrimidin-4-yl]-pyridin-2-yl}-5-methoxymethyl-1,4-dimethyl-1,2-dihydropyrazol-3-on;
2-(4-{2-[(3-Chlor-phenyl)-methoxymethyl-amino]-pyrimidin-4-yl}-pyridin-2-yl)-1,5-dimethyl-1,2-dihydropyrazol-3-on;
2-{4-[2-(3-Chlor-phenylamino)-pyrimidin-4-yl]-pyridin-2-yl}-1-ethyl-4,5-dimethyl-1,2-dihydro-pyrazol-3-on;
2-{4-[2-(3-Chlor-phenylamino)-pyrimidin-4-yl]-pyridin-2-yl}-1,4-dimethyl-1,2-dihydro-pyrazol-3-on;
2-{4-[2-(3-Chlor-phenylamino)-pyrimidin-4-yl]-pyridin-2-yl}-1,5-dimethyl-1,2-dihydro-pyrazol-3-on;
2-{4-[2-(3-Chlor-phenylamino)-pyrimidin-4-yl]-pyridin-2-yl}-5-methoxymethyl-4,4-dimethyl-2,4-dihydropyrazol-3-on;
2-{4-[2-(3-Chlor-phenylamino)-pyrimidin-4-yl]-pyridin-2-yl}-4,4-dimethyl-2,4-dihydro-pyrazol-3-on;
2-{4-[2-(3-Chlor-phenylamino)-pyrimidin-4-yl]-pyridin-2-yl}-4,4,5-trimethyl-2,4-dihydro-pyrazol-3-thion;
5-{4-[2-(3-Chlor-phenylamino)-pyrimidin-4-yl]-pyridin-2-yl}-7-methyl-5,6-diazaspiro[2.4]hept-6-en-4-on;
2-{4-[2-(3-Chlor-phenylamino)-pyrimidin-4-yl]-pyridin-2-yl}-4-ethyl-4,5-dimethyl-2,4-dihydro-pyrazol-3-on;
(3-Chlor-phenyl)-{4-[2-(5-methoxy-3-methyl-pyrazol-1-yl)-pyridin-4-yl]-pyrimidin-2-yl}-amin;
2-{4-[2-(3-Chlor-phenylamino)-pyrimidin-4-yl]-pyridin-2-yl}-1,4,5-trimethyl-1,2-dihydro-pyrazol-3-on;
2-{4-[2-(3-Chlor-phenylamino)-pyrimidin-4-yl]-pyridin-2-yl}-4,4,5-trimethyl-2,4-dihydro-pyrazol-3-on;
2-{4-[2-(3-Chlor-phenylamino)-pyrimidin-4-yl]-pyridin-2-yl}-1,5-dimethyl-1,2-dihydro-pyrazol-3-on;
4,5-Dichlor-2-{4-[2-(3-chlor-phenylamino)-pyrimidin-4-yl]-pyridin-2-yl}-2H-pyridazin-3-on;
2-{4-[2-(3-Chlor-phenylamino)-pyrimidin-4-yl]-pyridin-2-yl}-6-methyl-2H-pyridazin-3-on;
2-{4-[2-(3-Chlor-phenylamino)-pyrimidin-4-yl]-pyridin-2-yl}-6-methyl-4,5-dihydro-2H-pyridazin-3-on;
2-{4-[2-(3-Chlor-phenylamino)-pyrimidin-4-yl]-pyridin-2-yl}-6-phenyl-4,5-dihydro-2H-pyridazin-3-on;
4-Chlor-2-{4-[2-(3-chlor-phenylamino)-pyrimidin-4-yl}-pyridin-2-yl}-5-ethoxy-2H-pyridazin-3-on;
4-Chlor-2-{4-[2-(3-chlor-phenylamino)-pyrimidin-4-yl]-pyridin-2-yl}-5-ethylsulfanyl-2H-pyridazin-3-on;
5-Azido-4-chlor-2-{4-[2-(3-chlor-phenylamino)-pyrimidin-4-yl}-pyridin-2-yl}-2H-pyridazin-3-on;
1-{4-[2-(3-Chlor-phenylamino)-pyrimidin-4-yl]-pyridin-2-yl}-2-methyl-pyrazolidin-3-on;
(3-Chlor-phenyl)-{4-[2-(5-methoxy-3,4-dimethylpyrazol-1-yl)-pyridin-4-yl]-pyrimidin-2-yl}-amin;
2-{4-[2-(3-Chlor-phenylamino)-pyrimidin-4-yl]-pyridin-2-yl}-5-methoxymethyl-1-methyl-1,2-dihydropyrazol-3-on;
2-{4-[2-(3-Chlor-phenylamino)-pyrimidin-4-yl]-pyridin-2-yl}-1,5-dimethyl-3-oxo-2,3-dihydro-1H-pyrazol-4-carbaldehyd;
5-Chlor-2-{4-[2-(3-chlor-phenylamino)-pyrimidin-4-yl]-pyridin-2-yl}-4-(oxetan-3-yloxy)-2H-pyridazin-3-on; und
4-Chlor-2-{4-[2-(3-chlor-phenylamino)-pyrimidin-4-yl]-pyridin-2-yl}-5-(tetrahydro-furan-2-ylmethoxy)-2H-pyridazin-3-on.

10. Zusammensetzung zur Bekämpfung von und Schutz vor phytopathogenen Mikroorganismen, die eine Verbindung der Formel (I) gemäß Anspruch 1 als Wirkstoff zusammen mit einem geeigneten Träger umfaßt.

11. Verwendung einer Verbindung der Formel (I) gemäß Anspruch 1 im Schutz von Pflanzen gegen Befall durch phytopathogene Mikroorganismen.

12. Verfahren zur Bekämpfung und Prävention eines Befalls von Nutzpflanzen durch phytopathogene Mikroorganismen, das die Ausbringung einer Verbindung der Formel (I) gemäß Anspruch 1 als Wirkstoff auf die Pflanze, auf Teile von Pflanzen oder auf ihren Locus umfaßt.

13. Verfahren gemäß Anspruch 12, worin die phytopathogenen Mikroorganismen pilzliche Organismen sind.

## Revendications

1. Composé de formule I 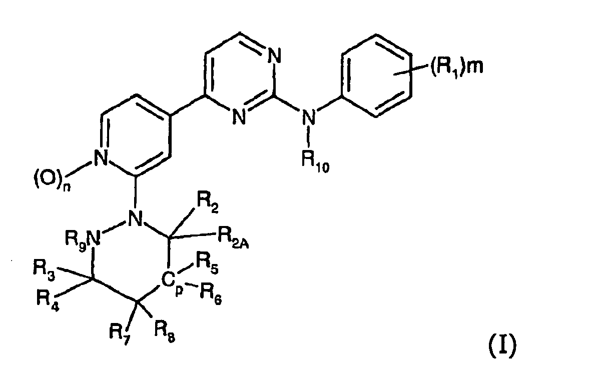 dans laquelle
m est égal à 0, 1, 2 ou 3;
n et p sont indépendamment l'un de l'autre égaux à 0 ou 1 ;
R₁ est un halogène, un alkyle facultativement substitué, un alcoxy facultativement substitué, un alcényloxy facultativement substitué, un alcynyloxy facultativement substitué, un thioalkyle facultativement substitué, un aryle facultativement substitué, COOR₁₁, CONR₁₂R₁₃, S(O)_{q}R₁₄, SO₂NR₁₅R₁₆ ou NR₁₅ₐR₁₆ₐ ; lorsqu'il y a plus d'un groupe R₁, ils peuvent être identiques ou différents ;
q est égal à 1 ou 2 ;
R₂, R₂ₐ, R₃, R₄, R₅, R₆, R₇, R₈ sont chacun indépendamment l'hydrogène, un alkyle facultativement substitué, COR₁₇, COOR₁₈ ou un aryle facultativement substitué, et de plus R₂ et R₃ peuvent également être indépendamment un alcoxy facultativement substitué, un alcényloxy facultativement substitué, un alcynyloxy facultativement substitué, ou un alkylthio facultativement substitué, COOR₁₉, CONR₂₀R₂₁, OH ou SH ;
R₆ et R₇ peuvent également être indépendamment un halogène, un alcoxy facultativement substitué, un alcényloxy facultativement substitué, un alcynyloxy facultativement substitué, un alcénylamino facultativement substitué, un alcynylamino facultativement substitué, un alkylthio facultativement substitué, un cycloalkyle facultativement substitué, un hétéroaryle facultativement substitué, un hétérocyclyle facultativement substitué, un cycloalkyloxy facultativement substitué, OH, SH, N₃, NR₂₂R₂₃ ou N(R₂₄)COR₂₅ ;
ou les chaînons de cycle CR₃R₄ ou CR₂R_{2A} sont indépendamment l'un de l'autre un groupe carbonyle (C=O) ou un groupe thionyle (C=S) ;
ou une ou deux des paires adjacentes de groupes R₉ et R₄, R₄ et R₈, R₅ et R₈, ou, si p est égal à zéro, R_{2A} et R₈ peuvent former une liaison, à condition que s'il y a 2 doubles liaisons dans le cycle, les doubles liaisons ne soient pas adjacentes l'une de l'autre ;
ou la paire de groupes R₇ et R₈ ou la paire de groupes R₆ et R₇, conjointement avec l'atome auquel ils sont liés, forme un cycle saturé en C₃ à C₇ ;
R₉ est l'hydrogène, un alkyle facultativement substitué, un alcényle facultativement substitué ou un alcynyle facultativement substitué ;
R₁₀ est l'hydrogène, un alkyle en C₁ à C₄, un alcényle en C₃ à C₄, un alcynyle en C₃ à C₄, -CH₂OR₂₆, CH₂SR₂₇, -C(O)R₂₈, -C(O)OR₂₉, SO₂R₃₀, SOR₃₁ ou SR₃₂ ;
R₂₆, R₂₇, R₂₈, R₂₉, R₃₀, R₃₁, R₃₂ sont indépendamment un alkyle en C₁ à C₈, un alcoxyalkyle en C₁ à C₈, un haloalkyle en C₁ à C₈, ou un phényl-(alkyle en C₁ à C₂) dans lequel le phényle peut être substitué par jusqu'à trois groupes choisis parmi un halogène ou un alkyle en C₁ à C₄,
R₁₁, R₁₂, R₁₃, R₁₄, R₁₅, R₁₆, R₁₅ₐ, R₁₆ₐ, R₁₇, R₁₈, R₁₉, R₂₀, R₂₁, R₂₂, R₂₃, R₂₄, et R₂₅ sont indépendamment H ou un alkyle facultativement substitué ; ou un sel de celui-ci.

2. Composé selon la revendication 1, dans lequel le groupement 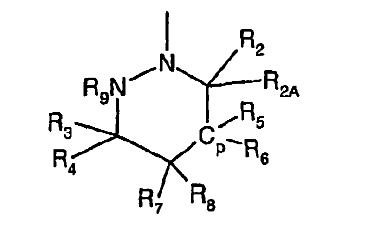 est un cycle à 5 et 6 chaînons choisi parmi les 2,4-dihydro-pyrazol-3-ones, la 2,4-dihydro-pyrazol-3-thione, les 1H-pyrazoles, les 2H-pyridazin-3-ones, les 4,5-dihydro-2H-pyridazin-3-ones, les 1,2-dihydro-pyrazol-3-ones, la 1,2-dihydro-pyrazol-3-thione, la pyrazolidin-3-one, la pyrazolidin-3-thione, la 2H-pyridazin-3-thione et la 4,5-dihydro-2H-pyridazin-3-thione.

3. Composé selon les revendications 1 ou 2, dans lequel R₁ est un halogène, un haloalcoxy en C₁ à C₃, CH(OH)R, COR, SO₂NRR', CH(NR'R")R, COORa ou CONRbRc où Ra, Rb, Rc, R, R', R" sont indépendamment H ou un alkyle inférieur.

4. Composé selon l'une quelconque des revendications 1 à 3, dans lequel R₂, R_{2A}, R₃, R₄, R₅, R₆, R₇, R₈ et R₉ sont indépendamment l'un de l'autre l'hydrogène ou un méthyle.

5. Composé selon l'une quelconque des revendications 1 à 4, dans lequel n est égal à zéro.

6. Composé selon l'une quelconque des revendications 1 à 5, dans lequel m est égal à 1 et le groupe R₁ est situé au niveau de la position 3 ou 4 du cycle phényle.

7. Composé selon l'une quelconque des revendications 1 à 6, dans lequel R₇ est l'hydrogène, un méthyle, un éthyle, un allyle, un propargyle, un méthoxyméthyle, un thiométhoxyméthyle ou un éthoxyméthyle, de préférence l'hydrogène ou un méthoxyméthyle.

8. Composé selon l'une quelconque des revendications 1 à 7, dans lequel R₁₀ est l'hydrogène, un méthyle, un éthyle, un allyle, un propargyle, un méthoxyméthyle, un thiométhoxyméthyle ou un éthoxyméthyle, de préférence l'hydrogène ou un méthoxyméthyle.

9. Composé selon l'une quelconque des revendications 1 à 8, dans lequel le composé est choisi parmi la (3-chloro-phényle)-{4-[2-(3,4,5-triméthyl-pyrazol-1-yle)-pyridin-4-yle]-pyrimidin-2-yle}-amine ;
la (3-chloro-phényle)-{4-[2-(5-méthoxy-3-méthoxyméthyl-pyrazol-1-yle)-pyridin-4-yle]-pyrimidin-2-yle}-amine ;
la (3-chloro-phényle)-{4-[2-(5-méthoxy-3-méthoxyméthyl-4-méthylpyrazol-1-yle)-pyridin-4-yle]-pyrimidin-2-yle}-amine ;
la (3-chloro-phényle)-{4-[2-(5-méthoxy-4-méthyl-pyrazol-1-yle)-pyridin-4-yle]-pyrimidin-2-yle}-amine ;
la (3-chloro-phényle)-{4-[2-(5-éthoxy-3,4-diméthyl-pyrazol-1-yle)-pyridin-4-yle]-pyrimidin-2-yle}-amine ;
la 2-{4-[2-(3-chloro-phénylamino)-pyrimidin-4-yle]-pyridin-2-yle}-5-méthoxyméthyl-1,4-diméthyl-1,2-dihydro-pyrazol-3-one ;
la 2-(4-{2-[(3-chloro-phényle)-méthoxyméthyl-amino]-pyrimidin-4-yle}-pyridin-2-yle)-1,5-diméthyl-1,2-dihydro-pyrazol-3-one ;
la 2-{4-[2-(3-chloro-phénylamino)-pyrimidin-4-yle]-pyridin-2-yle}-1-éthyl-4,5-diméthyl-1,2-dihydro-pyrazol-3-one ;
la 2-{4-[2-(3-chloro-phénylamino)-pyrimidin-4-yle]-pyridin-2-yle}-1,4-diméthyl-1,2-dihydro-pyrazol-3-one ;
la 2-{4-[2-(3-chloro-phénylamino)-pyrimidin-4-yle]-pyridin-2-yle}-1,5-diméthyl-1,2-dihydro-pyrazol-3-one ;
la 2-{4-[2-(3-chloro-phénylamino)-pyrimidin-4-yle]-pyridin-2-yle}-5-méthoxyméthyl-4,4-diméthyl-2,4-dihydro-pyrazol-3-one ;
la 2-{4-[2-(3-chloro-phénylamino)-pyrimidin-4-yle]-pyridin-2-yle}-4,4-diméthyl-2,4-dihydro-pyrazol-3-one ;
la 2-{4-[2-(3-chloro-phénylamino)-pyrimidin-4-yle]-pyridin-2-yle}-4,4,5-triméthyl-2,4-dihydro-pyrazol-3-thione ;
la 5-{4-[2-(3-chloro-phénylamino)-pyrimidin-4-yle]-pyridin-2-yle}-7-méthyl-5,6-diaza-spiro [2,4] hept-6-èn-4-one ;
la 2-{4-[2-(3-chloro-phénylamino)-pyrimidin-4-yle]-pyridin-2-yle}-4-éthyl-4,5-diméthyl-2,4-dihydro-pyrazol-3-one ;
la (3-chloro-phényle)-{4-[2-(5-méthoxy-3-méthyl-pyrazol-1-yle)-pyridin-4-yle]-pyrimidin-2-yle}-amine ;
la 2-{4-[2-(3-chloro-phénylamino)-pyrimidin-4-yle]-pyridin-2-yle}-1,4,5-triméthyl-1,2-dihydro-pyrazol-3-one ;
la 2-{4-[2-(3-chloro-phénylamino)-pyrimidin-4-yle]-pyridin-2-yle}-4,4,5-triméthyl-2,4-dihydro-pyrazol-3-one ;
la 2-{4-[2-(3-chloro-phénylamino)-pyrimidin-4-yle]-pyridin-2-yle}-1,5-diméthyl-1,2-dihydro-pyrazol-3-one ;
la 4,5-dichloro-2-{4-[2-(3-chloro-phénylamino)-pyrimidin-4-yle]-pyridin-2-yle}-2H-pyridazin-3-one ;
la 2-{4-[2-(3-chloro-phénylamino)-pyrimidin-4-yle]-pyridin-2-yle}-6-méthyl-2H-pyridazin-3-one ;
la 2-{4-[2-(3-chloro-phénylamino)-pyrimidin-4-yle]-pyridin-2-yle}-6-méthyl-4,5-dihydro-2H-pyridazin-3-one ;
la 2-{4-[2-(3-chloro-phénylamino)-pyrimidin-4-yle]-pyridin-2-yle}-6-phényl-4,5-dihydro-2H-pyridazin-3-one ;
la 4-chloro-2-{4-[2-(3-chloro-phénylamino)-pyrimidin-4-yle]-pyridin-2-yle}-5-éthoxy-2H-pyridazin-3-one ;
la 4-chioro-2-{4-[2-(3-chloro-phénylamino)-pyrimidin-4-yle]-pyridin-2-yle}-5-éthylsulfanyl-2H-pyridazin-3-one ;
la 5-azido-4-chloro-2-{4-[2-(3-chloro-phénylamino)-pyrimidin-4-yle]-pyridin-2-yle}-2H-pyridazin-3-one ;
la 1-{4-[2-(3-chloro-phénylamino)-pyrimidin-4-yle]-pyridin-2-yle}-2-méthyl-pyrazolidin-3-one ;
la (3-chloro-phényle)-{4-[2-(5-méthoxy-3,4-diméthyl-pyrazol-1-yle)-pyridin-4-yle]-pyrimidin-2-yle}-amine ;
la 2-{4-[2-(3-chloro-phénylamino)-pyrimidin-4-yle]-pyridin-2-yle}-5-méthoxyméthyl-1-méthyl-1,2-dihydro-pyrazol-3-one ;
le 2-{4-[2-(3-chloro-phénylamino)-pyrimidin-4-yle]-pyridin-2-yle}-1,5-diméthyl-3-oxo-2,3-dihydro-1H-pyrazol-4-carbaldéhyde ;
la 5-chloro-2-{4-[2-(3-chloro-phénylamino)-pyrimidin-4-yle]-pyridin-2-yle}-4-(oxétan-3-yloxy)-2H-pyridazin-3-one ; et
la 4-chloro-2-{4-[2-(3-chloro-phénylamino)-pyrimidin-4-yle]-pyridin-2-yle}-5-(tétrahydro-furan-2-ylméthoxy)-2H-pyridazin-3-one.

10. Composition pour le contrôle et la protection contre les microorganismes phytopathogènes, comprenant un composé de formule I selon la revendication 1 en tant qu'ingrédient actif, conjointement avec un support approprié.

11. Utilisation d'un composé de formule I selon la revendication 1 dans la protection des plantes contre une infestation par des microorganismes phytopathogènes.

12. Procédé de contrôle et de prévention d'une infestation de plantes cultivées par des microorganismes phytopathogènes, qui comprend l'application d'un composé de formule 1 selon la revendication 1 en tant qu'ingrédient actif à la plante, aux parties de plantes ou au locus de celles-ci.

13. Procédé selon la revendication 12, dans lequel les microorganismes phytopathogènes sont des organismes fongiques.
